# EUROPEAN PATENT SPECIFICATION

(11) **EP 1 434 592 B1**
(45) Date of publication and mention of the grant of the patent: **26.11.2008**
(21) Application number: 02762038.4
(22) Date of filing: 09.04.2002
(51) Int. Cl.: C07K 14/47, C12N 15/12, C07K 16/00, A61K 38/00, A61K 39/00

(54) **NUCLEIC ACID AND CORRESPONDING PROTEIN ENTITLED 121P2A3 USEFUL IN TREATMENT AND DETECTION OF CANCER**
NUKLEINSÄURE UND ENTSPRECHENDES PROTEIN MIT DER BEZEICHNUNG 121P2A3 ZUR BEHANDLUNG UND ZUM NACHWEIS VON KREBS
ACIDE NUCLEIQUE ET PROTEINE CORRESPONDANTE APPELE 121P2A3 UTILE POUR LE TRAITEMENT ET LA DETECTION DES CANCERS

(30) Priority: 10.04.2001 US 282739 P; 25.04.2001 US 286630 P; 22.06.2001 US 300373 P
(43) Date of publication of application: 07.07.2004
(60) Divisional application: 07001331.3
(73) Proprietor: Agensys, Inc., Santa Monica, CA 90404 (US)
(72) Inventor: CHALLITA-EID, Pia, M., Encino, CA 91436 (US); RAITANO, Arthur, B., Los Angeles, CA 90064 (US); FARIS, Mary, Los Angeles, CA 90077 (US); HUBERT, Rene, S., Los Angeles, CA 90026 (US); MITCHELL, Steve, Chappell, Gurnee, IL 60031 (US); AFAR, Daniel, E., H., Brisbane, CA 94005 (US); SAFFRAN, Douglas, Encinitas, CA 92024 (US); MORRISON, Karen, Santa Monica, CA 90403 (US); MORRISON, Robert, Kendall, Santa Monica, CA 90403 (US); GE, Wangmao, Culver City, CA 90230 (US); JAKOBOVITS, Aya, Beverly Hills, CA 90210 (US)
(74) Representative: Roques, Sarah Elizabeth
(86) International application number: PCT/US2002/011359
(87) International publication number: WO 2002/083068

(56) References cited:
- WO-A2-00/73801
- WO-A2-01/22920
- WO-A2-02/31111
- WO-A2-02/059271
- WO-A2-02/072757
- DATABASE EMBL [Online] 17 December 2003 (2003-12-17), "Sequence 11575 from Patent EP1074617." XP002316079 retrieved from EBI accession no. EM_PRO:AX876670 Database accession no. AX876670 -& EP 1 074 617 A (RESEARCH ASSOCIATION FORBIOTECHNOLOGY) 7 February 2001 (2001-02-07)
- DATABASE EPO Proteins [Online] 17 December 2003 (2003-12-17), "Sequence 11576 from Patent EP1074617." XP002316080 retrieved from EBI accession no. EPOP:AX876671 Database accession no. AX876671 & EP 1 074 617 A (RESEARCH ASSOCIATION FORBIOTECHNOLOGY) 7 February 2001 (2001-02-07)
- DATABASE EMBL [Online] 30 January 2004 (2004-01-30), "Sequence 21355 from Patent WO0160860." XP002316081 retrieved from EBI accession no. EM_PRO:CQ489488 Database accession no. CQ489488 -& WO 01/60860 A2 (MILLENNIUM PREDICTIVE MEDICINE, INC) 23 August 2001 (2001-08-23)
- DATABASE EMBL [Online] 3 February 2004 (2004-02-03), "Sequence 14356 from Patent WO02068579." XP002316082 retrieved from EBI accession no. EM_PRO:CQ728422 Database accession no. CQ728422 -& WO 02/068579 A2 (PE CORPORATION) 6 September 2002 (2002-09-06)
- DATABASE EMBL [Online] 3 February 2004 (2004-02-03), "Sequence 14350 from Patent WO02068579." XP002316083 retrieved from EBI accession no. EM_PRO:CQ728416 Database accession no. CQ728416 & WO 02/068579 A2 (PE CORPORATION) 6 September 2002 (2002-09-06)
- DATABASE EMBL [Online] 22 February 2000 (2000-02-22), "Homo sapiens cDNA FLJ10540 fis, clone NT2RP2001245." XP002316084 retrieved from EBI accession no. EM_PRO:AK001402 Database accession no. AK001402
- DATABASE EMBL [Online] 16 May 2000 (2000-05-16), "Human DNA sequence from clone RP11-30E16 on chromosome 10 Contains the C10orf3 gene for chromosome 10 open reading frame 3, the RBP4 gene for retinol binding protein 4 (plasma), the 5' end of the PDE6C gene for phosphodiesterase 6C (cGMP-specific, cone, alpha prime), the GPR120 gene for G protein-co" XP002316085 retrieved from EBI accession no. EM_PRO:AL356214 Database accession no. AL356214
- DATABASE EMBL [Online] 8 February 2001 (2001-02-08), "Mus musculus adult male lung cDNA, RIKEN full-length enriched library, clone:1200008O12 product:hypothetical protein, full insert sequence." XP002316086 retrieved from EBI accession no. EM_PRO:AK004655 Database accession no. AK004655
- LANTZ VALERIE A ET AL: "A class VI unconventional myosin is associated with a homologue of a microtubule-binding protein, cytoplasmic linker protein-170, in neurons and at the posterior pole of Drosophila embryos" JOURNAL OF CELL BIOLOGY, vol. 140, no. 4, 23 February 1998 (1998-02-23), pages 897-910, XP002315987 ISSN: 0021-9525
- FUKUSHI M ET AL: "Identification and cloning of a novel cellular protein Naf1, Nef-associated factor 1, that increases cell surface CD4 expression" FEBS LETTERS, ELSEVIER SCIENCE PUBLISHERS, AMSTERDAM, NL, vol. 442, no. 1, 8 January 1999 (1999-01-08), pages 83-88, XP004259009 ISSN: 0014-5793
- YAMADA YOICHI ET AL: "Receptor for hyaluronan-mediated motility and CD44 expressions in colon cancer assessed by quantitative analysis using real-time reverse transcriptase-polymerase chain reaction" JAPANESE JOURNAL OF CANCER RESEARCH, vol. 90, no. 9, September 1999 (1999-09), pages 987-992, XP002316136 ISSN: 0910-5050

## Description

### FIELD OF THE INVENTION

A gene and its encoded protein, termed 121P2A3, expressed in certain cancers is described herein. The invention relates to diagnostic methods useful in the management of cancers that express 121P2A3.

### BACKGROUND OF THE INVENTION

Cancer is the second leading cause of human death next to coronary disease. Worldwide, millions of people die from cancer every year. In the United States alone, as reported by the American Cancer Society, cancer causes the death of well over a half-million people annually, with over 1.2 million new cases diagnosed per year. While deaths from heart disease have been declining significantly, those resulting from cancer generally are on the rise. In the early part of the next century, cancer is predicted to become the leading cause of death.

Worldwide, several cancers stand out as the leading killers. In particular, carcinomas of the lung, prostate, breast, colon, pancreas, and ovary represent the primary causes of cancer death. These and virtually all other carcinomas share a common lethal feature. With very few exceptions, metastatic disease from a carcinoma is fatal. Moreover, even for those cancer patients who initially survive their primary cancers, common experience has shown that their lives are dramatically altered. Many cancer patients experience strong anxieties driven by the awareness of the potential for recurrence or treatment failure. Many cancer patients experience physical debilitations following treatment. Furthermore, many cancer patients experience a recurrence.

Worldwide, prostate cancer is the fourth most prevalent cancer in men. In North America and Northern Europe, it is by far the most common cancer in males and is the second leading cause of cancer death in men. In the United States alone, well over 30,000 men die annually of this disease - second only to lung cancer. Despite the magnitude of these figures, there is still no effective treatment for metastatic prostate cancer. Surgical prostatectomy, radiation therapy, hormone ablation therapy, surgical castration and chemotherapy continue to be the main treatment modalities. Unfortunately, these treatments are ineffective for many and are often associated with undesirable consequences.

On the diagnostic front, the lack of a prostate tumor marker that can accurately detect early-stage, localized tumors remains a significant limitation in the diagnosis and management of this disease. Although the serum prostate specific antigen (PSA) assay has been a very useful tool, however its specificity and general utility is widely regarded as lacking in several important respects.

Progress in identifying additional specific markers for prostate cancer has been improved by the generation of prostate cancer xenografts that can recapitulate different stages of the disease in mice. The LAPC (Los Angeles Prostate Cancer) xenografts are prostate cancer xenografts that have survived passage in severe combined immune deficient (SCID) mice and have exhibited the capacity to mimic the transition from androgen dependence to androgen independence (Klein et al., 1997, Nat. Med. 3:402). More recently identified prostate cancer markers include PCTA-1 (Su et al., 1996, Proc. Natl. Acad. Sci. USA 93: 7252), prostate-specific membrane (PSM) antigen (Pinto et al., Clin Cancer Res 1996 Sep 2 (9): 1445-51), STEAP (Hubert, et al., Proc Natl Acad Sci USA. 1999 Dec 7; 96(25): 14523-8) and prostate stem cell antigen (PSCA) (Reiter *et al.,* 1998, Proc. Natl. Acad. Sci. USA 95: 1735).

While previously identified markers such as PSA, PSM, PCTA and PSCA have facilitated efforts to diagnose and treat prostate cancer, there is need for the identification of additional markers and therapeutic targets for prostate and related cancers in order to further improve diagnosis and therapy.

Renal cell carcinoma (RCC) accounts for approximately 3 percent of adult malignancies. Once adenomas reach a diameter of 2 to 3 cm, malignant potential exists. In the adult, the two principal malignant renal tumors are renal cell adenocarcinoma and transitional cell carcinoma of the renal pelvis or ureter. The incidence of renal cell adenocarcinoma is estimated at more than 29,000 cases in the United States, and more than 11,600 patients died of this disease in 1998. Transitional cell carcinoma is less frequent, with an incidence of approximately 500 cases per year in the United States.

Surgery has been the primary therapy for renal cell adenocarcinoma for many decades. Until recently, metastatic disease has been refractory to any systemic therapy. With recent developments in systemic therapies, particularly immunotherapies, metastatic renal cell carcinoma may be approached aggressively in appropriate patients with a possibility of durable responses. Nevertheless, there is a remaining need for effective therapies for these patients.

Of all new cases of cancer in the United States, bladder cancer represents approximately 5 percent in men (fifth most common neoplasm) and 3 percent in women (eighth most common neoplasm). The incidence is increasing slowly, concurrent with an increasing older population. In 1998, there was an estimated 54,500 cases, including 39,500 in men and 15,000 in women. The age-adjusted incidence in the United States is 32 per 100,000 for men and 8 per 100,000 in women. The historic male/female ratio of 3:1 may be decreasing related to smoking patterns in women. There were an estimated 11,000 deaths from bladder cancer in 1998 (7,800 in men and 3,900 in women). Bladder cancer incidence and mortality strongly increase with age and will be an increasing problem as the population becomes more elderly.

Most bladder cancers recur in the bladder. Bladder cancer is managed with a combination of transurethral resection of the bladder (TUR) and intravesical chemotherapy or immunotherapy. The multifocal and recurrent nature of bladder cancer points out the limitations of TUR. Most muscle-invasive cancers are not cured by TUR alone. Radical cystectomy and urinary diversion is the most effective means to eliminate the cancer but carry an undeniable impact on urinary and sexual function. There continues to be a significant need for treatment modalities that are beneficial for bladder cancer patients.

An estimated 130,200 cases of colorectal cancer occurred in 2000 in the United States, including 93,800 cases of colon cancer and 36,400 of rectal cancer. Colorectal cancers are the third most common cancers in men and women. Incidence rates declined significantly during 1992-1996 (-2.1% per year). Research suggests that these declines have been due to increased screening and polyp removal, preventing progression of polyps to invasive cancers. There were an estimated 56,300 deaths (47,700 from colon cancer, 8,600 from rectal cancer) in 2000, accounting for about 11% of all U.S. cancer deaths.

At present, surgery is the most common form of therapy for colorectal cancer, and for cancers that have not spread, it is frequently curative. Chemotherapy, or chemotherapy plus radiation, is given before or after surgery to most patients whose cancer has deeply perforated the bowel wall or has spread to the lymph nodes. A permanent colostomy (creation of an abdominal opening for elimination of body wastes) is occasionally needed for colon cancer and is infrequently required for rectal cancer. There continues to be a need for effective diagnostic and treatment modalities for colorectal cancer.

There were an estimated 164,100 new cases of lung and bronchial cancer in 2000, accounting for 14% of all U.S. cancer diagnoses. The incidence rate of lung and bronchial cancer is declining significantly in men, from a high of 86.5 per 100,000 in 1984 to 70.0 in 1996. In the 1990s, the rate of increase among women began to slow. In 1996, the incidence rate in women was 42.3 per 100,000.

Lung and bronchial cancer caused an estimated 156,900 deaths in 2000, accounting for 28% of all cancer deaths. During 1992-1996, mortality from lung cancer declined significantly among men (-1.7% per year) while rates for women were still significantly increasing (0.9% per year). Since 1987, more women have died each year of lung cancer than breast cancer, which, for over 40 years, was the major cause of cancer death in women. Decreasing lung cancer incidence and mortality rates most likely resulted from decreased smoking rates over the previous 30 years; however, decreasing smoking patterns among women lag behind those of men. Of concern, although the declines in adult tobacco use have slowed, tobacco use in youth is increasing again.

Treatment options for lung and bronchial cancer are determined by the type and stage of the cancer and include surgery, radiation therapy, and chemotherapy. For many localized cancers, surgery is usually the treatment of choice. Because the disease has usually spread by the time it is discovered, radiation therapy and chemotherapy are often needed in combination with surgery. Chemotherapy alone or combined with radiation is the treatment of choice for small cell lung cancer; on this regimen, a large percentage of patients experience remission, which in some cases is long lasting. There is however, an ongoing need for effective treatment and diagnostic approaches for lung and bronchial cancers.

An estimated 182,800 new invasive cases of breast cancer were expected to occur among women in the United States during 2000. Additionally, about 1,400 new cases of breast cancer were expected to be diagnosed in men in 2000. After increasing about 4% per year in the 1980s, breast cancer incidence rates in women have leveled off in the 1990s to about 110.6 cases per 100,000.

In the U.S. alone, there were an estimated 41,200 deaths (40,800 women, 400 men) in 2000 due to breast cancer. Breast cancer ranks second among cancer deaths in women. According to the most recent data, mortality rates declined significantly during 1992-1996 with the largest decreases in younger women, both white and black. These decreases were probably the result of earlier detection and improved treatment.

Taking into account the medical circumstances and the patient's preferences, treatment of breast cancer may involve lumpectomy (local removal of the tumor) and removal of the lymph nodes under the arm; mastectomy (surgical removal of the breast) and removal of the lymph nodes under the arm; radiation therapy; chemotherapy; or hormone therapy. Often, two or more methods are used in combination. Numerous studies have shown that, for early stage disease, long-term survival rates after lumpectomy plus radiotherapy are similar to survival rates after modified radical mastectomy. Significant advances in reconstruction techniques provide several options for breast reconstruction after mastectomy. Recently, such reconstruction has been done at the same time as the mastectomy.

Local excision of ductal carcinoma *in situ* (DCIS) with adequate amounts of surrounding normal breast tissue may prevent the local recurrence of the DCIS. Radiation to the breast and/or tamoxifen may reduce the chance of DCIS occurring in the remaining breast tissue. This is important because DCIS, if left untreated, may develop into invasive breast cancer. Nevertheless, there are serious side effects or sequelae to these treatments. There is, therefore, a need for efficacious breast cancer treatments.

There were an estimated 23,100 new cases of ovarian cancer in the United States in 2000. It accounts for 4% of all cancers among women and ranks second among gynecologic cancers. During 1992-1996, ovarian cancer incidence rates were significantly declining. Consequent to ovarian cancer, there were an estimated 14,000 deaths in 2000. Ovarian cancer causes more deaths than any other cancer of the female reproductive system.

Surgery, radiation therapy, and chemotherapy are treatment options for ovarian cancer. Surgery usually includes the removal of one or both ovaries, the fallopian tubes (salpingo-oophorectomy), and the uterus (hysterectomy). In some very early tumors, only the involved ovary will be removed, especially in young women who wish to have children. In advanced disease, an attempt is made to remove all intra-abdominal disease to enhance the effect of chemotherapy. There continues to be an important need for effective treatment options for ovarian cancer.

There were an estimated 28,300 new cases of pancreatic cancer in the United States in 2000. Over the past 20 years, rates of pancreatic cancer have declined in men. Rates among women have remained approximately constant but may be beginning to decline. Pancreatic cancer caused an estimated 28,200 deaths in 2000 in the United States. Over the past 20 years, there has been a slight but significant decrease in mortality rates among men (about -0.9% per year) while rates have increased slightly among women.

Surgery, radiation therapy, and chemotherapy are treatment options for pancreatic cancer. These treatment options can extend survival and/or relieve symptoms in many patients but are not likely to produce a cure for most. There is a significant need for additional therapeutic and diagnostic options for pancreatic cancer.

### SUMMARY OF THE INVENTION

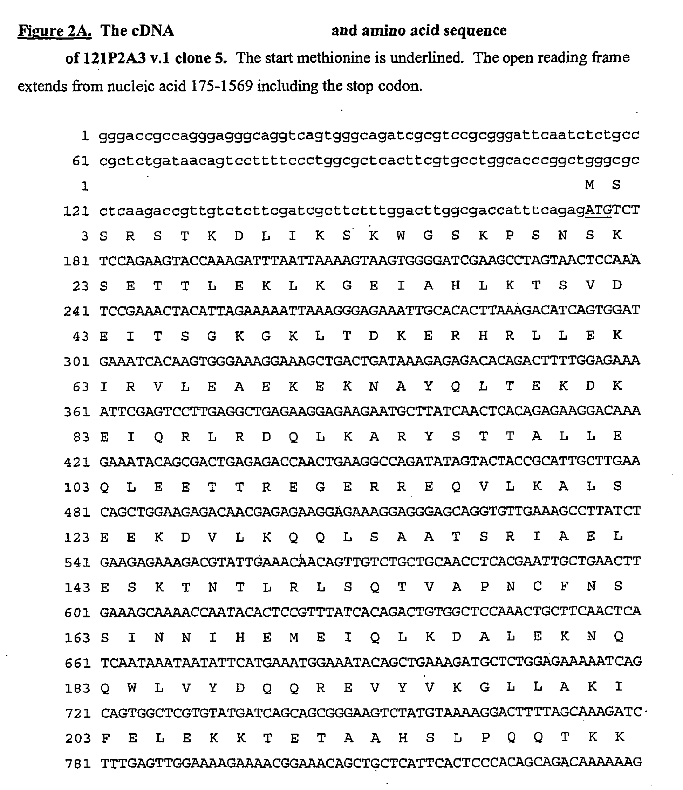

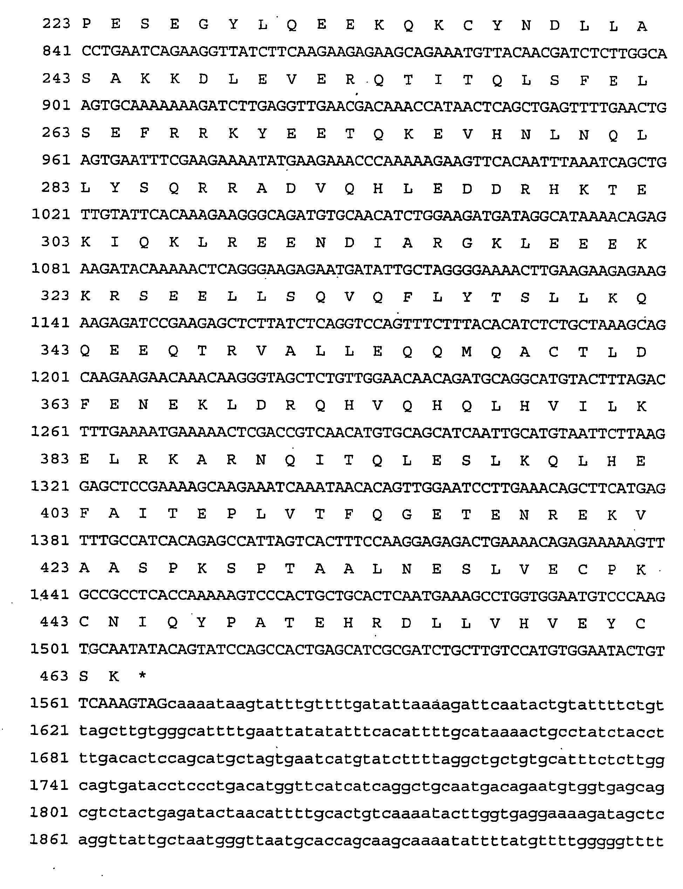

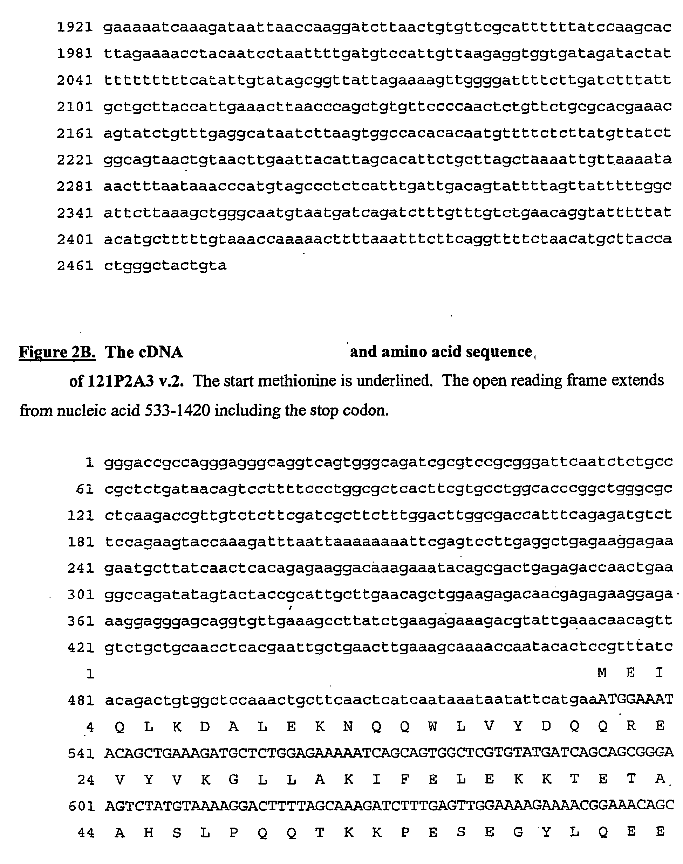

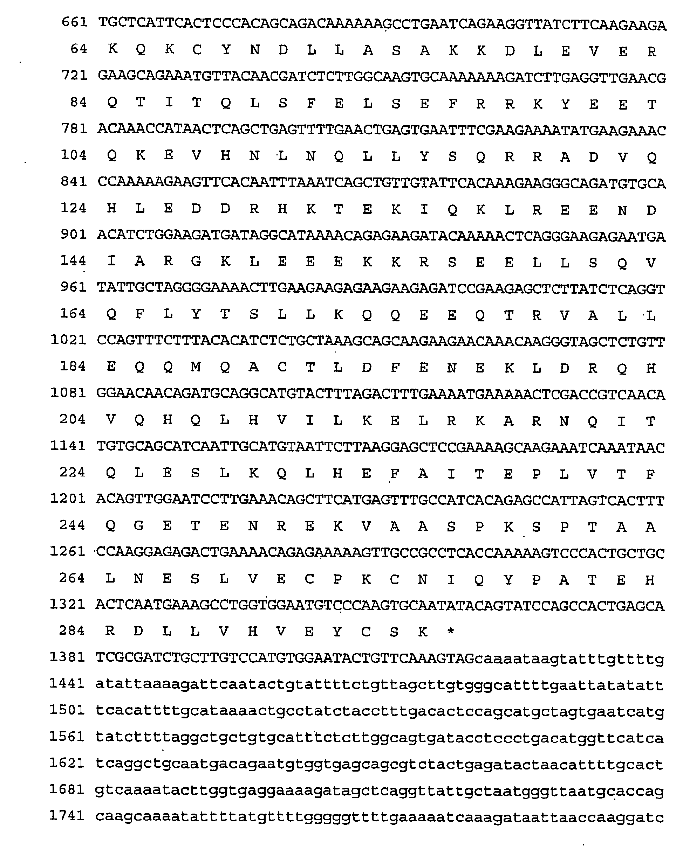

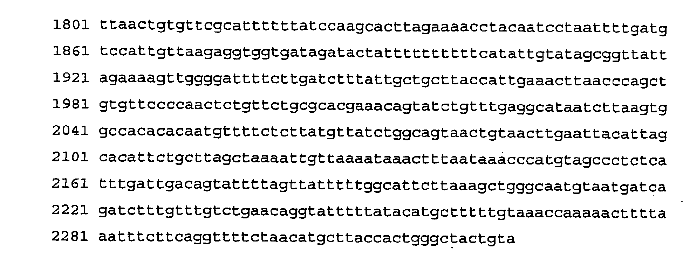

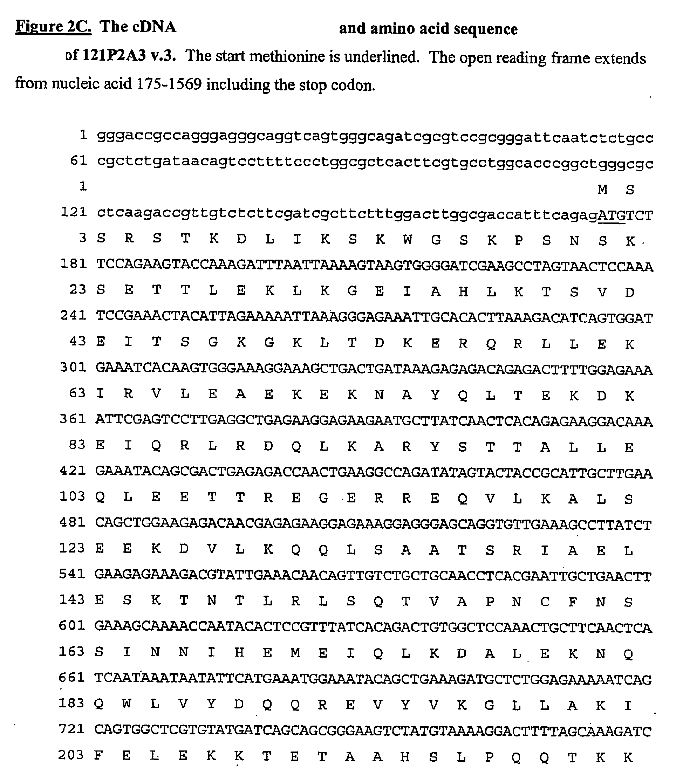

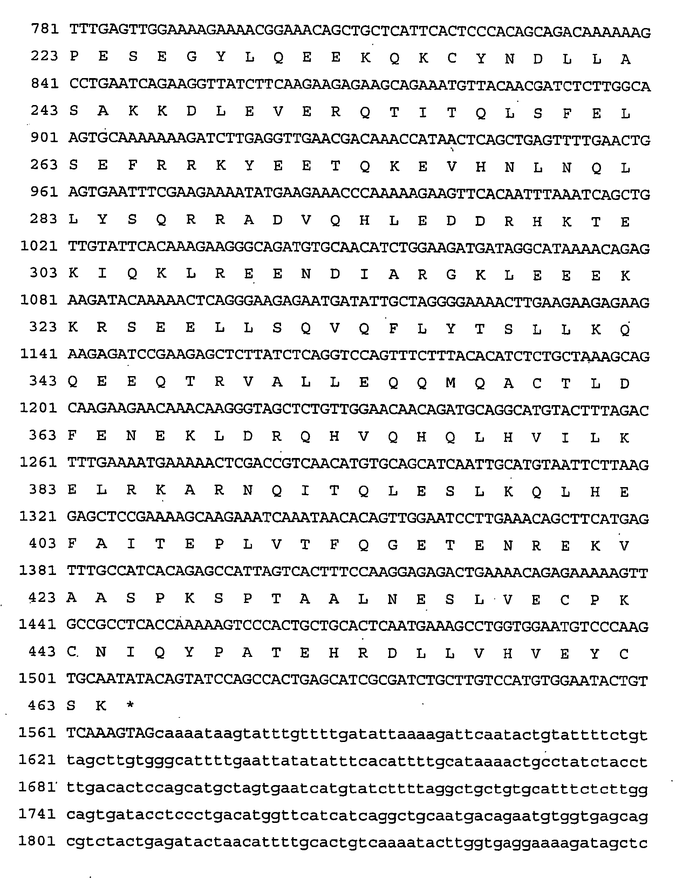

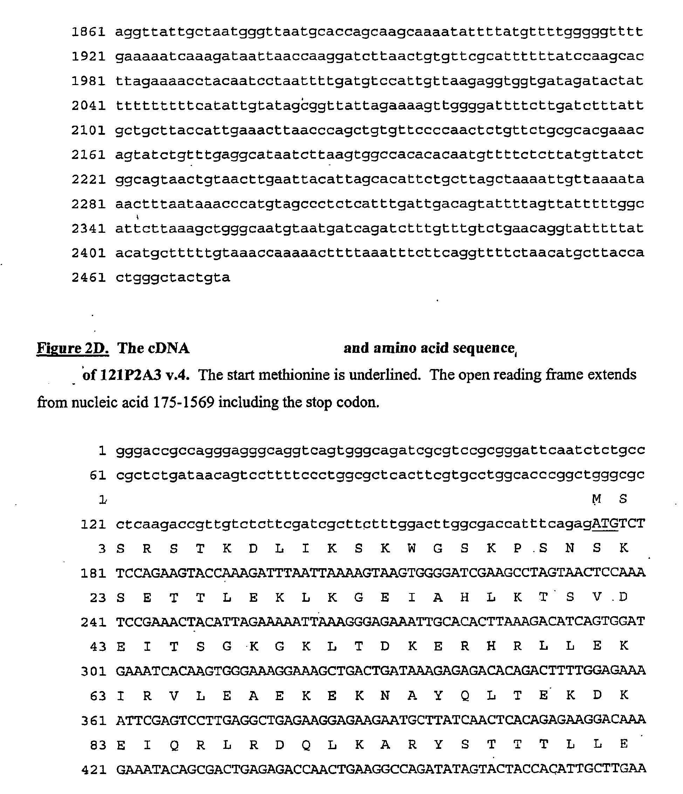

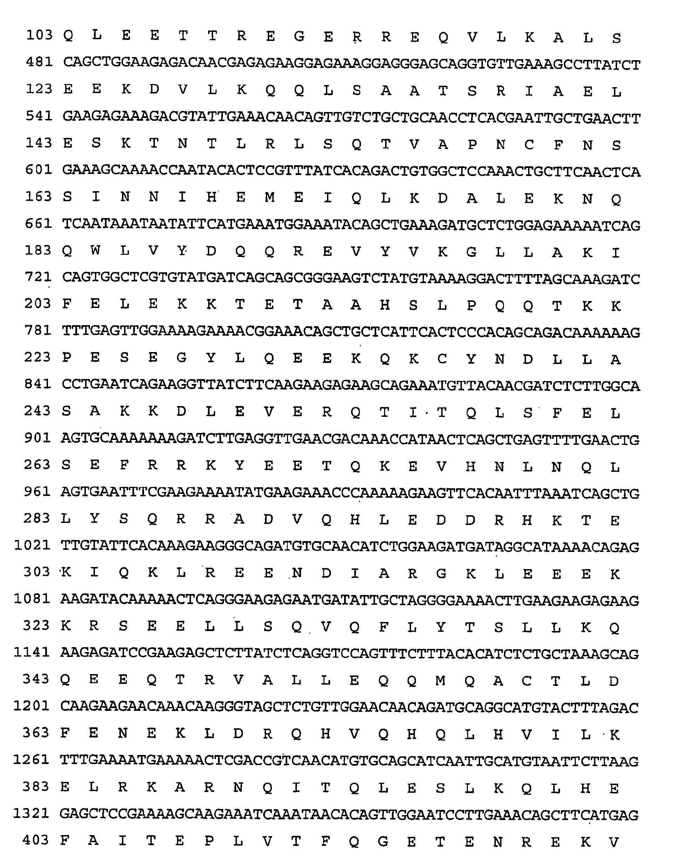

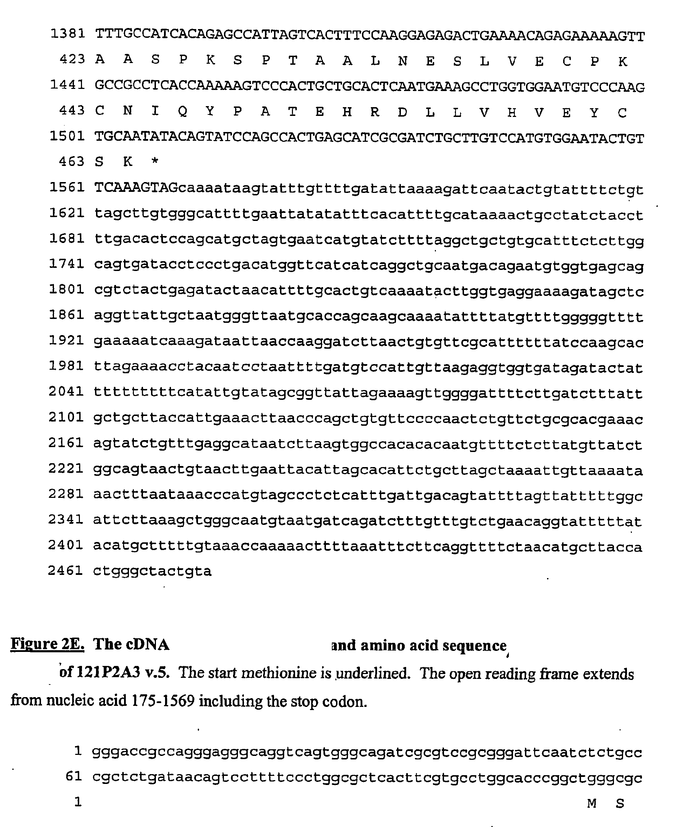

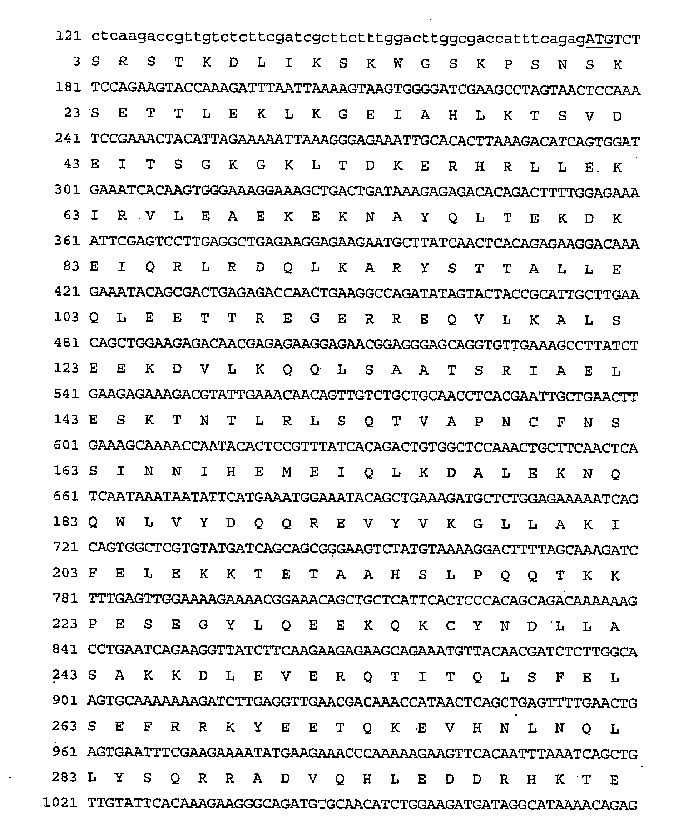

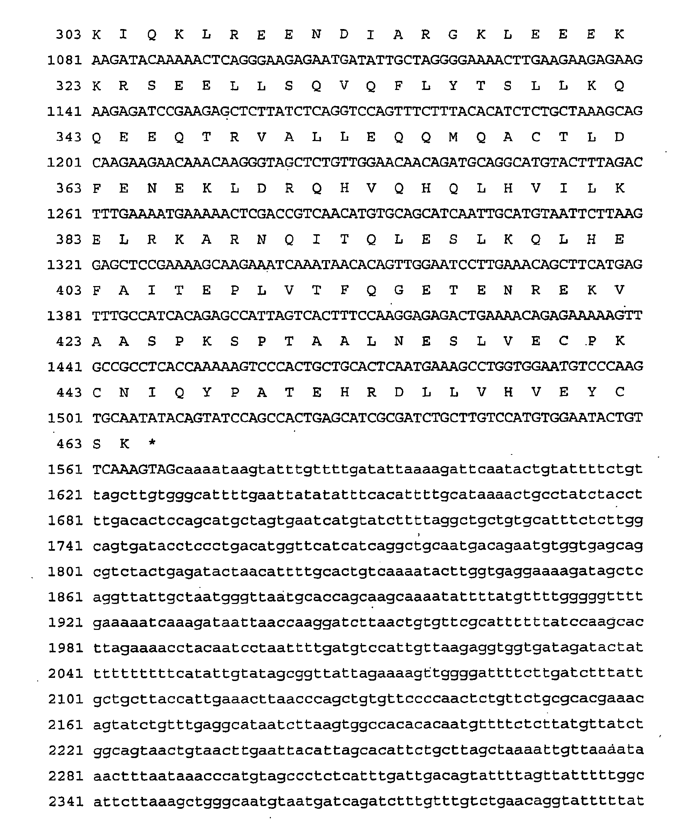

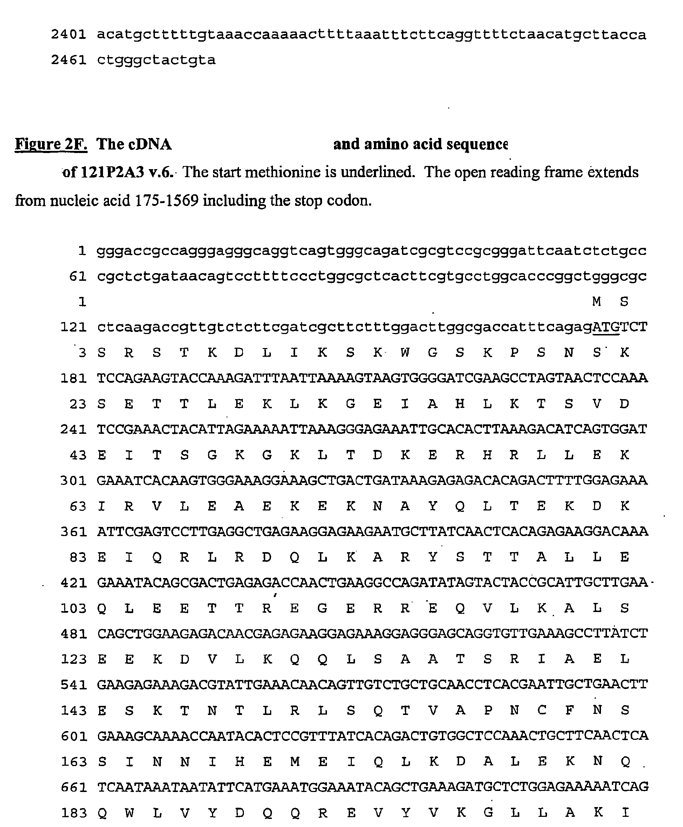

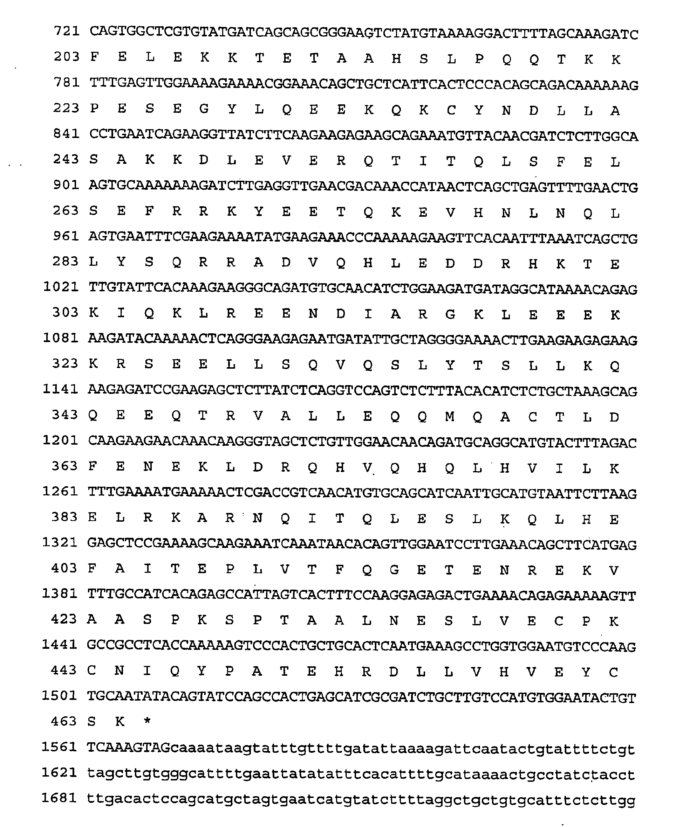

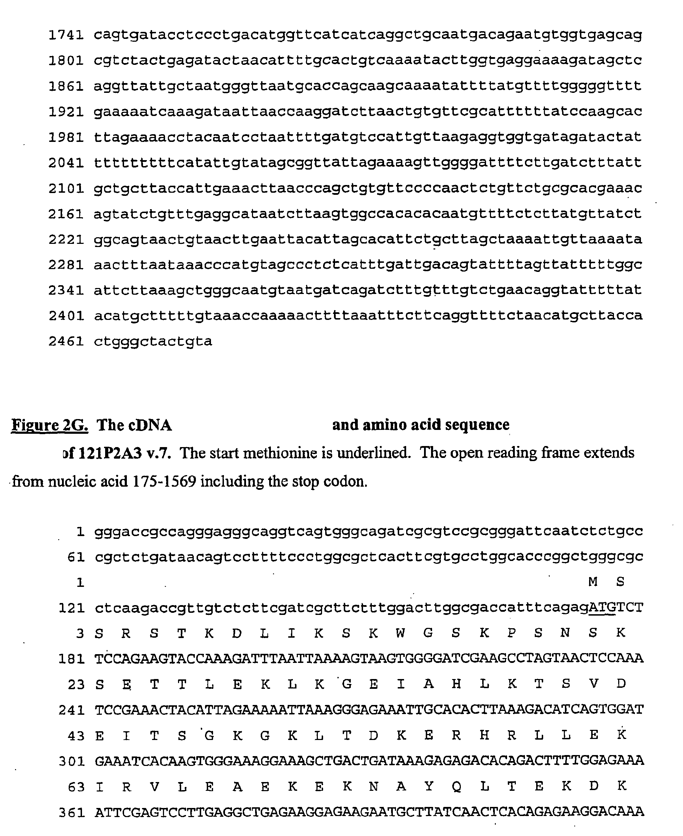

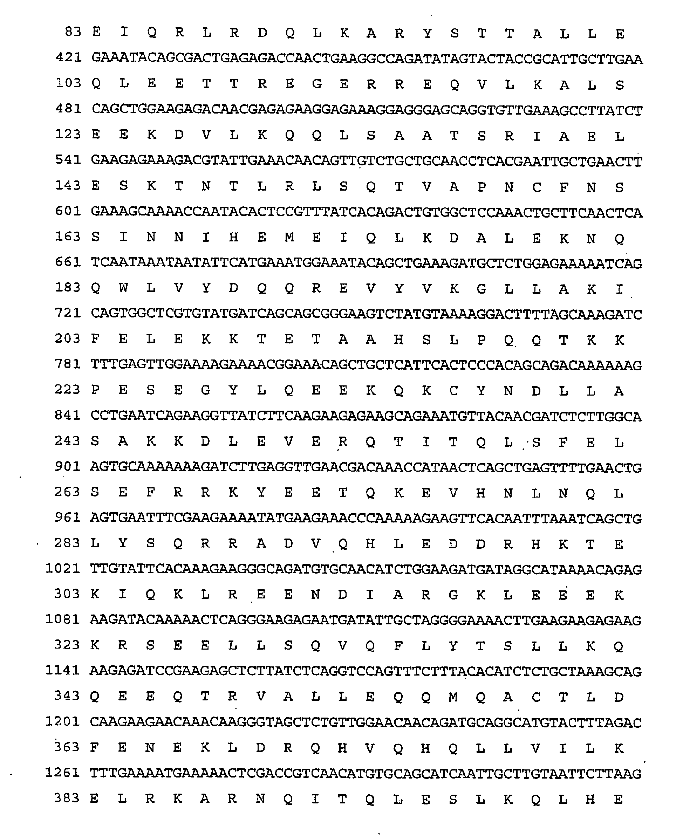

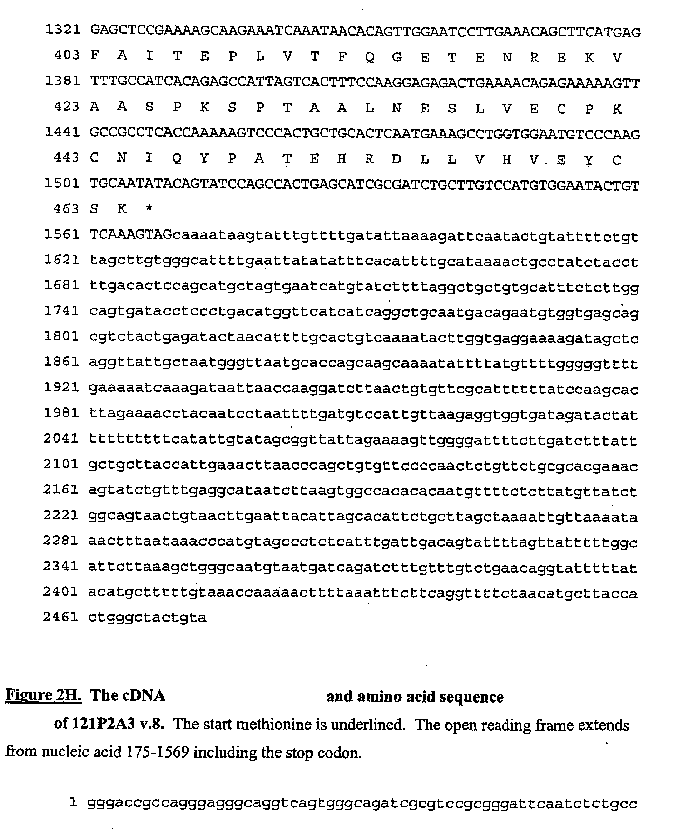

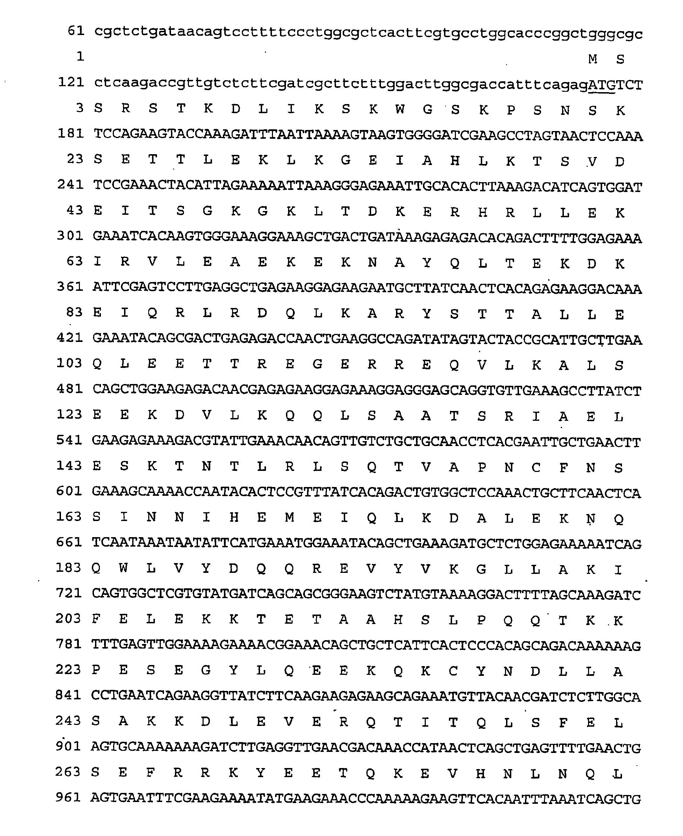

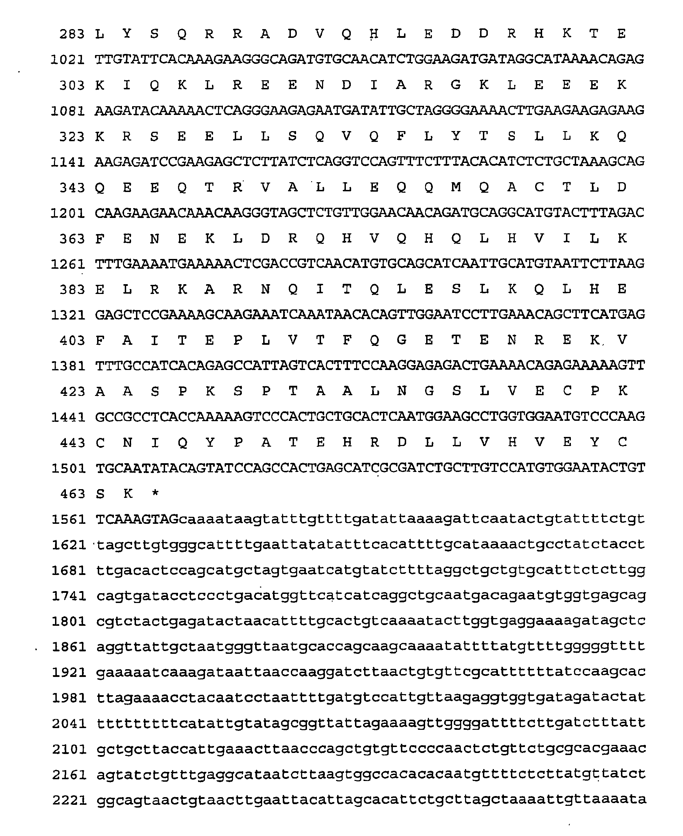

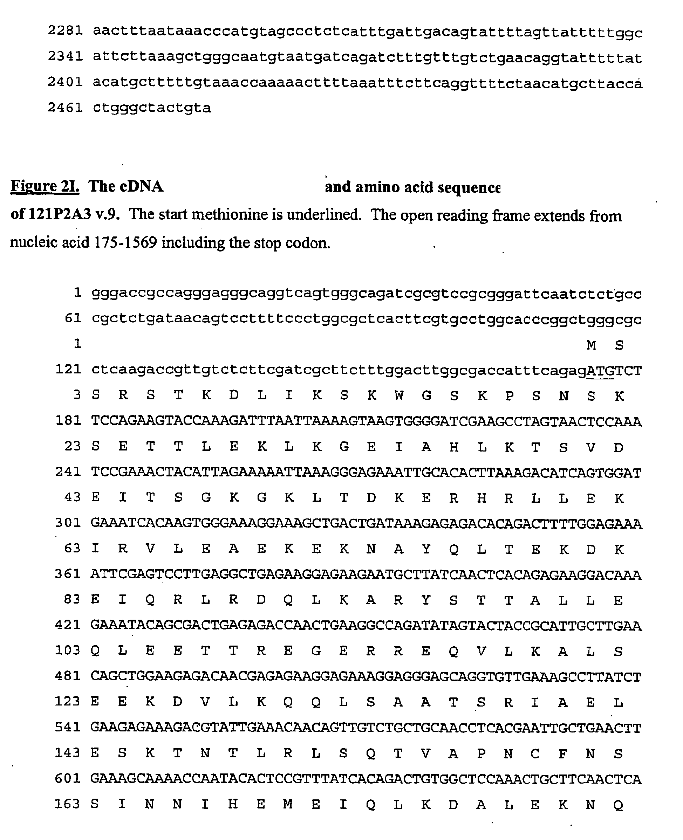

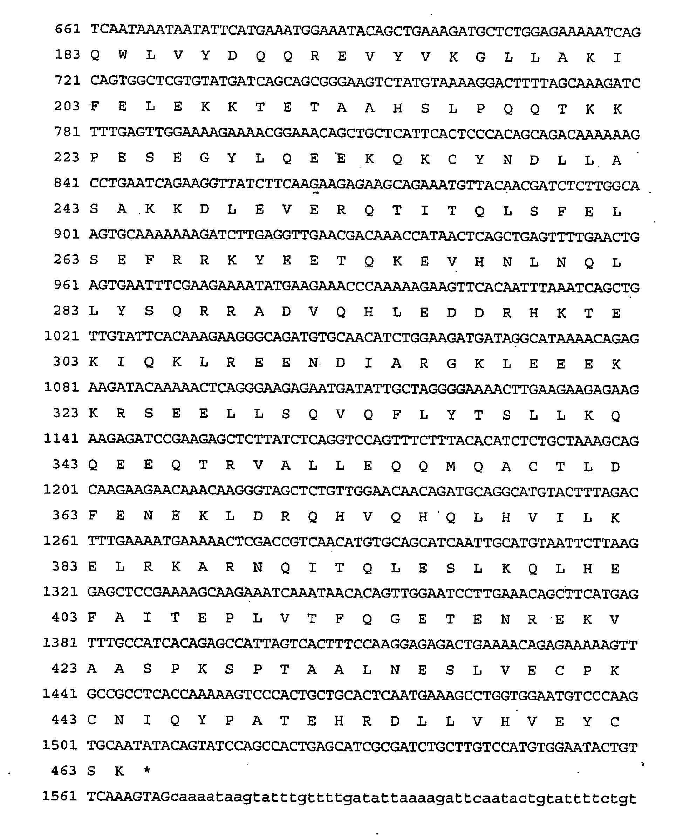

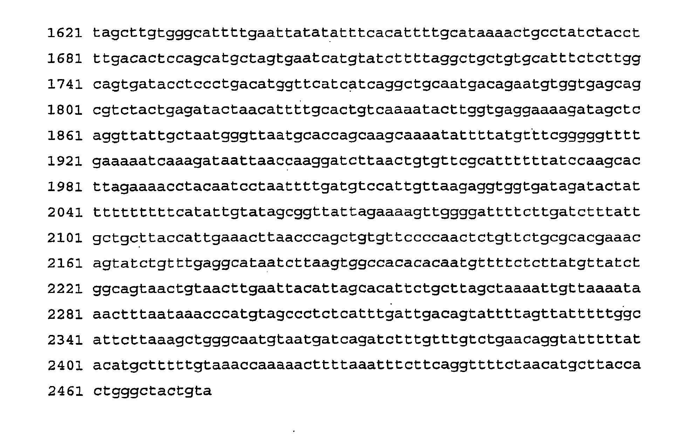

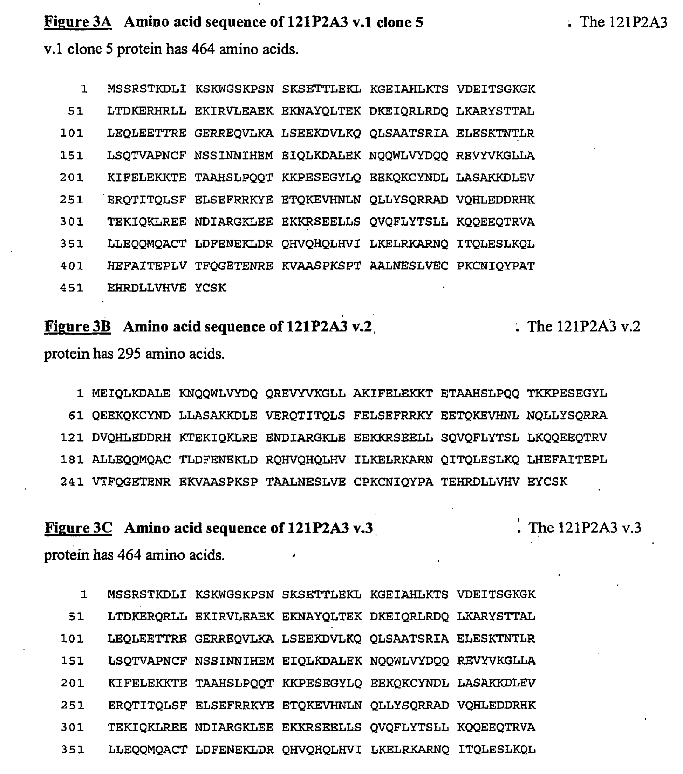

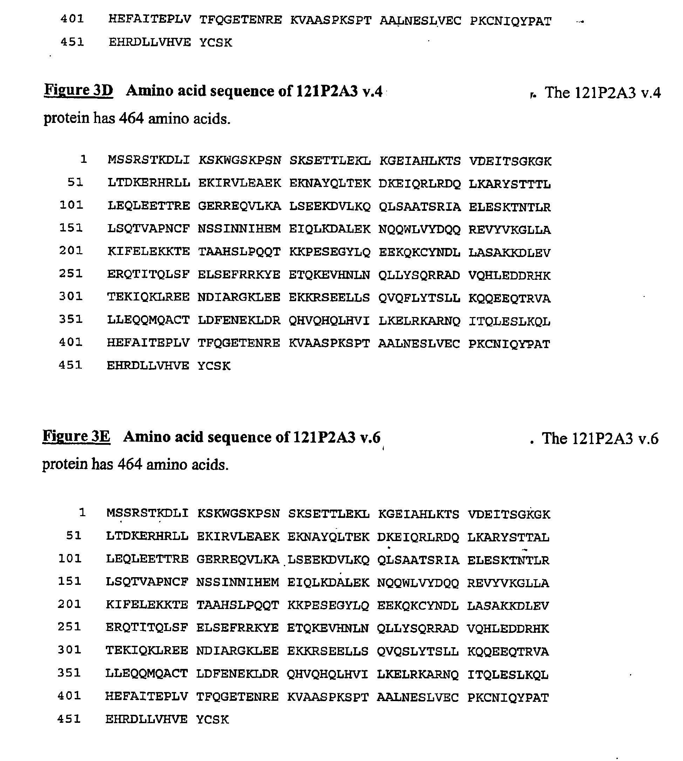

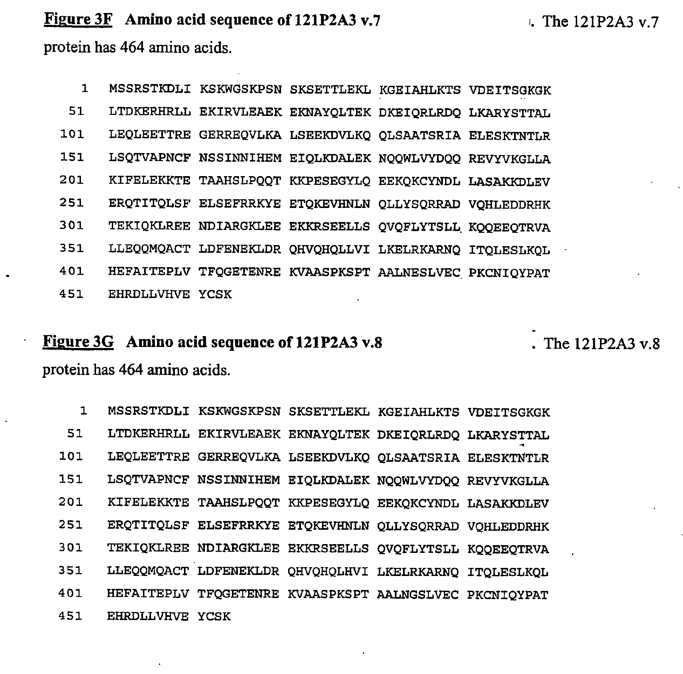

A gene, designated 121P2A3, has now been found to be over-expressed in the cancer(s) listed in Table I. Northern blot expression analysis of 121 P2A3 gene expression in normal tissues shows a restricted expression pattern in adult tissues. The nucleotide (Figure 2) and amino acid (Figure 2, and Figure 3) sequences of 121P2A3 are provided. The tissue-related profile of 121P2A3 in normal adult tissues, combined with the over-expression observed in the tissues listed in Table I, shows that 121P2A3 is aberrantly over-expressed in at least some cancers, and thus serves as a useful diagnostic, prophylactic, prognostic, and/or therapeutic target for cancers of the tissue(s) such as those listed in Table I.

Antibodies that bind to 121P2A3 proteins and polypeptide fragments thereof, including polyclonal and monoclonal antibodies, murine and other mammalian antibodies, chimeric antibodies, humanized and fully human antibodies, and antibodies labelled with a detectable marker are disclosed herein.

The invention provides a method for diagnosing cancer in a test prostate or colon sample, comprising:
contacting the test sample with an antibody or fragment thereof that specifically binds to a protein having an identical amino acid sequence as that shown in Figure 3A, 3B, 3C, 3D, 3E, 3F or 3G and does not bind to a peptide or a protein that has neither (a) an identical amino acid sequence as that shown in Figure 3A, 3B, 3C, 3D, 3E, 3F or 3G nor (b) the sequence of a fragment of a sequence shown in Figure 3A, 3B, 3C, 3D, 3E, 3F or 3G;
determining the level of expression of the protein in the test sample; and
comparing the level so determined to the level of expression of the protein in a corresponding normal sample;
wherein an increased level of expression of the protein in the test sample relative to the normal sample indicates the presence of cancer.

### BRIEF DESCRIPTION OF THE FIGURES

**Figure 1****.** The 121P2A3 SSH sequence of 259 nucleotides.
**Figure 2A****.** The cDNA and amino acid sequence of 121P2A3 v.1 clone 5. The start methionine is underlined. The open reading frame extends from nucleic acid 175-1569 including the stop codon.
**Figure 2B****.** The cDNA and amino acid sequence of 121P2A3 v.2. The start methionine is underlined. The open reading frame extends from nucleic acid 533-1420 including the stop codon.
**Figure 2C****.** The cDNA and amino acid sequence of 121P2A3 v.3, The start methionine is underlined. The open reading frame extends from nucleic acid 175-1569 including the stop codon.
**Figure 2D****.** The cDNA and amino acid sequence of 121P2A3 v.4. The start methionine is underlined. The open reading frame extends from nucleic acid 175-1569 including the stop codon.
**Figure 2E****.** The cDNA and amino acid sequence of 121P2A3 v.5. The start methionine is underlined. The open reading frame extends from nucleic acid 175-1569 including the stop codon.
**Figure 2F****.** The cDNA and amino acid sequence of 121P2A3 v.6. The start methionine is underlined. The open reading frame extends from nucleic acid 175-1569 including the stop codon.
**Figure 2G****_{.}** The cDNA and amino acid sequence of 121P2A3 v.7. The start methionine is underlined. The open reading frame extends from nucleic acid 175-1569 including the stop codon.
**Figure 2H****.** The cDNA and amino acid sequence of 121P2A3 v.8. The start methionine is underlined. The open reading frame extends from nucleic acid 175-1569 including the stop codon.
**Figure 2I****.** The cDNA and amino acid sequence of 121P2A3 v.9. The start methionine is underlined. The open reading frame extends from nucleic acid 175-1569 including the stop codon.

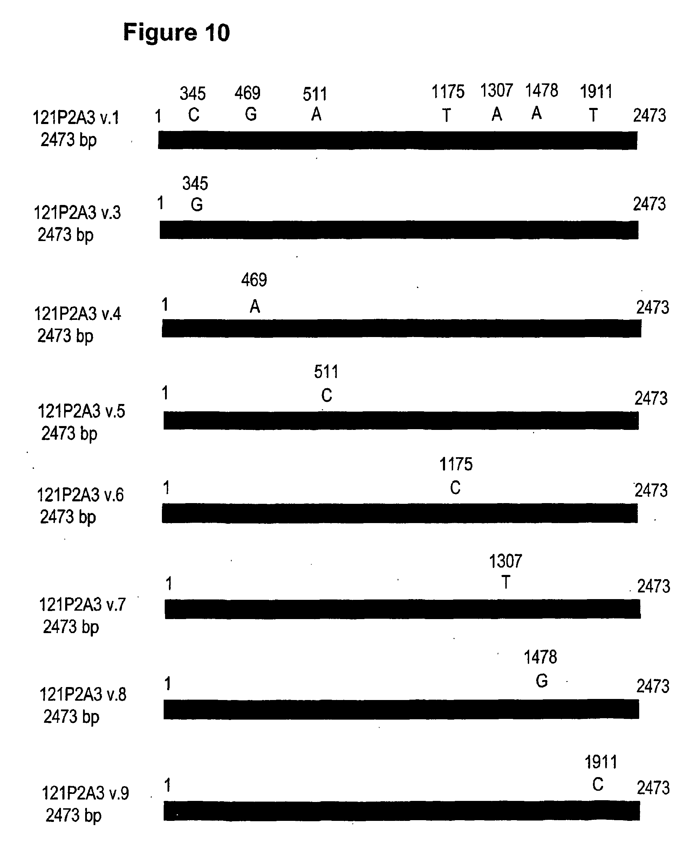

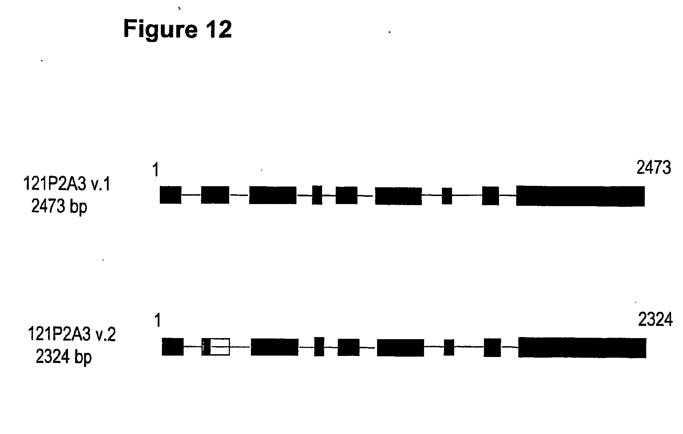

As used herein, a reference to 121P2A3 includes all variants thereof, including those shown in Figure 10 and Figure 12, unless a variant is specified.
**Figure 3A** Amino acid sequence of 121P2A3 v.1 clone 5 the 121P2A3 v.1 clone 5 protein has 464 amino acids.
**Figure 3B** Amino acid sequence of 121P2A3 v.2 The 121P2A3 v.2 protein has 295 amino acids.
**Figure 3C** Amino acid sequence of 121P2A3 v.3. The 121P2A3 v.3 protein has 464 amino acids.
**Figure 3D** Amino acid sequence of 121P2A3 v.4. The 121P2A3 v.4 protein has 464 amino acids.
**Figure 3E** Amino acid sequence of 121P2A3 v.6. The 121P2A3 v.6 protein has 464 amino acids.
**Figure 3F** Amino acid sequence of 121P2A3 v.7. The 121P2A3 v.7 protein has 464 amino acids.
**Figure 3G** Amino acid sequence of 121P2A3 v.8. The 121P2A3 v.8 protein has 464 amino acids.

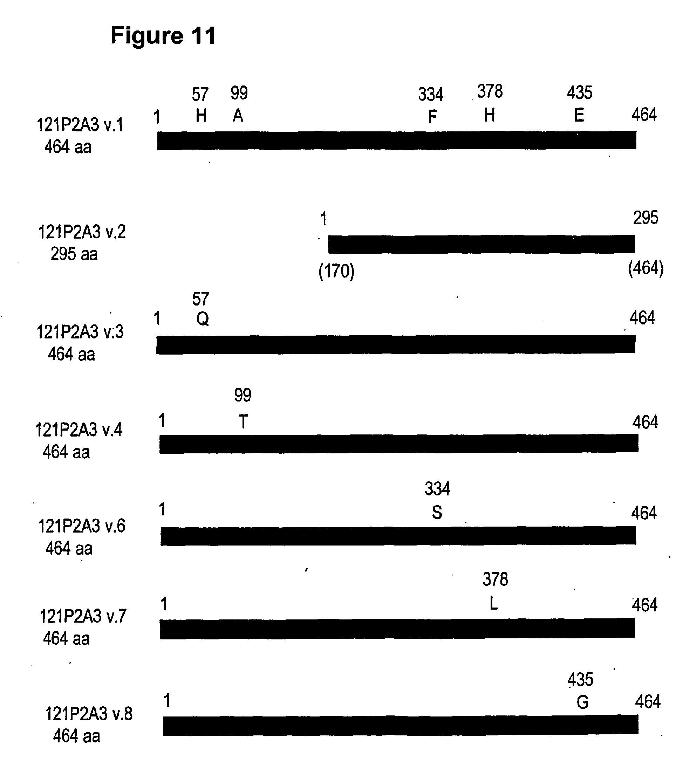

As used herein, a reference to 121P2A3 includes all variants thereof, including those shown in Figure 11, unless a variant is specified.

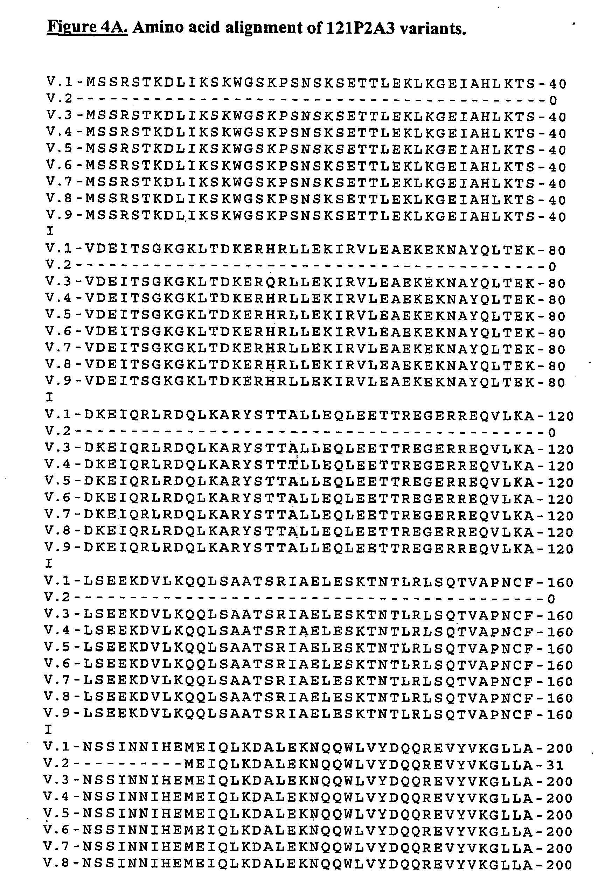

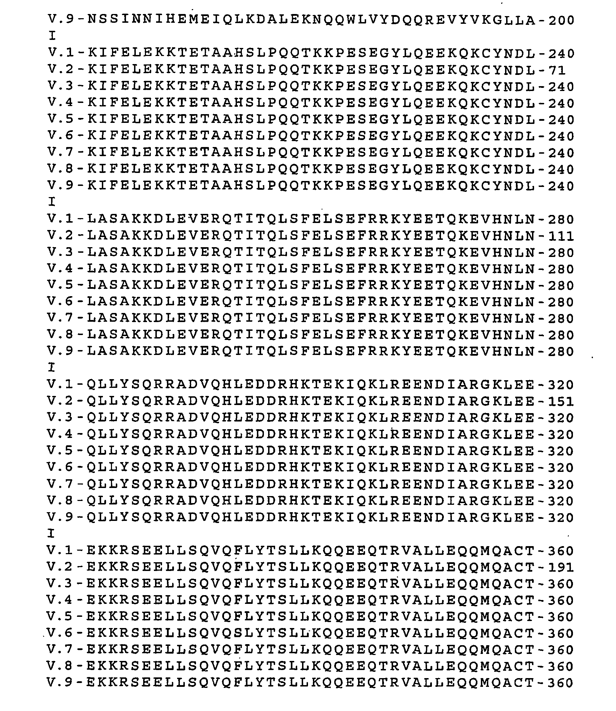

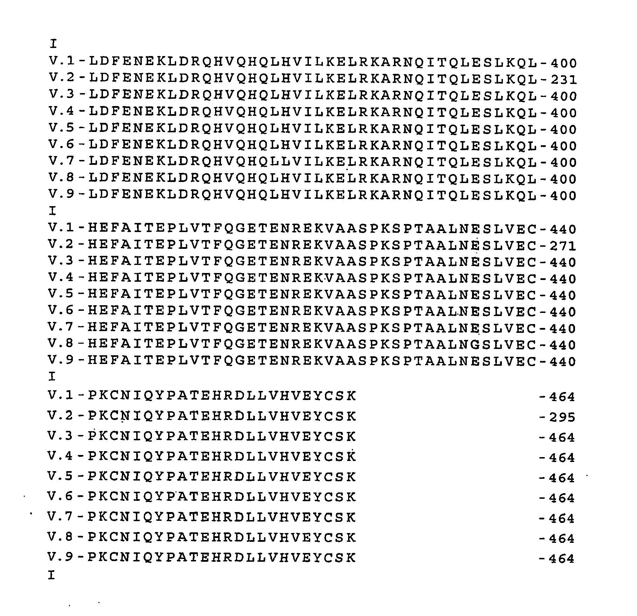

**Figure 4A****.** Amino acid alignment of 121P2A3 variants.

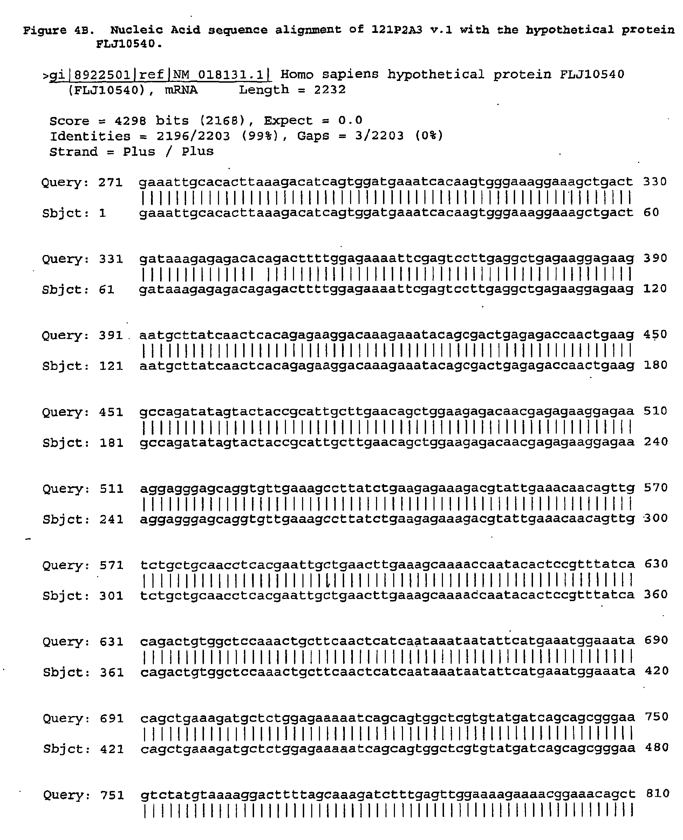

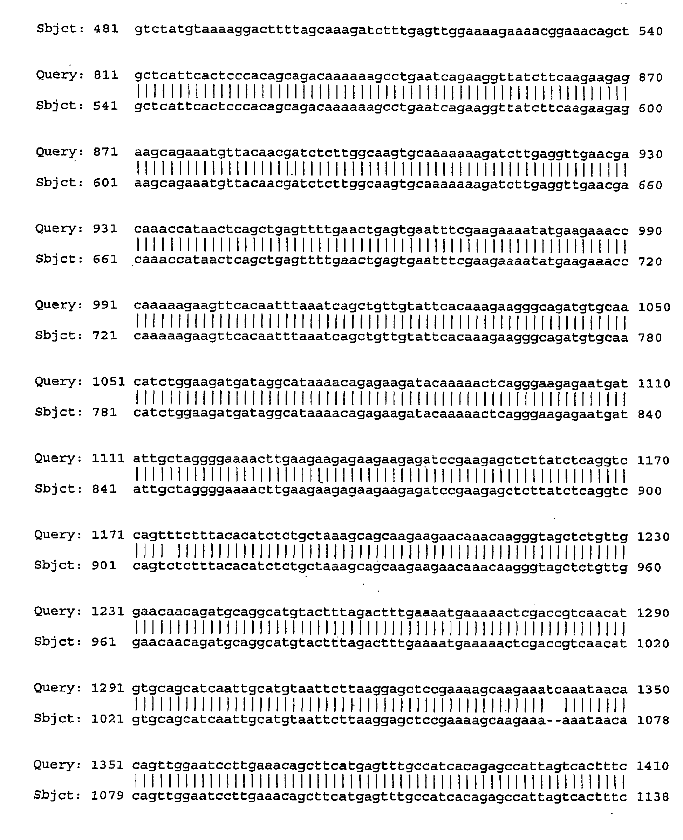

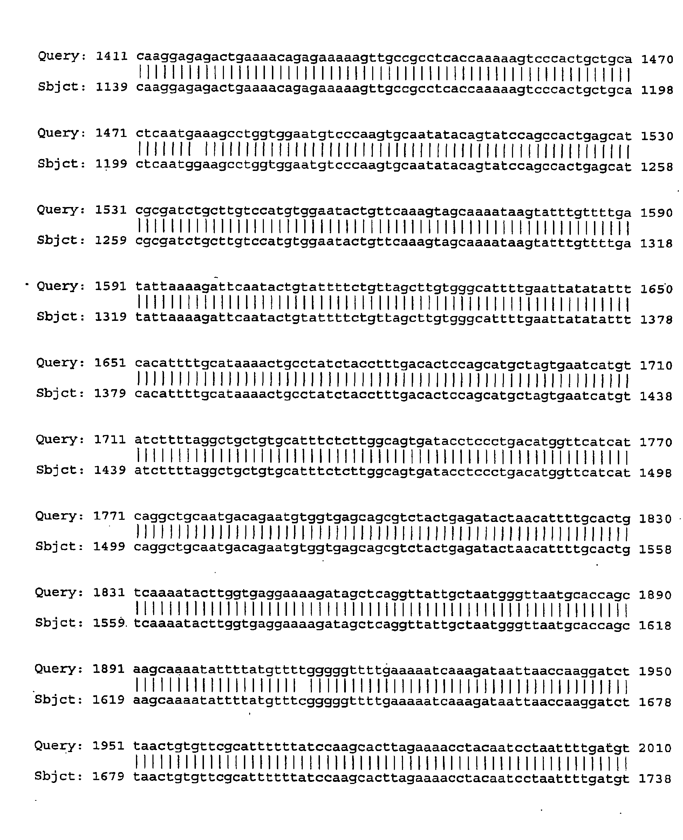

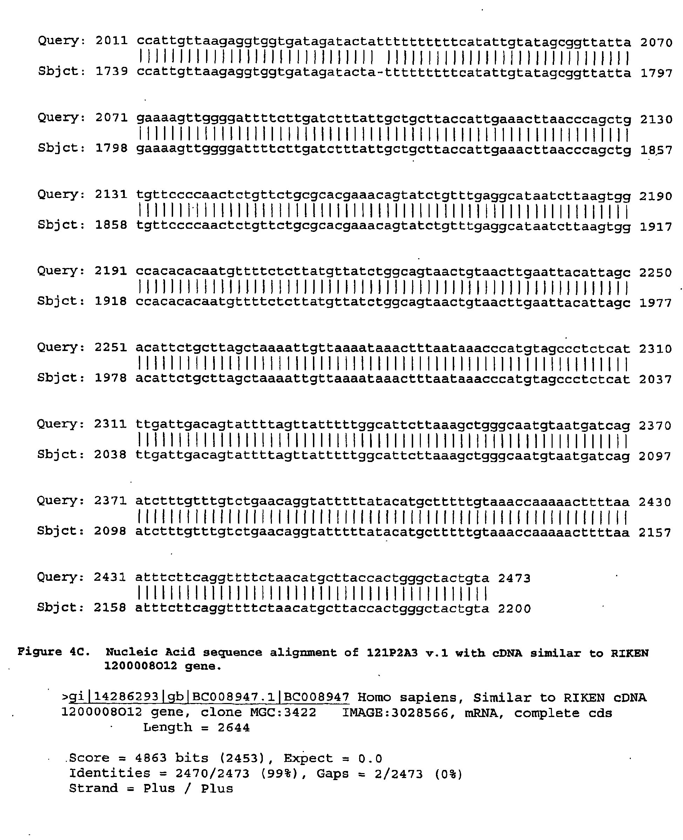

**Figure 4B****.** Nucleic Acid sequence alignment of 121P2A3 v.1 with the hypothetical protein FLJ10540.

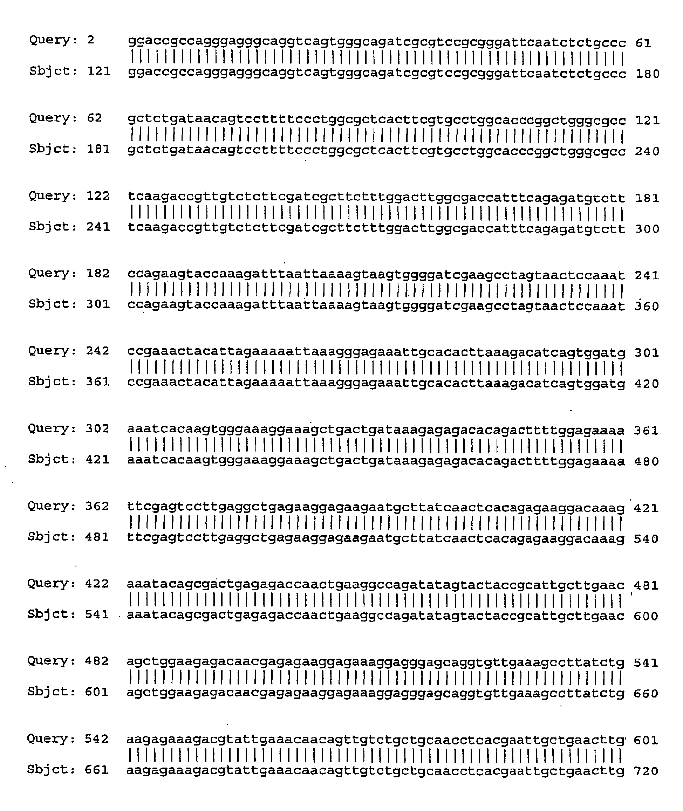

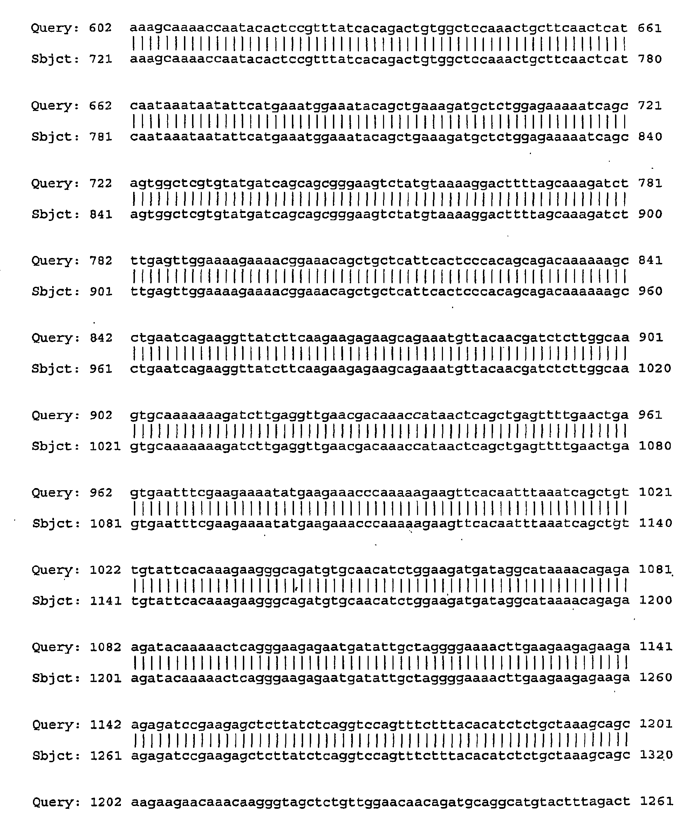

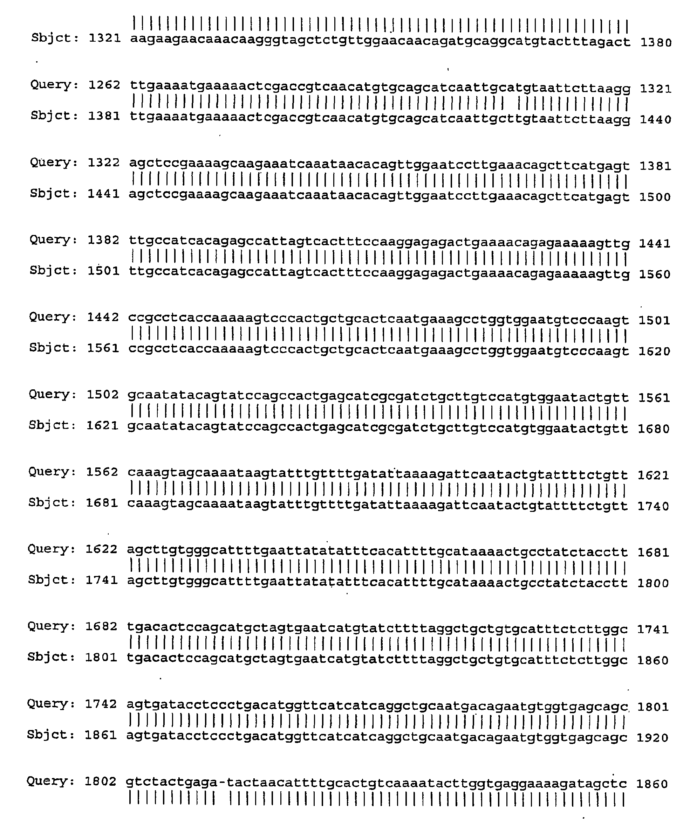

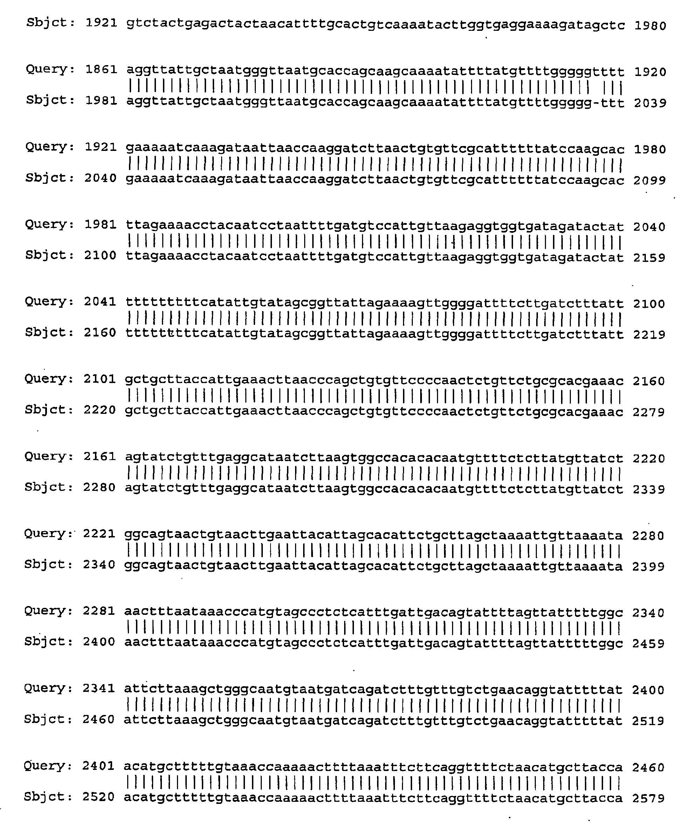

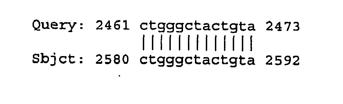

**Figure 4C****.** Nucleic Acid sequence alignment of 121P2A3 v.1 with cDNA similar to RIKEN 1200008O12 gene.

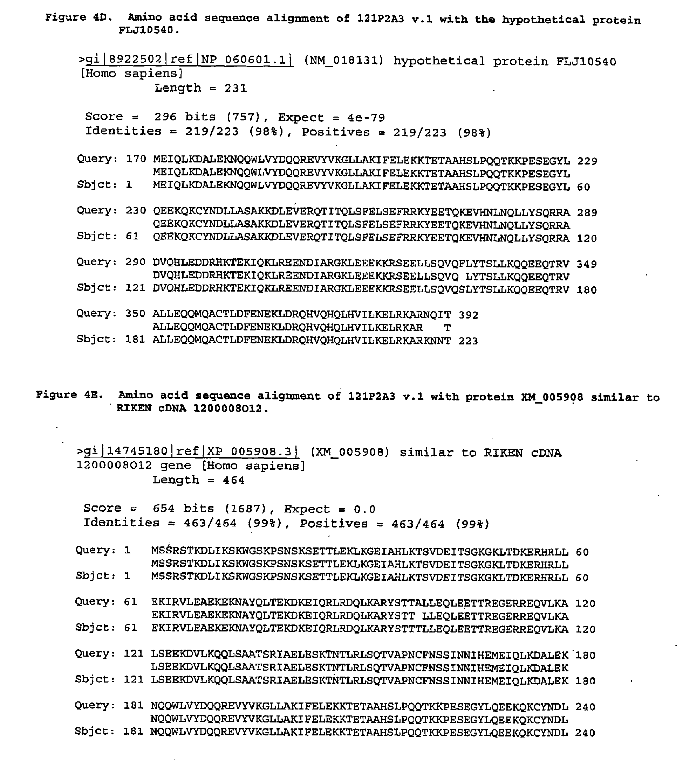

**Figure 4D****.** Amino acid sequence alignment of 121P2A3 v.1 with the hypothetical human protein FLJ10540.

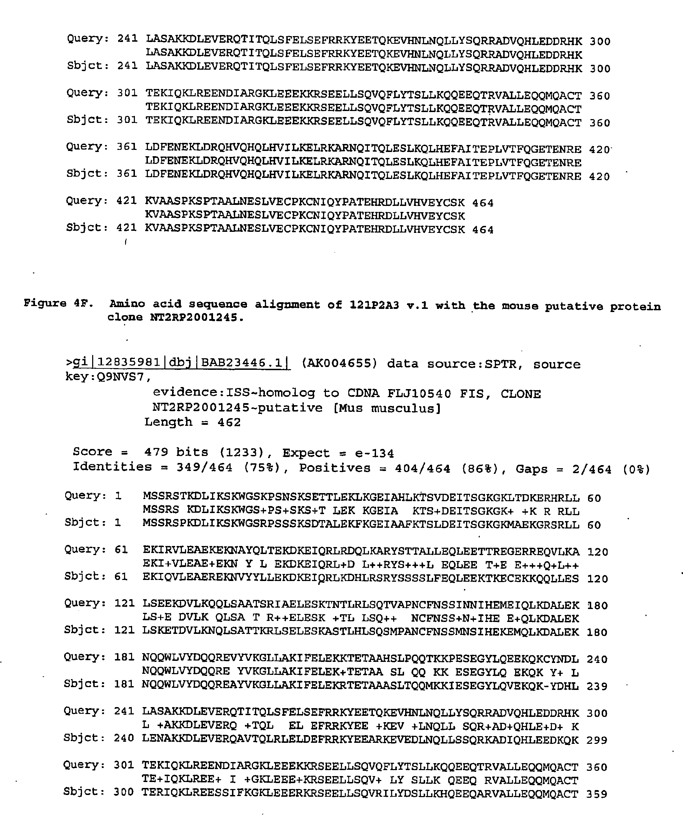

**Figure 4E****.** Amino acid sequence alignment of 121P2A3 v.1 with protein XM_005908 similar to RIKEN cDNA 1200008O12.

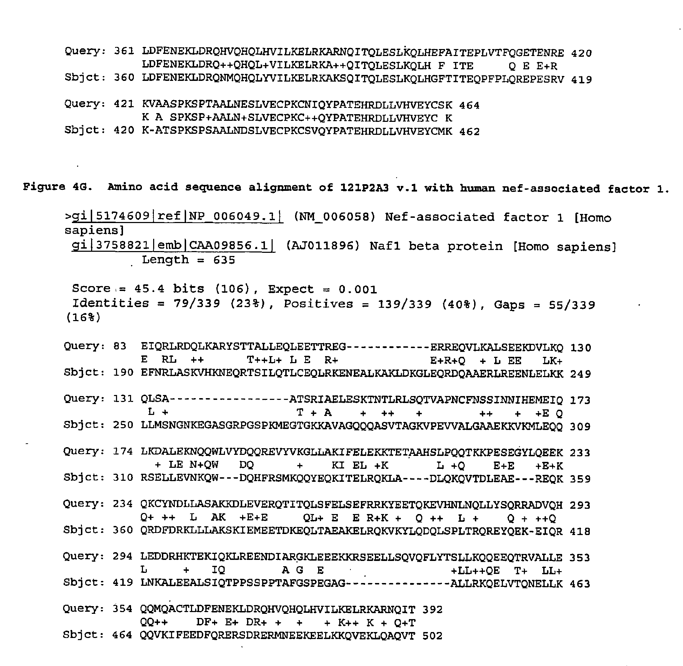

**Figure 4F****.** Amino acid sequence alignment of 121P2A3 v.1 with the mouse putative protein clone NT2RP2001245.
**Figure 4G****.** Amino acid sequence alignment of 121P2A3 v.1 with human nef-associated factor 1.

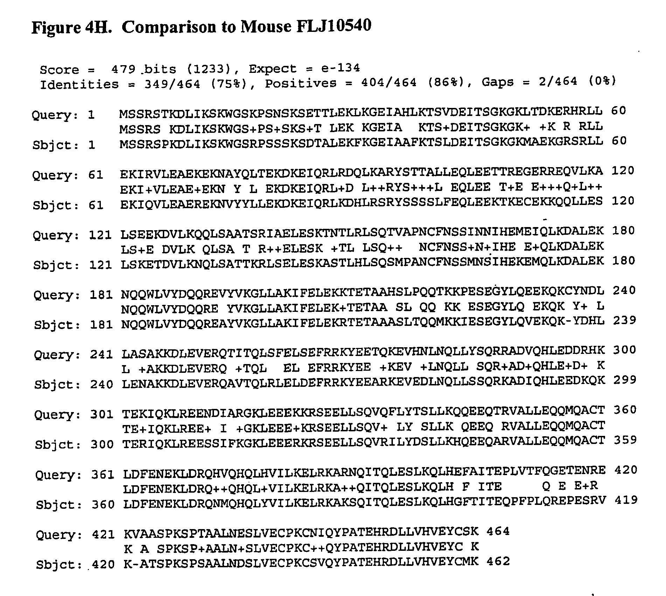

**Figure 4H****.** Amino acid sequence alignment of 121P2A3 v.1 with mouse FLJ10540 protein.

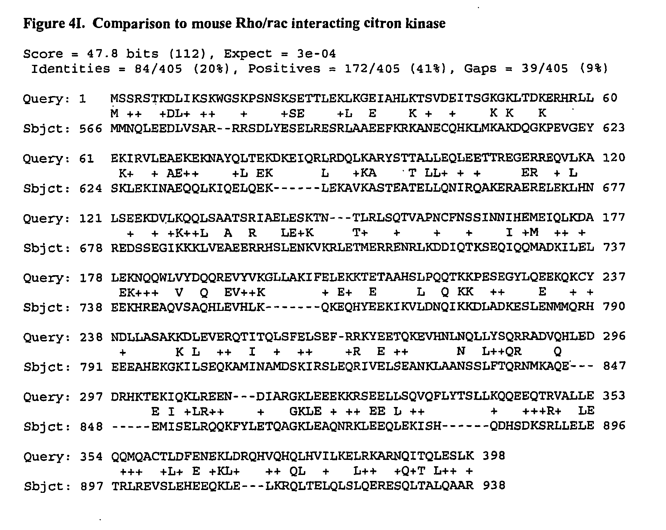

**Figure 4I****.** Amino acid sequence alignment of 121P2A3 v.1 with mouse Rho/rac interacting citron kinase.

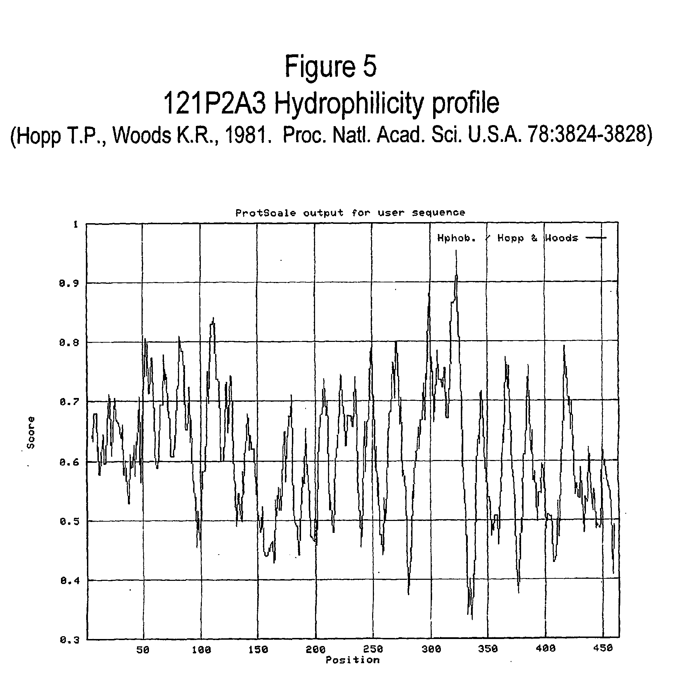

**Figure 5****.** Hydrophilicity amino acid profile of 121P2A3 variant 1, determined by computer algorithm sequence analysis using the method ofHopp and Woods (Hopp T.P., Woods K.R., 1981. Proc. Natl. Acad. Sci. U.S.A. 78:3824-3828) accessed on the Protscale website (www.expasy.ch/cgi-bin/protscale.pl) through the ExPasy molecular biology server.

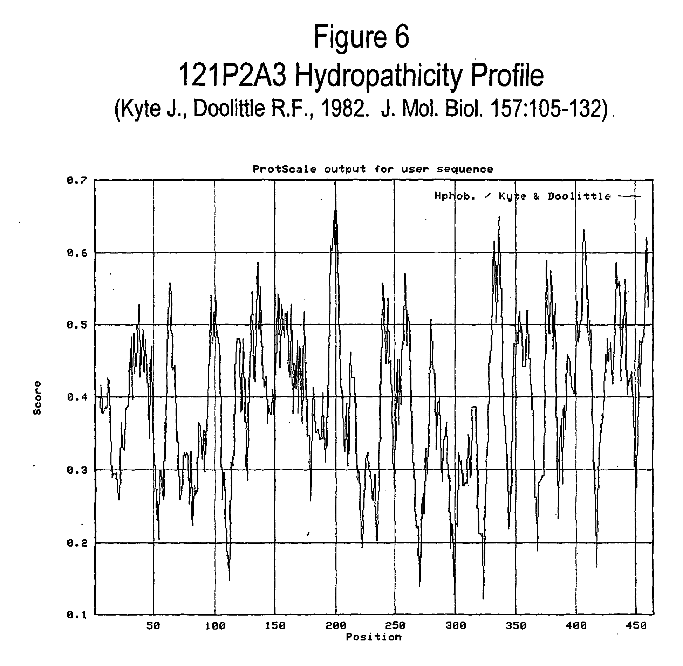

**Figure 6****.** Hydropathicity amino acid profile of 121P2A3 variant 1, determined by computer algorithm sequence analysis using the method of Kyte and Doolittle (Kyte J., Doolittle R.F., 1982. J. Mol. Biol. 157:105-132) accessed on the ProtScale website (www.expasy.ch/cgi-bin/protscale.pl) through the ExPasy molecular biology server.

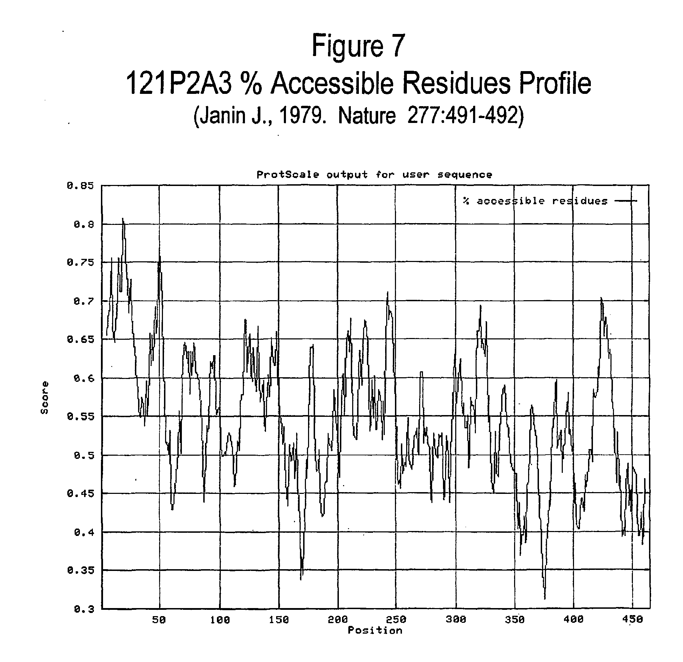

**Figure 7****.** Percent accessible residues amino acid profile of 121P2A3 variant 1, determined by computer algorithm sequence analysis using the method of Janin (Janin J., 1979 Nature 277:491-492) accessed on the ProtScale website (www.expasy.ch/cgi-bin/protscale.pl) through the ExPasy molecular biology server.

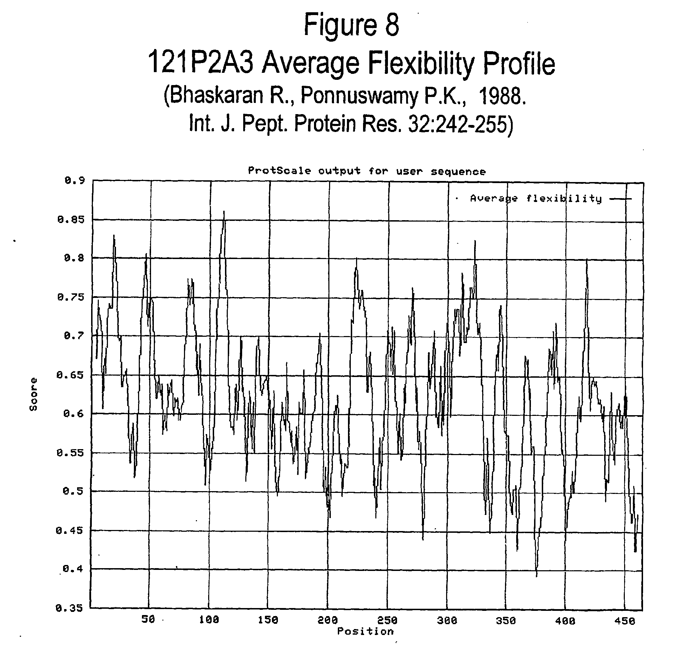

**Figure 8****.** Average flexibility amino acid profile of 121P2A3 variant 1, determined by computer algorithm sequence analysis using the method of Bhaskaran and Ponnuswamy (Bhaskaran R., and Ponnuswamy P.K., 1988. Int. J. Pept. Protein Res. 32:242-255) accessed on the ProtScale website (www.expasy.ch/cgi-bin/protscale.pl) through the ExPasy molecular biology server.

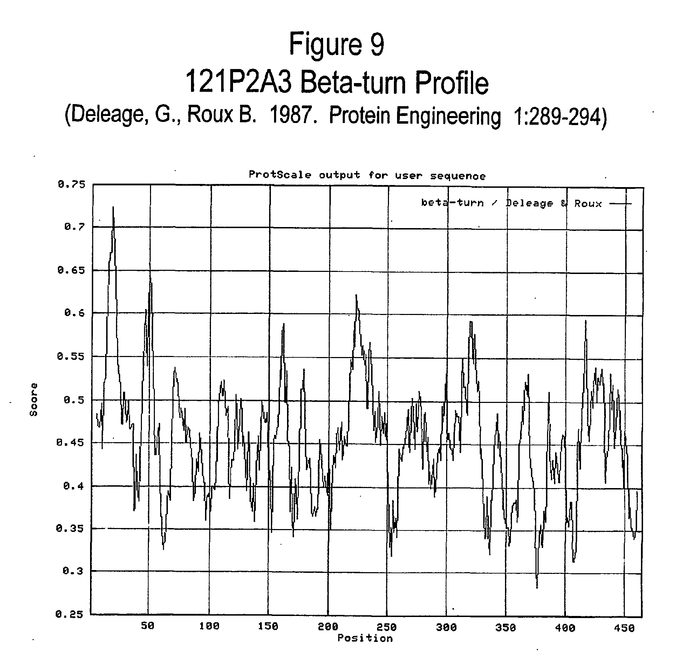

**Figure 9****.** Beta-turn amino acid profile of 121P2A3 variant 1, determined by computer algorithm sequence analysis using the method of Deleage and Roux (Deleage, G., Roux B. 1987 Protein Engineering 1:289-294) accessed on the ProtScale website (www.expasy.ch/cgi-bin/protscale.pl) through the ExPasy molecular biology server.
**Figure 10****.** Schematic alignment of SNP variants of 121P2A3. Variants 121P2A3 v.3 through v.9 are variants with single nucleotide differences. Though these SNP variants are shown separately, they could also occur in any combinations and in any one of the transcript variants that contains the base pairs. Numbers correspond to those of 121P2A3 v.1. The black boxes show the same sequence as 121P2A3 v.1. SNPs are indicated above the box.
**Figure 11****.** Schematic alignment of protein variants of 121P2A3. Protein variants correspond to nucleotide variants. Nucleotide variants 121P2A3 v.5 and v.9 in Figure 10 code for the same protein as 121P2A3 v.1. Black boxes represent the same sequence as 121P2A3 v.1. Single amino acid differences were indicated above the boxes. Numbers in "( )" underneath the box correspond to 121P2A3 v.1.
**Figure 12****.** Exon compositions of transcript variants of 121P2A3. Variant 121P2A3 v.2 is a splice variant whose exon 2 is 149 bp shorter than exon 2 of 121P2A3 v.1. Empty (white) box shows the portion of exon 2 in 121P2A3 v.1 but not in 121P2A3 v.2. Black boxes show the same sequence as 121P2A3 v.1. Numbers correspond to those of 121P2A3 v.1. Length of introns are not proportional.

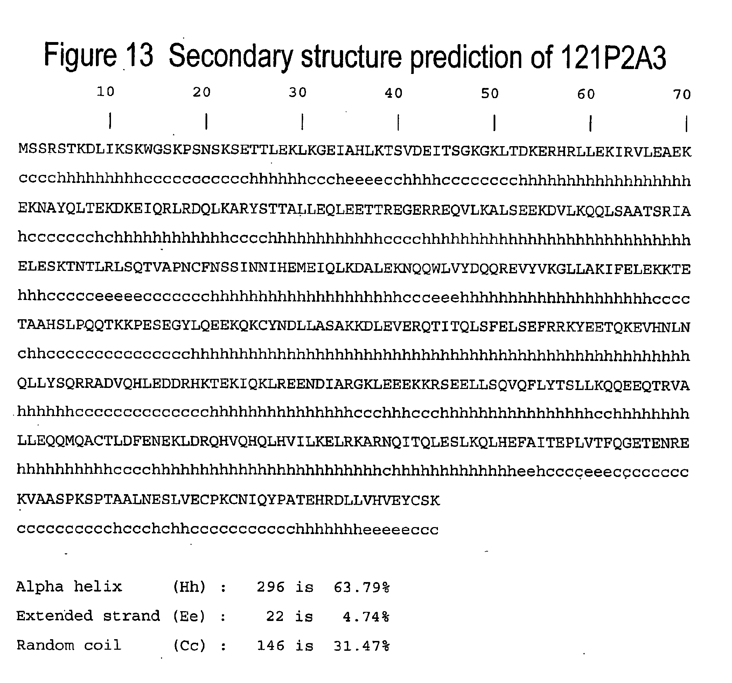

**Figure 13****.** Secondary structure prediction for 121P2A3 protein. The secondary structure of 121P2A3 protein was predicted using the HNN - Hierarchical Neural Network method (Guermeur, 1997, URL pbil.ibcp.fr/cgi-bin/npsa_automat.pl?page=npsa_nn.html), accessed from the ExPasy molecular biology server (URL www.expasy.ch/tools/). This method predicts the presence and location of alpha helices, extended strands, and random coils from the primary protein sequence. The percent of the protein in a given secondary structure is also listed.

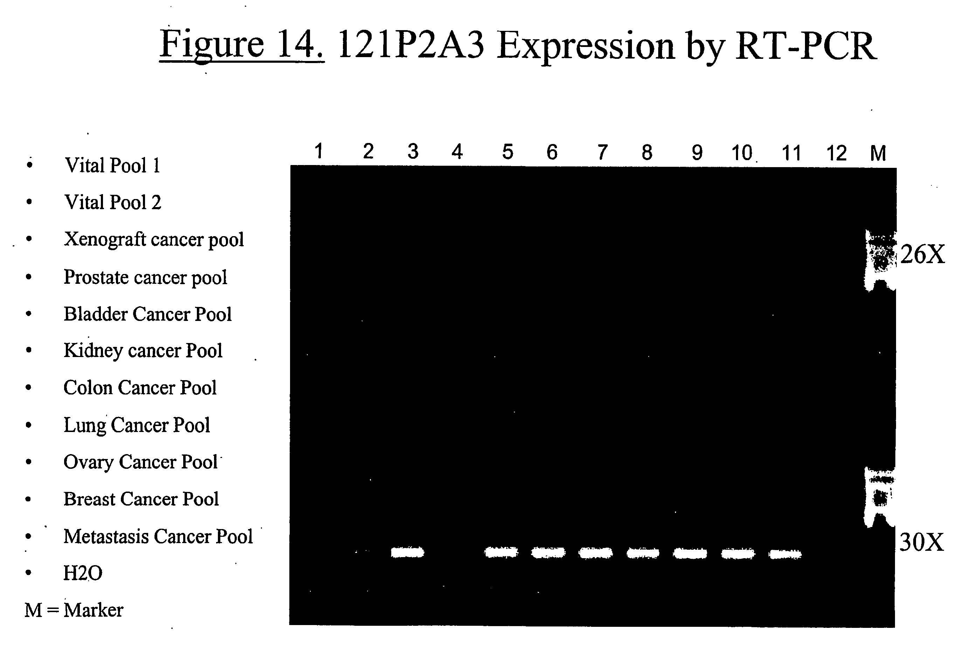

**Figure 14****.** Expression of 121P2A3 by RT-PCR. First strand cDNA was prepared from vital pool 1 (liver, lung and kidney), vital pool 2 (pancreas, colon and stomach), LAPC xenograft pool (LAPC-4AD, LAPC-4AI, LAPC-9AD and LAPC-9AI), prostate cancer pool, bladder cancer pool, kidney cancer pool, colon cancer pool, lung cancer pool, ovary cancer pool, breast cancer pool, and cancer metastasis pool. Normalization was performed by PCR using primers to actin and GAPDH. Semi-quantitative PCR, using primers to 121P2A3, was performed at 26 and 30 cycles of amplification. Results show strong expression of 121P2A3 in LAPC xenograft pool, bladder cancer pool, kidney cancer pool, colon cancer pool, lung cancer pool, ovary cancer pool, breast cancer pool, and cancer metastasis pool. Expression of 121P2A3 was also detected in prostate cancer pool. Very low expression was detected in vital pool 1 and 2.

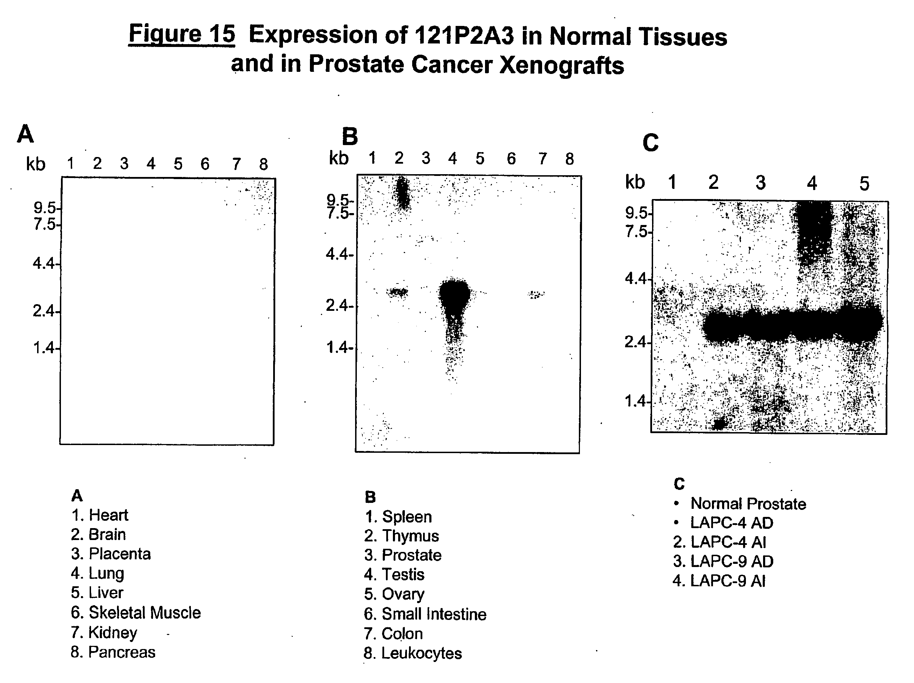

**Figure 15****.** Expression of 121P2A3 in normal tissues. Two multiple tissue northern blots (A and B; Clontech) both with 2 ug of mRNA/lane, and a LAPC xenograft blot both with 10 ug of total RNA/lane (C) were probed with the 121P2A3 SSH sequence. Size standards in kilobases (kb) are indicated on the side. Results show expression of an approximately 2.7 kb121P2A3 transcript in testis. Lower level expression was also detected in thymus and colon, but not in the other normal tissues tested. 121P2A3 expression was strongly demonstrated in all 4 LAPC prostate xenograft tissues but not in normal prostate.

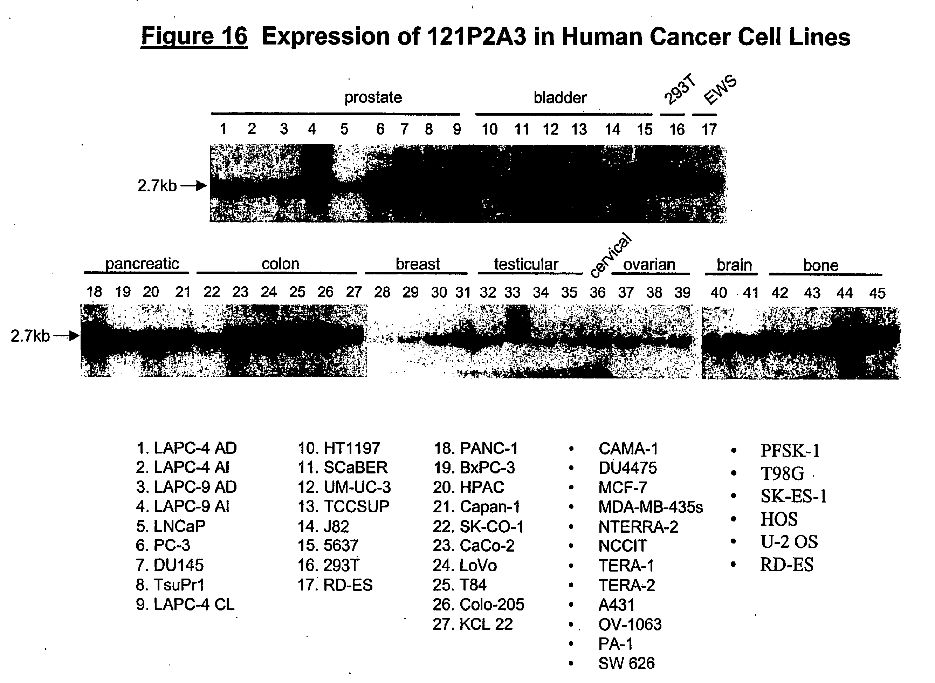

**Figure 16****.** Expression of 121P2A3 in human cancer cell lines. RNA was extracted from a number of human cancer cell lines. Northern blots with 10 ug of total RNA/lane were probed with the 121P2A3 SSH fragment. Size standards in kilobases (kb) are indicated on the side. Results show expression of 121P2A3 in all the cell lines tested.

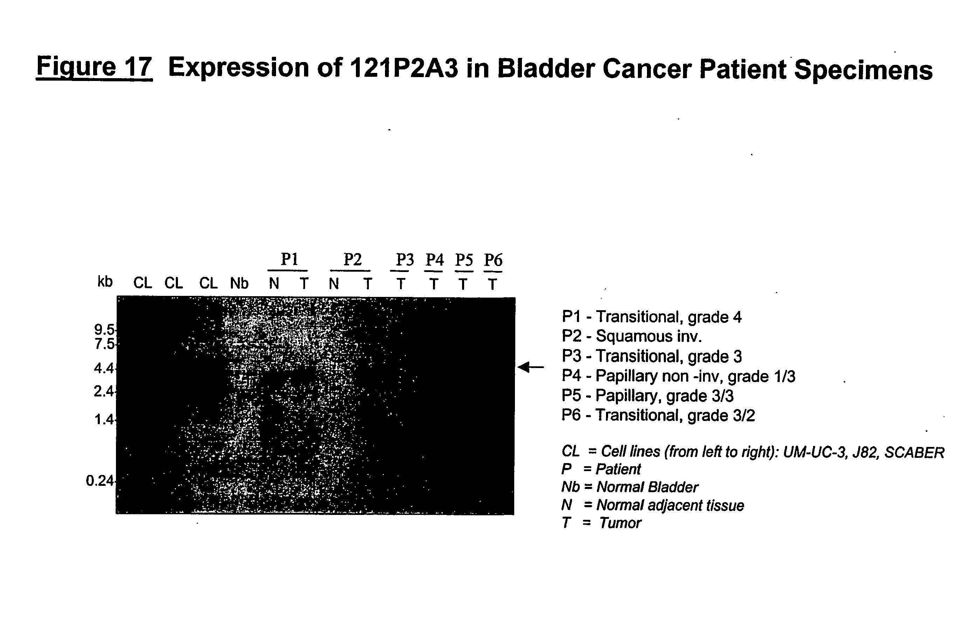

**Figure 17****.** Expression of 121P2A3 in bladder cancer patient tissues. RNA was extracted from normal bladder (Nb), bladder cancer cell lines (CL; UM-UC-3, J82, SCaBER), bladder cancer patient tumors (T) and normal adjacent tissue (N). Northern blots with 10 ug of total RNA were probed with the 121P2A3 SSH sequence. Size standards in kilobases are indicated on the side. Results show expression of 121P2A3 in patient bladder cancer tissues, and in all bladder cancer cell lines tested, but not in normal bladder.

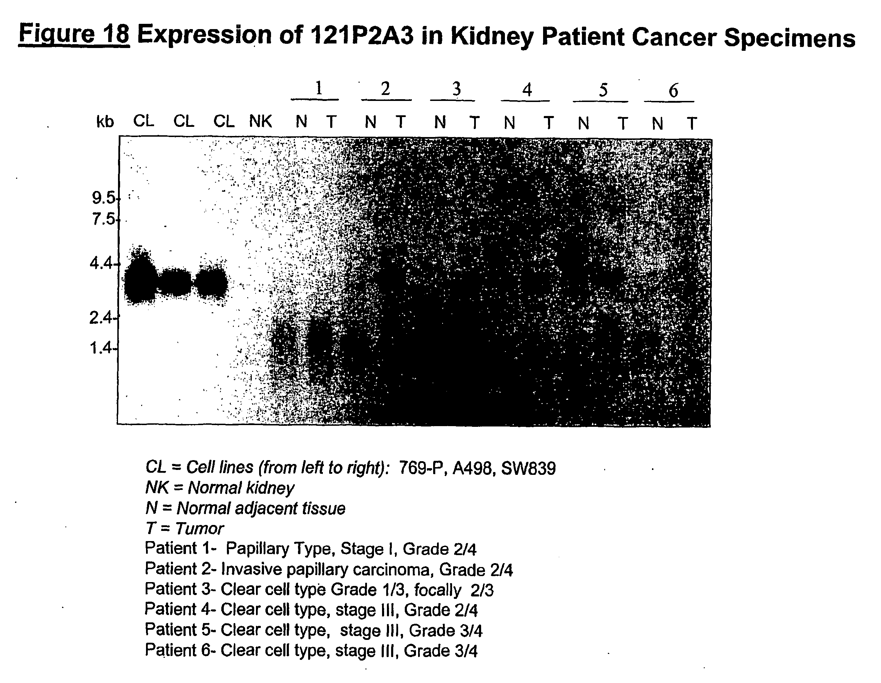

**Figure 18****.** Expression of 121P2A3 in kidney cancer patient tissues. RNA was extracted from kidney cancer cell lines (CL: 769-P, A498, SW839), normal kidney (NK), kidney cancer patient tumors (T) and their normal adjacent tissues (N). Northern blots with 10 ug of total RNA were probed with the 121P2A3 SSH sequence. Size standards in kilobases are on the side. Results show expression of 121P2A3 in patient kidney tumor tissues and in all kidney cancer cell lines tested, but not in normal kidney.

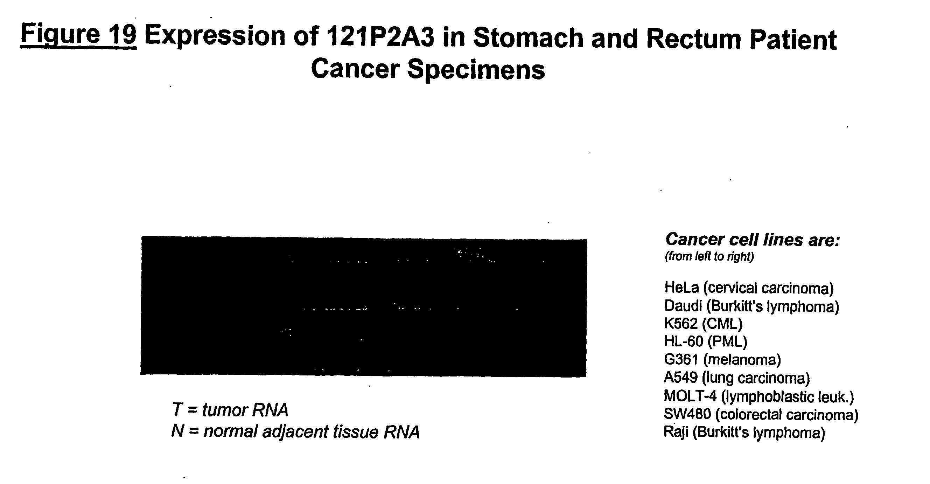

**Figure 19****.** Expression of 121P2A3 in stomach and rectum human cancer specimens. Expression of 121P2A3 was assayed in a panel of human stomach and rectum cancers (T) and their respective matched normal tissues (N) on RNA dot blots, and in human cancer cell lines. 121P2A3 expression was seen in both stomach and rectum cancers. The expression detected in normal adjacent tissues (isolated from diseased tissues) but not in normal tissues (isolated from healthy donors) may indicate that these tissues are not fully normal and that 121P2A3 may be expressed in early stage tumors. 121P2A3 was also found to be highly expressed in the following cancer cell lines; HeLa, Daudi, K562, HL-60, G361, A549, MOLT-4, SW480, and Raji.

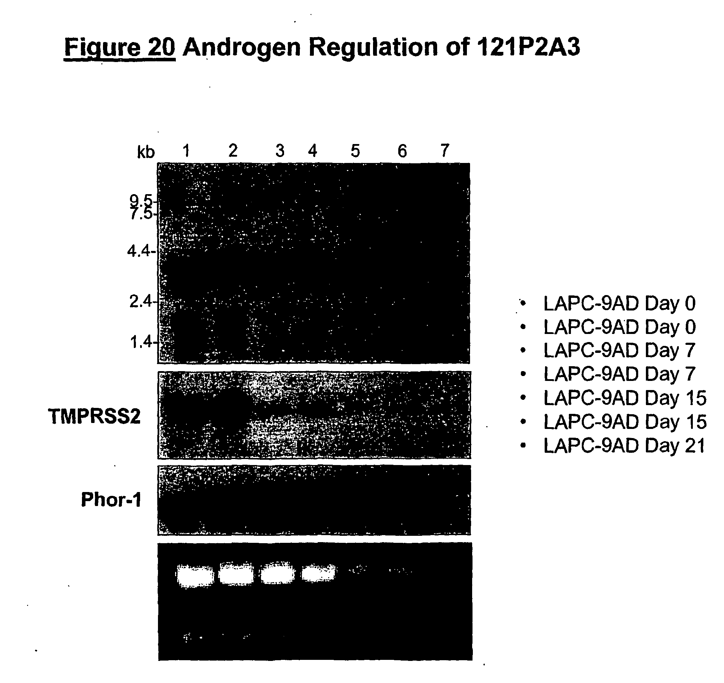

**Figure 20****.** Androgen regulation of 121P2A3. Male mice were injected with LAPC-9AD tumor cells. When tumor reached a palpable size (0.3-0.5cm in diameter), mice were castrated and tumors harvested at different time points following castration. RNA was isolated from the xenograft tissues. Northern blots with 10 ug of total RNA/lane were probed with the 121P2A3 SSH fragment. Size standards in kilobases (kb) are indicated on the side. Results show expression of 121P2A3 is downregulated within 7 days of castration. The experimental samples were confirmed by testing for the expression of the androgen-regulated prostate cancer gene TMPRSS2 and the androgen-independent gene PHOR-1 (B). This experiment shows that, as expected, TMPRSS2 expression level goes down 7 days after castration, whereas the expression of PHOR-1 does not change. A picture of the ethidium-bromide staining of the RNA gel is also presented confirming the quality of the RNA.

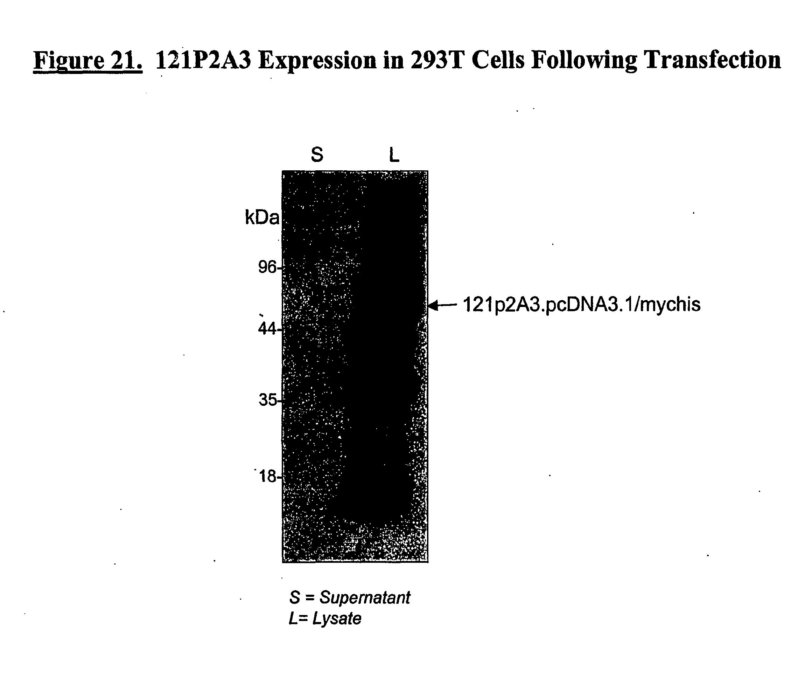

**Figure 21****.** 121P2A3 expression in 293T cells following transfection. 293T cells were transfected with 121P2A3 pcDNA3.1/mychis. Forty hours later, cell lysates (L) and supernatant (S) were collected. Samples were run on an SDS-PAGE acrylamide gel, blotted and stained with anti-his antibody. The blot was developed using the ECL chemiluminescence kit and visualized by autoradiography. Results show expression of the expected 54kDa molecular weight 121P2A3 from the 121P2A3 pcDNA3.1/mychis mammalian expression construct in the lysates of 121P2A3.pcDNA3.1/mychis transfected cells, but not in the supernatant.

### DETAILED DESCRIPTION OF THE INVENTION

### Outline of Sections

I.) Definitions
II.) 121P2A3-related Proteins
   II.A.) Expression of 121P2A3-related Proteins
   II.B.) Uses of 121P2A3-related Proteins
III.) 121P2A3 Antibodies
IV.) Methods for the Detection of 121P2A3
V.) Methods for Monitoring the Status of 121P2A3 Proteins
VII.) KITS

### I.) Definitions:

Unless otherwise defined all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

The terms "advanced prostate cancer", "locally advanced prostate cancer", "advanced disease" and "locally advanced disease" mean prostate cancers that have extended through the prostate capsule, and are meant to include stage C disease under the American Urological Association (AUA) system, stage C1- C2 disease under the Whitmore-Jewett system, and stage T3 - T4 and N+ disease under the TNM (tumor, node, metastasis) system. In general, surgery is not recommended for patients with locally advanced disease, and these patients have substantially less favorable outcomes compared to patients having clinically localized (organ-confined) prostate cancer. Locally advanced disease is clinically identified by palpable evidence of induration beyond the lateral border of the prostate, or asymmetry or induration above the prostate base. Locally advanced prostate cancer is presently diagnosed pathologically following radical prostatectomy if the tumor invades or penetrates the prostatic capsule, extends into the surgical margin, or invades the seminal vesicles.

The term "analog" refers to a molecule which is structurally similar or shares similar or corresponding attributes with another molecule (e.g. a 121P2A3-related protein). For example an analog of a 121P2A3 protein can be specifically bound by an antibody or T cell that specifically binds to 121P2A3.

The term "antibody" is used in the broadest sense. Therefore an "antibody" can be naturally occuring or man-made such as monoclonal antibodies produced by conventional hybridoma technology. Anti-121P2A3 antibodies comprise monoclonal and polyclonal antibodies as well as fragments containing the antigen-binding domain and/or one or more complementarity determining regions of these antibodies.

An "antibody fragment" is defined as at least a portion of the variable region of the immunoglobulin molecule that binds to its target, i.e., the antigen-binding region. In one embodiment if specifically covers single anti-121P2A3 antibodies and clones thereof (including agonist, antagonist and neutralizing antibodies) and anti-121P2A3 antibody compositions with polyepitopic specificity.

The term "homolog" refers to a molecule which exhibits homology to another molecule, by for example, having sequences of chemical residues that are the same or similar at corresponding positions.

The term "mammal" refers to any organism classified as a mammal, including mice, rats, rabbits, dogs, cats, cows, horses and humans. In one embodiment of the invention, the mammal is a mouse. In another embodiment of the invention, the mammal is a human.

The terms "metastatic prostate cancer" and "metastatic disease" mean prostate cancers that have spread to regional lymph nodes or to distant sites, and are meant to include stage D disease under the AUA system and stage TxNxM+ under the TNM system. As is the case with locally advanced prostate cancer, surgery is generally not indicated for patients with metastatic disease, and hormonal (androgen ablation) therapy is a preferred treatment modality. Patients with metastatic prostate cancer eventually develop an androgen-refractory state within 12 to 18 months of treatment initiation. Approximately half of these androgen-refractory patients die within 6 months after developing that status. The most common site for prostate cancer metastasis is bone. Prostate cancer bone metastases are often osteoblastic rather than osteolytic (i.e., resulting in net bone formation). Bone metastases are found most frequently in the spine, followed by the femur, pelvis, rib cage, skull and humerus. Other common sites for metastasis include lymph nodes, lung, liver and brain. Metastatic prostate cancer is typically diagnosed by open or laparoscopic pelvic lymphadenectomy, whole body radionuclide scans, skeletal radiography, and/or bone lesion biopsy.

The term "monoclonal antibody" refers to an antibody obtained from a population of substantially homogeneous antibodies, i.e., the antibodies comprising the population are identical except for possible naturally occurring mutations that are present in minor amounts.

A "motif", as in biological motif of a 121P2A3-related protein, refers to any pattern of amino acids forming part of the primary sequence of a protein, that is associated with a particular function (e.g. protein-protein interaction, protein-DNA interaction, etc) or modification (e.g. that is phosphorylated, glycosylated or amidated), or localization (e.g. secretory sequence, nuclear localization sequence, etc.) or a sequence that is correlated with being immunogenic, either humorally or cellularly. A motif can be either contiguous or capable of being aligned to certain positions that are generally correlated with a certain function or property.

A "pharmaceutical excipient" comprises a material such as an adjuvant, a carrier, pH-adjusting and buffering agents, tonicity adjusting agents, wetting agents, preservative, and the like.

"Pharmaceutically acceptably refers to a non-toxic, inert, and/or composition that is physiologically compatible with humans or other mammals.

The term "polynucleotide" means a polymeric form of nucleotides of at least 10 bases or base pairs in length, either ribonucleotides or deoxynucleotides or a modified form of either type of nucleotide, and is meant to include single and double stranded forms of DNA and/or RNA. In the art, this term if often used interchangeably with "oligonucleotide". A polynucleotide can comprises a nucleotide sequence disclosed herein wherein thymidine (T), as shown for example in Figure 2, can also be uracil (U); this definition pertains to the differences between the chemical structures of DNA and RNA, in particular the observation that one of the four major bases in RNA is uracil (U) instead of thymidine (T).

The term "polypeptide" means a polymer of at least about 4, 5, 6, 7, or 8 amino acids. Throughout the specification, standard three letter or single letter designations for amino acids are used. In the art, this term is often used interchangeably with "peptide" or "protein".

The term "variant" refers to a molecule that exhibits a variation from a described type or norm, such as a protein that has one or more different amino acid residues in the corresponding position(s) of a specifically described protein (e.g. the 121P2A3 protein shown in Figure 2 or Figure 3. An analog is an example of a variant protein. Splice isoforms and single nucleotides polymorphisms (SNPs) are further examples of variants.

The "121P2A3 proteins" are those specifically identified in figure 3. Allelic variants, conservative substitution variants, analogs and homologs can be isolated/generated and characterized without undue experimentation following the methods outlined herein or readily available in the art.

### II.) 121P2A3 Proteins

121P2A3 proteins are polypeptides having all of the amino acid sequence of human 121P2A3 as shown in Figure 2 or Figure 3.

### II.A.) Expression of 121P2A3 Proteins

In an embodiment described in the examples that follow, 121P2A3 can be conveniently expressed in cells (such as 293T cells) transfected with a commercially available expression vector such as a CMV-driven expression vector encoding 121P2A3 with a C-terminal 6XHis and MYC tag (pcDNA3.1/mycHIS, Invitrogen or Tag5, GenHunter Corporation, Nashville TN). The Tag5 vector provides an IgGK secretion signal that can be used to facilitate the production of a secreted 121P2A3 protein in transfected cells. The secreted HIS-tagged 121P2A3 in the culture media can be purified, e.g., using a nickel column using standard techniques.

### II.B.) Uses of 121P2A3 Proteins

121P2A3 protein fragments/subsequences are particularly useful in generating and characterizing domain-specific antibodies (e.g., antibodies recognizing an extracellular or intracellular epitope of a 121P2A3 protein), for identifying agents or cellular factors that bind to 121P2A3 or a particular structural domain thereof, and in various therapeutic and diagnostic contexts, including but not limited to diagnostic essays, cancer vaccines and methods of preparing such vaccines.

Proteins encoded by the 121P2A3 genes, or by analogs, homologs or fragments thereof, have a variety of uses, including but not limited to generating antibodies and in methods for identifying ligands and other agents and cellular constituents that bind to a 121P2A3 gene product. Antibodies raised against a 121P2A3 protein or fragment thereof are useful in diagnostic and prognostic assays, and imaging methodologies in the management of human cancers characterized by expression of 121P2A3 protein, such as those listed in Table I.

Various immunological assays useful for the detection of 121P2A3 proteins are used, including but not limited to various types of radioimmonoassays, enzyme-linked immunosorbent assays (ELISA), enzyme-linked immunofluorescent assays (ELIFA), immunocytochemical methods, and the like. Antibodies can be labeled and used as immunonological imaging reagents capable of detecting 121P2A3-expressing cells (e.g., in radioscintigraphic imaging methods)- 121P2A3 proteins are also particularly useful in generating cancer vaccines, as further described herein.

### III.) 121P2A3 Antibodies

Antibodies that bind to 121P2A3 proteins are disclosed herein. The antibodies used in the method of the invention specifically bind to a 121P2A3 protein and do not bind (or bind weakly) to peptides or proteins that are not 121P2A3 proteins or fragments thereof.

121P2A3 antibodies are particularly useful in cancer (see, e.g., Table I) diagnostic and prognostic assays, and imaging methodologies. Similarly, such antibodies are useful in the diagnosis and/or prognosis of other cancers, to the extent 121P2A3 is also expressed or overexpressed in these other cancers.

Immunological assays useful for the detection and quantification of 121P2A3 proteins are also disclosed herein. Such assays can comprise one or more 121P2A3 antibodies capable of recognizing and binding a 121P2A3 protein, as appropriate. These assays are performed within various immunological assay formats well know in the art, including but not limited to various types of radioimmunoassays, enzyme-linked immunosorbent assays (ELISA), enzyme-linked immunofluorescent assays (ELIFA), and the like.

Immunological non-antibody assays also comprise T cell immunogenicity assays (inhibitory or stimulatory) as well as major histocompatibility complex (MHC) binding assays.

In addition, immunological imaging methods capable of detecting prostate cancer and other cancers expressing 121P2A3 are also provided by the invention, including but not limited to riadioscintigraphic imaging methods using labeled 121P2A3 antibodies. Such assays are clinically useful in the detection, monitoring, and prognosis of 121P2A3 expressing cancers such as prostate cancer.

121P2A3 antibodies are also used in methods for purifying a 121P2A3 protein and for isolating 121P2A3 homologues and related molecules. For example, a method of purifying a 121P2A3 protein comprises incubating a 121P2A3 antibody, which has been coupled to a solid matrix, with a lysate or other solution containing a 121P2A3 protein under conditions that permit the 121P2A3 antibody to bind to the 121P2A3 protein; washing the solid matrix to eliminate impurities; and eluting the 121P2A3 protein from the coupled antibody. Other uses of 121P2A3 antibodies include generating anti-idiotypic antibodies that mimic a 121P2A3 protein.

Various methods for the preparation of antibodies are well known in the art. For example, antibodies can be prepared by immunizing a suitable mammalian host using a 121P2A3 protein, peptide, or fragment, in isolated or immunoconjugated form (Antibodies: A Laboratory Manual, CSH Press, Eds., Harlow, and Lane (1988); Harlow, Antibodies, Cold Spring Harbor Press, NY (1989)). In addition, fusion proteins of 121P2A3 can also be used, such as a 121P2A3 GST-fusion protein. In a particular embodiment, a GST fusion protein comprising all or most of the amino acid sequence of Figure 2 or Figure 3 is produced, then used as an immunogen to generate appropriate antibodies. In another embodiment, a 1211P2A3 protein is synthesized and used as an immunogen.

The amino acid sequence of a 121P2A3 protein as shown in Figure 2 or Figure 3 can be analyzed to select specific regions of the 121P2A3 protein for generating antibodies. For example, hydrophobicity and hydrophilicity analyses of a 121P2A3 amino acid sequence are used to identify hydrophilic regions in the 121P2A3 structure. Regions of a 121P2A3 protein that show immunogenic structure, as well as other regions and domains, can readily be identified using various other methods known in the art, such as Chou-Fasman, Garnier-Robson, Kyte-Doolittle, Eisenberg, Karplus-Schultz or Jameson-Wolf analysis. Hydrophilicity profiles can be generated using the method ofHopp, T.P. and Woods, K.R., 1981, Proc. Natl. Acad. Sci. U.S.A. 78:3824-3828. Hydropathicity profiles can be generated using the method of Kyte, J. and Doolittle, R.F., 1982, J. Mol. Biol. 157:105-132. Percent (%) Accessible Residues profiles can be generated using the method of Janin. J., 1979, Nature 277:491-492. Average Flexibility profiles can be generated using the method of Bhaskaran R., Ponnuswamy P.K., 1988, Inc. J. Pept. Protein Res. 32:242-255. Beta-turn profiles can be generated using the method of Deleage, G., Roux B. 1987, Protein Engineering 1:289-294. Thugs, each region identified by any of these programs or methods can be used. Methods for the generation of 121P2A3 antibodies are further illustrated by way of the examples provided herein. Methods for preparing a protein or polypeptide for use as an immunogen are well known in the art. Also well known in the art are methods for preparing immunogenic conjugates of a protein with a carrier, such as BSA, KLH or other carrier protein. In some circumstances, direct conjugation using, for example, carbodiimide reagents are used; in other instances linking reagents such as those supplied by Pierce Chemical Co., Rockford, IL, are effective. Administration of a 121P2A3 immunogen is often conducted by injection over a suitable time period and with use of a suitable adjuvant, as is understood in the art. During the immunization schedule, titers of antibodies can be taken to determine adequacy of antibody formation.

121P2A3 monoclonal antibodies can be produced by various means well known in the art. For example, immortalized cell lines that secrete a desired monoclonal antibody are prepared using the standard hybridoma technology of Kohler and Milstein or modifications that immortalize antibody-producing B cells, as is generally known. Immortalized cell lines that secrete the desired antibodies are screened by immunoassay in which the antigen is a 121P2A3 protein. When the appropriate immortalized cell culture is identified, the cells can be expanded and antibodies produced either from *in vitro* cultures or from ascites fluid.

The antibodies or fragments can also be produced, by recombinant means: Regions that bind specifically to the desired regions of a 121P2A3 protein can also be produced in the context of chimeric or complementarity determining region (CDR) grafted antibodies of multiple species origin. Humanized or human 121P2A3 antibodies can also be produced, and are preferred for use in therapeutic contexts. Methods for humanizing murine and other non-human antibodies, by substituting one or more of the non-human antibody CDRs for corresponding human antibody sequences, are well known (see for example, Jones et al., 1986, Nature 321: 522-525; Riechmann et al., 1988, Nature 332: 323-327; Verhoeyen et al., 1988, Science 239: 1534-1536). See also, Carter et al., 1993, Proc. Nad. Acad. Sci. USA 89: 4285 and Sims et al., 1993, J. Immunol. 151: 2296.

Methods for producing fully human monoclonal antibodies include phage display and transgenic methods (for review, see Vaugban et al., 1998, Nature Biotechnology 16: 535-539). Fully human 121P2A3 monoclonal antibodies can be generated using cloning technologies employing large human Ig gene combinatorial libraries (i.e., phage display) (Griffiths and Hoogenboom, Building an in vitro immune system: human antibodies from phage display libraries. In: Protein Engineering of Antibody Molecules for Prophylactic and Therapeutic Applications in Man, Clark, M. (Ed.), Nottingham Academic, pp 45-64 (1993); Burton and Barbas, Human Antibodies from combinatorial libraries. Id., pp 65-82)- Fully human 121P2A3 monoclonal antibodies can also be produced using transgenic mice engineered to contain human immunoglobulin gene loci as described in PCT Patent Application WO98/24893, Kucherlapati and Jakobovits *et al.*, published December 3, 1997 (see also, Jakobovits, 1998, Exp. Opin. Invest. Drugs 7(4): 607-614; U.S. patents 6,162,963 issued 19 December 2006; 6,150,584 issued 12 November 2000; and, 6,114598 issued 5 September 2000). This method avoids the *in vitro* manipulation required with phage display technology and efficiently produces high affinity authentic human antibodies.

Reactivity of 121P2A3 antibodies with a 121P2A3 protein can be established by a number of well known means, including Western blot, immunoprecipitation, ELISA, and FACS analyses using, as appropriate, 121P2A3-related proteins, 121P2A3-expressing cells or extracts thereof. A 121P2A3 antibody or fragment thereof can be labeled with a detectable marker or conjugated to a second molecule. Suitable detectable markers include, but are not limited to, a radioisotope, a fluorescent compound, a bioluminescent compound, chemiluminescent compound, a metal chelator or an enzyme. Further, bi-specific antibodies specific for two or more 121P2A3 epitopes are generated using methods generally known in the art. Homodimeric antibodies can also be generated by cross-linking techniques known in the art (e.g., Wolff et al., Cancer Res. 53; 2560-2565).

### IV.) Methods for the Detection of 121P2A3

Methods for detecting 121P2A3 proteins, are disclosed herein. The Expression profile of 121P2A3 makes it a diagnostic marker for metastasized disease. Accordingly, the status of 121P2A3 gene products provides information useful for predicting a variety of factors including susceptibility to advanced stage disease, rate of progression, and/or tumor aggressiveness.

Assays for detecting the presence of a 121P2A3 protein in a tissue or other biological sample such as serum, semen, bone, prostate, urine, cell preparations, and the like are also disclosed herein. Methods for detecting a 121P2A3 protein are also well known and include, for example, immunoprecipitation, immunohistochemical analysis, Western blot analysis, molecular binding assays, ELISA, ELIFA and the like. For example, a method of detecting the presence of a 121P2A3 protein in a biological sample comprises first contacting the sample with a 121P2A3 antibody, a 121P2A3-reactive fragment thereof, or a recombinant protein containing an antigen binding region of a 121P2A3 antibody; and then detecting the binding of 121P2A3 protein in the sample.

### V) Methods for Monitoring the Status of 121P2A3-Proteins

Oncogenesis is known to be a multistep process where cellular growth becomes progressively dysregulated and cells progress from a normal physiological state to precancerous and then cancerous states (see, e.g., Alers et al., Lab Invest. 77(5): 437-438 (1997) and Isaacs et al., Cancer Surv. 23: 19-32 (1995)). In this context, examining a biological sample for evidence of dysregulated cell growth (such as aberrant 121P2A3 expression in cancers) allows for early detection of such aberrant physiology, before a pathologic state such as cancer has progressed to a stage that therapeutic options are more limited and or the prognosis is worse. In such examinations, the status of 121P2A3 in a biological sample of interest can be compared, for example, to the status of 121P2A3 in a corresponding normal sample (e.g. a sample from that individual or alternatively another individual that is not affected by a pathology). An alteration in the status of 121P2A3 in the biological sample (as compared to the normal sample) provides evidence of dysregulated cellular growth. In addition to using a biological sample that is not affected by a pathology as a normal sample, one can also use a predetermined Normative value such as a predetermined normal level of mRNA expression (see, e.g., Grever et al., J. Comp. Neurol. 1996 Dec 9; 376(2): 306-14 and U.S. Patent No. 5,837,501) to compare 121P2A3 status in a sample.

The term "status" in this context is used according to its art accepted meaning and refers to the condition or state of a gene and its products. Typically, skilled artisans use a number of parameters to evaluate the condition or state of a gene and its products. These include, but are not limited to the location of expressed gene products (including the location of 121P2A3 expressing cells) as well as the level, and biological activity of expressed gene products (such as 121P2A3 polypeptides). Typically, an alteration in the status of 121P2A3 comprises a change in the location of 121P2A3 and/or 121P2A3 expressing cells and/or an increase in 121P2A3 protein expression.

121P2A3 status in a sample can be analyzed by a number of means well known in the art, including without limitation_{,} immunohistochemical analysis, Western blot analysis, and tissue array analysis. Typical protocols for evaluating the status of a 121P2A3 protein are found, for example in Ausubel et al. eds., 1995, Current Protocols In Molecular Biology, Units 2 (Northern Blotting), 4 (Southern Blotting), 15 (Immunoblotting) and 18 (PCR Analysis). Thus, the status of 121P2A3 in a biological sample is evaluated by various methods utilized by skilled artisans including, but not limited to Western and/or immunohistochemical analysis (to examine, for example alterations in polypeptide sequences, alterations in polypeptide localization within a sample, alterations in expression levels of 121P2A3 proteins and/or associations of 121P2A3 proteins with polypeptide binding partners).

The Expression profile of 121P2A3 makes it a diagnostic marker for local and/or metastasized disease, and provides information on the growth or oncogenic potential of a biological sample. In particular, the status of 121P2A3 provides information useful for predicting susceptibility to particular disease stages, progression, and/or tumor aggressiveness. The invention provides methods and assays for determining 121P2A3 status and diagnosing cancers that express 121P2A3, such as cancers of the tissues listed in Table I. For Example, because 121P2A3 mRNA is so highly expressed in prostate and other cancers relative to normal prostate tissue, assays that evaluate the levels of 121P2A3 mRNA transcripts or proteins in a biological sample can be used to diagnose a disease associated with 121P2A3 dysregulation, and can provide prognostic information useful in defining appropriate therapeutic options.

The expression status of 121P2A3 provides information including the presence, stage and location of dysplastic, precancerous and cancerous cells, predicting susceptibility to various stages of disease, and/or for gauging tumor aggressiveness. Moreover, the expression profile makes it useful as an imaging reagent for metastasized disease. Consequently, an aspect of the invention is directed to the various molecular prognostic and diagnostic methods for examining the status of 121P2A3 in biological samples such as those from individuals suffering from, or suspected of suffering from a pathology characterized by dysregulated cellular growth, such as cancer.

As described above, the status of 121P2A3 in a biological sample can be examined by a number of well-known procedures in the art. For example, the status of 121P2A3 in a biological sample taken from a specific location in the body can be examined by evaluating the sample for the presence or absence of 121P2A3 expressing cells (e.g. those that express 121P2A3 mRNAs or proteins). This examination can provide evidence of dysregulated cellular growth, for example, when 121P2A3-expressing cells are found in a biological sample that does not normally contain such cells (such as a lymph node), because such alterations in the status of 121P2A3 in a biological sample are often associated with dysregulated cellular growth. Specifically, one indicator of dysregulated cellular growth is the metastases of cancer cells from an organ of origin (such as the prostate) to a different area of the body (such as a lymph node). In this context, evidence of dysregulated cellular growth is important for example because occult lymph node metastases can be detected in a substantial proportion of patients with prostate cancer, and such metastases are associated with known predictors of disease progression (see, e.g., Murphy et al., Prostate 42(4): 315-317 (2000); Su et al., Semin. Surg. Oncol. 18(1): 17-28 (2000) and Freeman et al., J Urol 1995 Aug 154(2 Pt 1):474-8).

In one aspect, methods for monitoring 121P2A3 gene products by determining the status of 121P2A3 gene products expressed by cells from an individual suspected of having a disease associated with dysregulated cell growth (such as hyperplasia or cancer) and then comparing the status so determined to the status of 121P2A3 gene products in a corresponding normal sample are disclosed herein. The presence of aberrant 121P2A3 gene products in the test sample relative to the normal sample provides an indication of the presence of dysregulated cell growth within the cells of the individual.

Assays useful in determining the presence of cancer in an individual, comprising detecting a significant increase in 121P2A3 protein expression in a test cell or tissue sample relative to expression levels in the corresponding normal cell or tissue are described herein. The presence of significant 121P2A3 expression in any of the tissues listed in table I is useful to indicate the emergence, presence and/or severity of a cancer, since the corresponding normal tissues do not express 121P2A3 mRNA or express it at lower levels.

121P2A3 status is determined at the protein level rather than at the nucleic acid level. For example, such a method comprises determining the level of 121P2A3 protein expressed by cells in a test tissue sample and comparing the level so determined to the level of 121P2A3 expressed in a corresponding normal sample. In one embodiment, the presence of 121P2A3 protein is evaluated, for example, using immunohistochemical methods. 121P2A3 antibodies or binding partners capable of detecting 121P2A3 protein expression are used in a variety of assay formats well known in the art for this purposes.

In a further embodiment, one can evaluate the status of 121P2A3 amino acid sequences in a biological sample in order to identify perturbations in the structure of these molecules. These perturbations can include insertions, deletions, substitutions and the like. Such evaluations are useful because perturbations in the nucleotide and amino acid sequences are observed in a large number of proteins associated with a growth dysregulated phenotype (see, e.g., Marrogi et al., 1999, J. Cutan, Pathol. 26(8):369-378). For example, a mutation in the sequence of 121P2A3 may be indicative of the presence or promotion of a tumor. Such assays therefore have diagnostic and predictive value where a mutation in 121P2A3 indicates a potential loss of function or increase in tumor growth.

A wide variety of assays for observing perturbations in amino acid sequences are well known in the art. For example, the size and structure of amino acid sequences of 121P2A3 gene products are observed by the Western protocols discussed herein. In addition, other methods for observing perturbations in amino acid sequences such as single strand conformation polymorphism analysis are well known in the art (see, e.g., U.S. Patent Nos. 5,382,510 issued 7 September 1999, and 5,952,170 issued 17 January 1995).

The assessment of the susceptibility that an individual has for developing cancer is also disclosed herein. In one embodiment, a method for predicting susceptibility to cancer comprises detecting

121P2A3 protein in a tissue sample, its presence indicating susceptibility to cancer, wherein the degree of 121P2A3 expression correlates to the degree of susceptibility. In a specific embodiment the presence of 121P2A3 in prostate or other tissue is examined, with the presence of 121P2A3 in the sample providing an indication of prostate cancer susceptibility (or the emergence or existence of a prostate tumor). Similarly, one can evaluate the integrity 121P2A3 amino acid sequences in a biological sample, in order to identify perturbations in the structure of these molecules such as insertions, deletions, substitutions and the like. The presence of one or more perturbations in 121P2A3 gene products in the sample is an indication of cancer susceptibility (or the emergence or existence of tumor).

Methods for gauging tumor aggressiveness are also described herein. In one embodiment, a method for gauging aggressiveness of a tumor comprises determining the level of 121P2A3 protein expressed by tumor cells, comparing the level so determined to the level of 121P2A3 protein expressed in a corresponding normal tissue taken from the same individual or a normal tissue reference sample, wherein the degree of 121P2A3 protein expression in the tumor sample relative to the normal sample indicates the degree of aggressiveness. In a specific embodiment, aggressiveness of a tumor is evaluated by determining the extent to which 121P2A3 is expressed in the tumor cells, with higher expression levels indicating more aggressive tumors. Another embodiment is the evaluation of the integrity of 121P2A3 amino acid sequences in a biological sample, in order to identify perturbations in the structure of these molecules such as insertions, deletions, substitutions and the like. The presence of one or more perturbations indicates more aggressive tumors.

Method for observing the progression of a malignancy in an individual over time are also disclosed herein. In one embodiment, methods for observing the progression of a malignancy in an individual over time comprise determining the level of 121P2A3 protein expressed by cells in a sample of the tumor, comparing the level so determined to the level of 121P2A3 protein expressed in an equivalent tissue sample taken from the same individual at a different time, wherein the degree of 121P2A3 protein expression in the tumor sample over time provides information on the progression of the cancer. In a specific embodiment, the progression of a cancer is evaluated by determining 121P2A3 expression in the tumor cells over time, where increased expression over time indicates a progression of the cancer. Also, one can evaluate the integrity 121P2A3 amino acid sequences in a biological sample in order to identify perturbations in the structure of these molecules such as insertions, deletions, substitutions and the like, where the presence of one or more perturbations indicates a progression of the cancer.

The above diagnostic approaches can be combined with any one of a wide variety of prognostic and diagnostic protocols known in the art. For example, methods for observing a coincidence between the expression of 121P2A3 gene products (or perturbations in 121P2A3 gene products) and a factor that is associated with malignancy, as a means for diagnosing and prognosticating the status of a tissue sample are also disclosed herein. A wide variety of factors associated with malignancy can be utilized, such as the expression of genes associated with malignancy (e.g. PSA, PSCA and PSM expression for prostate cancer etc.) as well as gross cytological observations (see, e.g., Bocking et al.,1984, Anal. Quant. Cytol. 6(2):74-88; Epstein, 1995, Hum. Pathol. 26(2):223-9; Thorson et al., 1998, Mod. Pathol. 11(6)543-51; Baisden et al., 1999, Am. J. Surg. Pathol. 23(8):918-24). Methods for observing a coincidenoe between the expression of 121P2A3 gene and 121P2A3 gene products (or perturbations in 121P2A3 gene and 121P2A3 gene products) and another factor that is associated with malignancy are useful, for example, because the presence of a set of specific factors that coincide with disease provides information crucial for diagnosing and prognosticating the status of a tissue sample.

In one embodiment, methods for observing a coincidence between the expression of 121P2A3 gene products (or perturbations in 121P2A3 gene products) and another factor associated with malignancy entails detecting the overexpression of 121P2A3 protein in a tissue sample, detecting the overexpression of PSA mRNA or protein in a tissue sample (or PSCA or PSM expression), and observing a coincidence of 121P2A3 protein and PSA mRNA or protein overexpression (or PSCA or PSM expression). In a specific embodiment, the expression of 121P2A3 and PSA mRNA in prostate tissue is examined, where the coincidence of 121P2A3 and PSA mRNA overexpression in the sample indicates the existence of prostate cancer, prostate cancer susceptibility or the emergence or status of a prostate tumor.

Methods for detecting and quantifying the expression of 121P2A3 protein are described herein, and standard protein detection and quantification technologies are well known in the art specify embodiment, polyclonal or monoclonal antibodies specifically reactive with the wild-type 121P2A3 protein can be used in an immunohistochemical assay of biopsied tissue.

### VI.) Diagnostic and Prognostic Embodiments of 121P2A3.

As disclosed herein, 121P2A3 anti-polypeptide antibodies arc used in well known diagnostics and prognostic assays that examine conditions associated with dysregulated cell growth such as cancer, in particular the cancers listed in Table I (see, e.g., both its specific pattern of tissue expression as well as its overexpression in certain cancers as described for example in the Example entitled "Expression analysis of 121P2A3 in normal tissues, and patient specimens").

121P2A3 can be analogized to a prostate associated antigen PSA, the archetypal marker that has been used by medical practitioners for years to identify and monitor the presence of prostate cancer (see, e.g., Merrill et al., J. Urol. 163(2): 503-5120 (2000); Polascik et al., J. Urol. Aug; 162(2):293-306 (1999) and Fortier et al., J. Nat. Cancer lost. 91(19): 1635-1640(1999)). A variety of other diagnostic markers are also used in similar contexts including p53 and K-ras (see, e.g., Tulchinsky et al., Int. J Mol Med 1999 Jul 4(1):99-102 and Minimoto et al., Cancer Detect Prev 2000;24(1):1-12). Therefore, this disclosure of anti-121P2A3 antibodies used to identify the presence of these molecules and their properties allows skilled artisans to utilize these molecules in methods that are analogous to those used, for example, in a variety of diagnostic assays directed to examining conditions associated with cancer.

Typical embodiments of diagnostic methods which utilize the 121P2A3 antibodies are analogous to those methods from well-established diagnostic assays which employ, e.g., PSA antibodies. For example, just as PSA polypeptides are used to generate antibodies specific for PSA which can then be used to observe the presence and/or the level of PSA proteins in methods to monitor PSA protein overexpression (see, e.g., Stephan et al., Urology 55(4):560-3 (2000)) or the metastasis of prostate cells (see, e.g., Alanen et al., Pathol. Res. Pract 192(3):233-7 (1996)), the 121P2A3 polypeptides described herein can be utilized to generate antibodies for use in detecting 121P2A3 overexpression or the metastasis of prostate cells and cells of other cancers expressing this gene.

Specifically, because metastases involves the movement of cancer cells from an organ of origin (such as the lung or prostate gland etc.) to a different area of the body (such as a lymph node), assays which examine a biological sample for the presence of cells expressing 121P2A3 polypeptides can be used to provide evidence of metastasis. For example, when a biological sample from tissue that does not normally contain 121P2A3-expressing cells (lymph node) is found to contain 121P2A3-expressing cells such as the 121P2A3 expression seen in LAPC4 and LAPC9, xenografts isolated from lymph node and bone metastasis, respectively, this finding is indicative of metastasis.

Alternatively, 121P2A3 polypeptides can be used to provide evidence of cancer, for example, when cells in a biological sample that do not normally express 121P2A3 or express 121P2A3 at a different level are found to express 121P2A3 or have an increased expression of 121P2A3 (see, e.g., the 121P2A3 expression in the cancers listed in Table I and in patient samples etc. shown in the accompanying Figures). In such assays, artisans may further wish to generate supplementary evidence of metastasis by testing the biological sample for the presence of a second tissue restricted marker (in addition to 121P2A3) such as PSA, PSCA etc. (see, e.g., Alanen et al., Pathol. Res. Pract. 192(3): 233-237 (1996)).

Furthermore, PSA polypeptides which contain an epitope that can be recognized by an antibody or T cell that specifically blinds to that epitope are used in methods of monitoring PSA. 121P2A3 polypeptide fragments and polypeptide analogs or variants can also be used in an analogous manner. This practice of using polypeptide fragments or polypeptide variants to generate antibodies (such as anti-PSA antibodies or T cells) is typical in the art with a wide variety of systems such as fusion proteins being used by practitioners (see, e.g., Current Protocols In Molecular Biology, Volume 2, Unit 16, Frederick M. Ausubel et al. eds., 1995). In this context, each epitope(s) functions to provide the architecture with which an antibody or T cell is reactive. Typically, skilled artisans create a variety of different polypeptide fragments that can be used in order to generate immune responses specific for different portions of a polypeptide of interest (see, e.g., U. S. Patent No. 5,840,501 and U.S. Patent No. 5,939,533). For example it may be preferable to utilize a polypeptide comprising one of the 121P2A3 biological motifs discussed herein or a motif-bearing subsequence which is readily identified by one of skill in the art based on motifs available in the art Polypeptide fragments, variants or analogs are useful in this context as long as they comprise an epitope capable of generating an antibody specific for a 121P2A3 polypeptide shown in Figure 3.

As shown herein, the 121P2A3 polypeptides, as well as the anti-121P2A3 antibodies used to identify the presence of these molecules exhibit specific properties that make them useful in diagnosing cancers such as those listed in Table I. Diagnostic assays that measure the presence of 121P2A3 gene products, in order to evaluate the presence or onset of a disease condition described herein, such as prostate cancer, arc used to identify patients for preventive measures or further monitoring, as has been done so successfully with PSA. Moreover, these materials satisfy a need in the art for molecules having similar or complementary characteristics to PSA in situations where, for example, a definite diagnosis of metastasis of prostatic origin cannot be made on the basis of a test for PSA alone (see, e.g., Alanen et al., Pathol. Res. Pract. 192(3): 233-237 (1996)), and consequently, materials such as anti-121P2A3 antibodies, need to be employed to confirm a metastases of prostatic origin.

### VII.) Kits

For use in the diagnostic applications described herein, kits are also disclosed herein. Such kits can comprise a carrier, package or container that is compartmentalized to receive one or more containers such as vials, tubes, and the like, each of the container(s) comprising one of the separate elements to be used in the method. For example, the container(s) can comprise a probe that is or can be detectably labeled. Such probe can be an antibody specific for a 121P2A3 The kit can include all or part of the amino acid sequence of Figure 2 or Figure 3 or analogs thereof, or a nucleic acid molecules that encodes such amino acid sequences.

The kit of the invention will typically comprise the container described above and one or more other containers comprising materials desirable from a commercial and user standpoint, including buffers, diluents, filters, needles, syringes, and package inserts with instructions for use.

A label can be present on the container to indicate that the composition is used for a specific therapy or non-therapeutic application, and can also indicate directions for either *in vivo* or *in vitro* use, such as those described above. Directions and or other information can also be included on an insert which is included with the kit.

### EXAMPLES:

Various aspects of the invention are further described and illustrated by way of the several examples that follow, none of which are intended to limit the scope of the invention.

### Example 1: SSH-Generated Isolation of a cDNA Fragment of the 121P2A3 Gene

To isolate genes that are involved in the progression of androgen dependent (AD) prostate cancer to androgen independent (AI) cancer, an experiment was conducted with the LAPC-9 AD xenograft in male SCID mice. Mice that harbored LAPC-9 AD xenografts were castrated when the tumors reached a size of 1 cm in diameter. The tumors regressed in size and temporarily stopped producing the androgen dependent protein PSA. Seven to fourteen days post-castration, PSA levels were detectable again in the blood of the mice. Eventually the tumors develop an AI phenotype and start growing again in the castrated males. Tumors were harvested at different time points after castration to identify genes that are turned on or off during the transition to androgen independence.

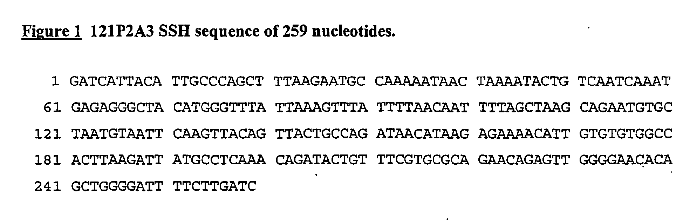

The gene 121P2A3 was derived from an LAPC-9 AD minus LAPC-9 AD (28 days post-castration) subtraction. The SSH DNA sequence of 259 bp is listed in Figure 1. A cDNA (121P2A3 clone 5) of 2473 bp was isolated from a LAPC-9AD cDNA library, revealing an ORF of 464 amino acids (Figures 2 and 3). Variants of 121P2A3 v.1 were also identified, and these are listed in Figures 2 and 3.

The 121P2A3 protein shows homology to a novel hypothetical protein FLJ10540 isolated from the human teratocarcinoma cell line NT2 (Figure 4B and 4D). The two proteins are 98% identical over a 223 amino acid region starting from position 170 of 121P2A3 v.1. The 121P2A3 protein also shows homology to the XM_005908 (similar to RIKEN cDNA 1200008012) gene. The gene XM_005908 was isolated from rhabdomyosarcoma cDNA library, validating the expression of 121P2A3 in human cancers. 121P2A3 v.1 and XM_005908 proteins are 99% identical over 464 amino acids (Figure 4E).

Amino acid sequence analysis of 121P2A3 reveals 75% identity over 464 amino acid region to a mouse putative protein (Figure 4F). 121P2A3 v.1 also shows homology to the human nef-associated factor-1 (naf-1), The two proteins are 23% identical over a 339 amino acid region (Figure 4G).

Additional homology analysis is presented in Example 44.

### Materials and Methods

### LAPC Xenografts and Human Tissues:

LAPC xenografts were obtained from Dr. Charles Sawyers (UCLA) and generated as described (Klein et al, 1997, Nature Med. 3: 402-408; Craft et al., 1999, Cancer Res. 59: 5030-5036). Androgen dependent and independent LAPC-9 AD and AI xenografts were grown in male SCID mice and were passaged as small tissue chunks in recipient males. LAPC-9 AI xenografts were derived from LAPC-9 AD tumors, respectively. To generate the AI xenografts, male mice bearing AD tumors were castrated and maintained for 2-3 months. After the tumors re-grew, the tumors were harvested and passaged in castrated males or in female SCID mice.

### Cell Lines:

Human cell lines (e.g., HeLa) were obtained from the ATCC and were maintained in DMEM with 5% fetal calf serum.

### Human Tissues:

The patient cancer and normal tissues were purchased from different sources such as the NDRI (Philadelphia, PA). mRNA for some normal tissues were purchased from Clontech, Palo Alto, CA.

### RNA Isolation:

Tissues were homogenized in Trizol reagent (Life Technologies, Gibco BRL) using 10 ml/ g tissue isolate total RNA. Poly A RNA was purified from total RNA using Qiagen's Oligotex mRNA Mini and Midi kits. Total and mRNA were quantified by spectrophotometric analysis (O.D. 260/280 nm) and analyzed by gel electrophoresi

### Oligonucleotides:

The following HPLC purified oligonucleotides were used.
DPNCDN (cDNA synthesis primer):
   5'TTTTGATCAAGCTT₃₀3'
Adaptor 1: 
5'CTAATACGACTCACTATAGGGCTCGAGCGGCCGCCCGGGCAG3',
3'GGCCCGTCCTAG5',
Adaptor 2: 
5'GTAATACGACTCACTATAGGGCAGCGTGGTCGCGGCCGAG3',
3'CGGCTCCTAG5'
PCR primer 1:
   5'CTAATACGACTCACTATAGGGC3'
Nested primer (NP)1:
   5'TCGAGCGGCCGCCCGGGCAGGA3'
Nested primer (NP)2:
   5'AGCGTGGTCGCGGCCGAGGA3'

### Suppression Subtractive Hybridization:

Suppression Subtractive Hybridization (SSH) was used to identify cDNAs corresponding to genes that are differentially expressed in prostate cancer. The SSH reaction utilized cDNA from two LAPC-9 AD xenografts. Specifically, to isolate genes that are involved in the progression of androgen dependent (AD) prostate cancer to androgen independent (AI) cancer, an experiment was conducted with the LAPC-9 AD xenograft in male SCID mice. Mice that harbored LAPC-9 AD xenografts were castrated when the tumors reached a size of 1 cm in diameter. The tumors regressed in size and temporarily stopped producing the androgen dependent protein PSA. Seven to fourteen days post-castration, PSA levels were detectable again in the blood of the mice. Eventually the tumors develop an AI phenotype and start growing again in the castrated males. Tumors were harvested at different time points after castration to identify genes that are turned on or off during the transition to androgen independence.

The gene 121P2A3 was derived from an LAPC-9 AD tumor (grown in intact male mouse) minus an LAPC-9 AD tumor (28 days post-castration) subtraction. The SSH DNA sequence 121P2A3 (Figure 1) was identified.

The cDNA derived from an LAPC-9 AD tumor (28 days post-castration) was used as the source of the "driver" cDNA, while the cDNA from the LAPC-9 AD tumor (grown in intact male mouse) was used as the source of the "tester" cDNA. Double stranded cDNAs corresponding to tester and driver cDNAs were synthesized from 2 µg of poly(A)⁺ RNA isolated from the relevant xenograft tissue, as described above, using CLONTECH's PCR-Select cDNA Subtraction Kit and 1 ng of oligonucleotide DPNCDN as primer. First-and second-strand synthesis were carried out as described in the Kit's user manual protocol (CLONTECH Protocol No. PT1117-1, Catalog No. K1804-1). The resulting cDNA was digested with Dpn II for 3 hrs at 37°C. Digested cDNA was extracted with phenol/chloroform (1:1) and ethanol precipitated.

Driver cDNA was generated by combining in a 1:1 ratio Dpn II digested cDNA from the relevant xenograft source (see above) with a mix of digested cDNAs derived from the human cell lines HeLa, 293, A431, Colo205, and mouse liver.

Tester cDNA was generated by diluting 1 µl of Dpn II digested cDNA from the relevant xenograft source (see above) (400 ng) in 5 µl of water. The diluted cDNA (2 µl, 160 ng) was then ligated to 2 µl of Adaptor 1 and Adaptor 2 (10 µM), in separate ligation reactions, in a total volume of 10 µl at 16°C overnight, using 400 u of T4 DNA ligase (CLONTECH). Ligation was terminated with 1 µl of 0.2 M EDTA and heating at 72°C for 5 min.

The first hybridization was performed by adding 1.5 µl (600 ng) of driver cDNA to each of two tubes containing 1.5 µl (20 ng) Adaptor 1- and Adaptor 2- ligated tester cDNA. In a final volume of 4 µl, the samples were overlaid with mineral oil, denatured in an MJ Research thermal cycler at 98°C for 1.5 minutes, and then were allowed to hybridize for 8 hrs at 68°C. The two hybridizations were then mixed together with an additional 1 µl of fresh denatured driver cDNA and were allowed to hybridize overnight at 68°C. The second hybridization was then diluted in 200 µl of 20 mM Hepes, pH 8.3, 50 mM NaCl, 0.2 mM EDTA, heated at 70°C for 7 min. and stored at -20°C.

### PCR Amplification, Cloning and Sequencing of Gene Fragments Generated from SSH:

To amplify gene fragments resulting from SSH reactions, two PCR amplifications were performed. In the primary PCR reaction 1 µl of the diluted final hybridization mix was added to 1 µl of PCR primer 1 (10 µM), 0.5 µl dNTP mix (10 µM), 2.5 µl 10 x reaction buffer (CLONTECH) and 0.5 µl 50 x Advantage cDNA polymerase Mix (CLONTECH) in a final volume of 25 µl. PCR 1 was conducted using the following conditions: 75°C for 5 min., 94°C for 25 sec., then 27 cycles of 94°C for 10 sec, 66°C for 30 sec, 72°C for 1.5 min. Five separate primary PCR reactions were performed for each experiment. The products were pooled and diluted 1:10 with water. For the secondary PCR reaction, 1 µl from the pooled and diluted primary PCR reaction was added to the same reaction mix as used for PCR 1, except that primers NP1 and NP2 (10 µM) were used instead of PCR primer 1, PCR 2 was performed using 10-12 cycles of 94°C for 10 sec, 68°C for 30 sec, and 72°C for 1.5 minutes. The PCR products were analyzed using 2% agarose gel electrophoresis.

The PCR products were inserted into pCR2.1 using the T/A vector cloning kit (Invitrogen). Transformed *E. coli* were subjected to blue/white and ampicillin selection. White colonies were picked and arrayed into 96 well plates and were grown in liquid culture overnight. To identify inserts, PCR amplification was performed on 1 ml of bacterial culture using the conditions of PCR1 and MP1 and NP2 as primers. PCR products were analyzed using 2% agarose gel electrophoresis.

Bacterial clones were stored in 20% glycerol in a 96 well format. Plasmid DNA was prepared, sequenced, and subjected to nucleic acid homology searches of the GenBank, dBest, and NCR-CGAP databases.

### RT-PCR Expression Analysis:

First strand cDNAs can be generated from 1 µg of mRNA with oligo (dT)12-18 priming using the Gibco-BRL Superscript Preamplification system. The manufacturer's protocol was used which included an incubation for 50 min at 42°C with reverse transcriptase followed by RNAse H treatment at 37°C for 20 min. After completing the reaction, the volume can be increased to 200 µl with water prior to normalization. First strand cDNAs from 16 different normal human tissues can be obtained from Clontech,

Normalization of the first strand cDNAs from multiple tissues was performed by using the primers 5'acatcgccgcgctcgscgtcgacaa3' and 5'agccacacgcagctcattgtagaagg 3' to amplify β-actin. First strand cDNA (5 µl) were amplified in a total volume of 50 µl containing 0.4 µM primers, 0.2 µM each dNTPs, 1XPCR buffer (Clontech, 10 mM Tris-HCL, 15 mM MgCl₂, 50 mM KCl, pH8.3) and 1X Klentaq DNA polymerase (Clontech). Five µl of the PCR reaction can be removed at 18, 20, and 22 cycles and used for agarose gel electrophoresis. PCR was performed using an MJ Research thermal cycler under the following conditions: initial denaturation can be at 94°C for 15 sec, followed by a 18, 20, and 22 cycles of 94°C for 15, 65°C for 2 min, 72°C for 5 sec. A final extension at 72°C was carried out for 2 min. After agarose gel electrophoresis, the band intensities of the 283 b.p. β-actin bands from multiple tissues were compared by visual inspection. Dilution factors for the first strand cDNAs were calculated to result in equal β-actin band intensities in all tissues after 22 cycles of PCR. Three rounds of normalization can be required to achieve equal band intensities in all tissues after 22 cycles of PCR.

To determine expression levels of the 121F2A3 gene, 5 µl of normalized first strand cDNA were analyzed by PCR using 26, and 30 cycles of amplification. Semi-quantitative expression analysis can be achieved by comparing the PCR products at cycle numbers that give light band intensities. The primers used for RT-PCR were designed using the 121F2A3 SSH sequence and are listed below:
**121P2A3.1**
   5'- TGTCAATCAAATGAGAGGGCTACA 3'
**121P2A3.2**
   5'- CTGTTTGAGGCATAATCTTAAGTGG 3'

A typical RT-PCR expression study is shown in Figure 14. First strand cDNA was prepared from vital po 1 (liver, lung and kidney), vital pool 2 (pancreas, colon and stomach), LAPC xenograft pool (LAPC-4AD, LAPC-4AL, LAPC-9AD and LAPC-9AI), prostate cancer pool, bladder cancer pool, kidney cancer pool, colon cancer pool lung cancer pool, ovary cancer pool, breast cancer pool, and cancer metastasis pool. Normalization was performed t PCR using primers to actin and GAPDH. Semi-quantitative PCR, using primers to 121P2A3, was performed at 26 and 30 cycles of amplification. Results show strong expression of 121P2A3 in LAPC xenograft pool, bladder caner pool, kidney cancer pool, colon cancer pool, lung cancer pool, ovary cancer pool, breast cancer pool, and cancer metastasis pool. Expression of 121P2A3 was also detected in prostate cancer pooL Very low expression was detect in vital pool 1 and 2.

### Example 2: Full Length Cloning of 121P2A3

To isolate genes that are involved in the progression of androgen dependent (AD) prostate cancer to androgen independent (AI) cancer, an experiment was conducted with the LAPC-9 AD xenograft in male SCID mice. Mice that harbored LAPC-9 AD xenografts were castrated when the tumors reached a size of 1 cm in diameter. The tumors regressed in size and temporarily stopped producing the androgen dependent protein PSA. Seven to fourteen days post-castration, PSA levels were detectable again in the blood of the mice. Eventually the tumors develop an A1 phenotype and start growing again in the castrated males. Tumors were harvested at different time points after castration to identify genes that are turned on or off during the transition to androgen independence.

The gene 121P2A3 was derived from an LAPC-9 AD (no castration) minus LAPC-9AD (28 days post-castration) subtraction. The SSH DNA sequence (Figure 1) was designated 121P2A3. cDNA clone 121P2A3-clone 5 (Figure 4) was identified by screening an LAPC-9AD cDNA library (Lambda ZAP Express, Stratagene) using the 121P2A3 SSH DNA as a probe.

121P2A3 clone 5 cDNA was deposited under the terms of the Budapest Treaty on 1 March 2001, with the American Type Culture Collection (ATCC; 10801 University Blvd., Manassas, VA 20110-2209 USA) as plasmid 121P2A3-5, and bas been assigned Accession No. PTA-3138.

### Example 3: Chromosomal Mapping of the 121P2A3 Gene

The chromosomal localization of 121P2A3 was determined using the NCBI Human Genome web site (URL www.ncbi.nlm.nih.gov/genome/seq/page.cgi?F=HsBlast.html&&ORG=Hs). The mapping program placed 121P2A3 on chromosome 10q23.32, a genomic region found to be rearranged in certain cancers.

### Example 4: Expression analysis of 121P2A3 in normal tissues, cancer cell lines and patient samples

Analysis by RT-PCR demonstrates that 121P2A3 expression in multiple human cancer tissues (Figure 14). First strand cDNA was prepared from vital pool 1 (liver, lung and kidney), vital pool 2 (pancreas, colon and stomach), LAPC xenograft pool (LAPC-4AD, LAPC-4AI, LAPC-9AD and LAPC-9AI), prostate cancer pool, bladder cancer pool, kidney cancer pool, colon cancer pool, lung cancer pool, ovary cancer pool, breast cancer pool, and cancer metastasis pool. Normalization was performed by PCR using primers to actin and GAPDH. Semi-quantitative PCR, using primers to 121P2A3, was performed at 26 and 30 cycles of amplification. Results show strong expression of 121P2A3 in LAPC xenograft pool, bladder cancer pool, kidney cancer pool, colon cancer pool, lung cancer pool, ovary cancer pool, breast cancer pool, and cancer metastasis pool. Expression of 121P2A3 was also detected in prostate cancer pool. Very low expression was detected in vital pool I and 2.

Extensive northern blot analysis of 121P2A3 in 16 human normal tissues and in xenograft tissues confirms the expression observed by RT-PCR (Figure 15). Two multiple tissue northern blots (A and B; Clontech) both with 2 ug of mRNA/lane, and a LAPC xenograft blot with 10 ug of total RNA/lane (C) were probed with the 121P2A3 SSH sequence. Size standards in kilobases (kb) are indicated on the side. Results show expression of an approximately 2.7 kb121P2A3 transcript in testis. Lower level expression was also detected in thymus and colon but not in the other normal tissues tested. 121P2A3 expression is also shown in prostate cancer xenografts but not in normal prostate.

121P2A3 expression was detected in all cell lines tested (Figure 16). RNA was extracted from a number of human cancer cell lines. Northern blots with 10 ug of total RNA/lane were probed with the 121P2A3 SSH fragment. Results show expression in prostate (LAPC4AD, LAPC4AI, LAPC9AD, LAPC 9AI, LNCaP, PC-3, DUI45, Tsu-Prl and LAPC-4 CL), bladder (HT1197, SCaBER, UM-UC-3, TCCSUP, J82, 5637), 293T cell line, Ewing's sarcoma (RD-ES), pancreas (PANC-1, Bx PC-3, HPAC, Capan-1) colon (SK-CO-1, Caco-2, LoVo, T84, Colo205), breast (CAMA-1, DU4475, MCF-7, MDA-MB435s), testicular (NTERRA-2, NCCIT, TERA-1, TERA-2), cervical (A431), ovarian (OV-1063, PA-1, SW 626), brain (PFSK-1, T98G) and bone (SK-ES-1, HOS, U-2 OS, RD-ES) cancer cell lines. These results suggest that 121P2A3 is a testis specific gene that is upregulated in multiple cancers.

Expression of 121P2A3 in patient bladder cancer specimens is shown in Figure 17. RNA was extracted from normal bladder (Nb), bladder cancer cell lines (CL; UM-UC-3, J82, SCaBER), bladder cancer patient tumors (T) and normal adjacent tissue (N). Northern blots with 10 ug of total RNA, were probed with the 121P2A3 SSH sequence. Size standards in kilobases arc indicated on the side. Results show expression of 121P2A3 in patient bladder cancer tissues, and in all bladder cancer cell lines tested, but not in normal bladder.

Figure 13 shows that 121P2A3 was expressed in kidney cancer patient specimens. RNA was extracted from kidney cancer cell lines (CL: 769-P, A498, SW839), normal kidney (NK), kidney cancer patient tumors (T) and their normal adjacent tissues (N). Northern blots with 10 ug of total RNA were probed with the 121P2A3 SSH sequence. Size standards in kilobases are on the side. Results show expression of 121P2A3 in patient kidney tumor tissues and in all kidney cancer cell lines tested, but not in normal kidney.

121P2A3 is also expressed in stomach, and rectum patient cancer samples (Figure 19). The expression detected in normal adjacent tissues (isolated from diseased tissues) but not in normal tissues (isolated from healthy donors) indicates that these tissues are not fully normal and that 121P2A3 can be expressed in early stage tumors. 121P2A3 was also found to be highly expressed in the nine human cancer cell lines tested, the cervical carcinoma HeLa, the CML line K562, the PML line HL-60, the melanoma line G361, the lung carcinoma line A549, the lymphoblastic leukemia line MOLT-4, the colorectal carcinoma SW480, and Burkitt's lymphoma lines Daudi and Raji.

In order to assay for androgen regulation of 121P2A3 expression, LAPC-9AD tumor cells were injected in male mice (Figure 20). When tumor reached a palpable size (0.3-0.5cm in diameter), mice were castrated and tumors harvested at different time points following castration. RNA was isolated from the xenograft tissues. Northern blots with 10 ug of total RNA/lanc were probed with the 121P2A3 SSH fragment. Size standards in kilobases (kb) are indicated on the side. Results show expression of 121P2A3 is downregulated within 7 days of castration. The experimental samples were confirmed by testing for the expression of the androgen-regulated prostate cancer gene TMPRSS2, and the androgen-independent gene PHOR-1 (B). This experiment shows that, as expected, TMPRSS2 expression level goes down 7 days after castration, whereas the expression of PHOR-1 does not change. A picture of the ethidium-bromide staining of the RNA gel is also presented confirming the quality of the RNA.

121P2A3 expression is reminiscent of a cancer-testis gene. Its restricted normal tissue expression and the upregulation detected in human cancers indicate that 121P2A3 is therapeutic and prophylactic target and a diagnostic and prognostic marker for human cancers.

### Example 5: Transcript Variants of 121P2A3

Transcript variants are variants of mature mRNA from the same gene which arise by alternative transcription or alternative splicing, Alternative transcripts are transcripts from the same gene but start transcription at different points. Splice variants are mRNA variants spliced differently from the same transcript. In eukaryotes, when a multi-exon gene is transcribed from genomic DNA, the initial RNA is spliced to produce functional mRNA, which has only exons and is used for translation into an amino acid sequence. Accordingly, a given gene can have zero to many Alternative transcripts and each transcript can have zero to many splice variants. Each transcript variant has a unique exon makeup, and can have different coding and/or non-coding (5' or 3' end) portions, from the original transcript. Transcript variants can code for similar or different proteins with the same or a similar function or can encode proteins with different functions, and can be expressed in the same tissue at the same time, or in different tissues at the same time, or in the same tissue, at different times, or in different tissues at different times. Proteins encoded by transcript variants can have similar or different cellular or extracellular localizations, e.g., secreted versus intracellular.

Transcript variants are identified by a variety of art-accepted methods. For example, alternative transcripts and splice variants are identified by full-length cloning experiment or by use of full-length transcript and EST sequences. First, all human ESTs were grouped into clusters which show direct or indirect identity with each other. Second, ESTs in the same cluster were further grouped into sub-clusters and assembled into a consensus sequence. The original gene sequence is compared to the consensus sequence(s) or other full-length sequences. Each consensus sequence is a potential splice variant for that gene (see, e.g., URL www.doubletwist.com/products/c11_agentsOverview.jhtml). Even when a variant is identified that is not a full-length clone, that portion of the variant is very useful for antigen generation and for further cloning of the full-length splice variant, using technique known in the art,

Moreover, computer programs are available in the art that identity transcript variants based on genomic sequences. Genomic-based transcript variant identification programs include FgenesH (A. Salamov and V. Solovyev, "Ab initio gene finding in Drosophila genomic DNA," Genome Research. 2000 April; 10(4):516-22); Grail (URL compbio.ornl.gov/Grail-bin/EmptyGrailForm) and GenScan (URL genes.mit.edu/GENSCAN.html). For a general discussion of splice variant identification protocols see., e.g., Southan, C., A genomic perspective on human proteases, FEBS Lett. 2001 Jun 8;498(2-3):214-8; de Souza, S.J., et al., Identification of human chromosome 22 transcribed sequences with ORF expressed sequence tags, Proc. Natl Acad Sci USA. 2000 Nov 7; 97(23):12690-3.

To further confirm the parameters of a transcript variant, a variety of techniques are available in the art, such as full-length cloning, proteomic validation, PCR-based validation, and 5' RACE validation, etc. (see e.g., Proteoroic Validation: Brennan, S.O., et al., Albumin banks peninsula: a new termination variant characterized by electrospray mass spectrometry, Biochem Biophys Acta. 1999 Aug 17;1433(1-2):321-6; Ferranti P, et al., Differential splicing of pre-messenger RNA produces multiple forms of mature caprine alpha(s1)-casein, Eur J Biochem. 1997 Oct 1;249(1):1-7. For PCR-based Validation: Wellmann S, et al., Specific reverse transcription-PCR quantification of vascular endothelial growth factor (VEGF) splice variants by LightCycler technology, Glin Chem. 2001 Apr,47(4)-654-60; Jia, H.P., et al., Discovery of new human beta-defensins using a genomics-based approach, Gene. 2001 Jan 24; 263(1-2):211-8. For PCR-based and 5' RACE Validation: Brigle, K.E., et al., Organization of the marine reduced folate carrier gene and identification of variant splice forms, Biochem Biopbys Acta. 1997 Aug 7; 1353(2): 191-8).

It is known in the art that genomic regions are modulated in cancers. When the genomic region to which a gene maps is modulated in a particular cancer, the alternative transcripts or splice variants of the gene are modulated as well. Disclosed herein is that 121P2A3 has a particular expression profile related to cancer. Alternative transcripts and splice variants of 121P2A3 may also be involved in cancers in the same or different tissues, thus serving as tumor-associated markers/antigens.

The exon composition of the original transcript, designated as 121P2A3 v.1, is shown in Table LIII. Using the full-length gene and EST sequences, one transcript variant was identified, designated as 121P2A3 v.2. Compared with 121P2A3 v.1, transcript variant 121P2A3 v.2 has a shorter exon 2, as shown in Figure 12. All other exons are the same corresponding exons of 121P2A3 v.1. Theoretically, each different combination of exons in spatial order, e.g. exons 2 and 3, is a potential splice variant Figure 12 shows the schematic alignment of exons of the two transcript variants.

Table LIV shows nucleotide sequence of the transcript variant. Table LV shows the alignment of the transcript variant with nucleic acid sequence of 121P2A3 v-1. Table LVI lays out amino acid translation-of the transcript variant for the identified reading frame orientation. Table LVII displays alignments of the amino acid sequence encoded by the splice variant with that of 121P2A3 v.1.

### Example 6: Single Nucleotide Polymorphisms of 121P2A3

A Single Nucleotide Polymorphism (SNP) is a single base pair variation in a nucleotide sequence at a specific location. At any given point of the genome, there are four possible nucleotide base pairs: A/T, C/G, G/C and T/A. Genotype refers to the specific base pair sequence of one or more locations in the genome of an individual. Haplotype refers to the base pair sequence of more than one location on the same DNA molecule (or the same chromosome in higher organisms), often in the context of one gene or in the context of several tightly linked genes. SNPs that occur on a cDNA are called cSNPs. These cSNPs may change amino acids, of the protein encoded by the gene and thus change the functions of the protein. Some SNPs cause inherited diseases; others contribute to quantitative variations in phenotype and reactions to environmental factors including diet and drugs among individuals. Therefore, SNPs and/or combinations of alleles (called haplotypes) have many applications, including diagnosis of inherited diseases, determination of drug reactions and dosage, identification of genes responsible for diseases, and analysis of the genetic relationship between individuals (P. Nowotny, J. M. Kwon and A. M. Goate, " SNP analysis to dissect human traits," Curr. Opin. Neurobiol. 2001 Oct; 11(5):637-641; M. Pirmohamed and B. K. Park, "Genetic susceptibility to adverse drug reactions," Trends Pharmacol. Sci. 2001 Jun; 22(6):298-305; J. H. Riley, C. J. Allan, E. Lai and A, Roses, " The use of single nucleotide polymorphisms in the isolation of common disease genes," Pharmacogenomcis. 2000 Feb; 1(1):39-47; R. Judson, J. C. Stephens and A. Windemuth, "The predictive power of haplotypes in clinical response," Pharmacogenomics. 2000 feb; 1(1):15-26).

SNPs are identified by a variety of art-accepted method (P. Bean, "The promising voyage of SNP target discovery," Am. Clin. Lab. 2001 Oct-Nov; 20(9):18-20; K. M. Weiss, "In search of human variation," Genome Res. 1998 Jul; 8(7):691-697; M. M. She, "Enabling large-scale pharmacogenetic studies by high-throughput mutation detection and genotyping technologies," Clin. Chem. 2001 Feb; 47(2):164-172). For example, SNPs are identified by sequencing DNA fragments that show polymorphism by gel-based methods such as restriction fragment length polymorphism (RFLP) and denaturing gradient gel electrophoresis (DGGE). They can also be discovered by direct sequencing of DNA samples pooled from different individuals or by comparing sequences from different DNA samples. With the rapid accumulation of sequence data in public and private databases, one can discover SNPs by comparing sequences using computer programs (Z. Gu, L. Hillier and P. Y. Kwok, "Single nucleotide polymorphism hunting in cyberspace," Hum. Mutat 1998; 12(4):221-225). SNPs can be verified and genotype or haplotype of an individual can be determined by a variety of methods including direct sequencing and high throughput microarrays (P. Y. Kwok, "Methods for genotyping single nucleotide polymorphisms," Annu. Rev. Genomics Hum. Genet. 2001; 2:235-258; M. Kokoris, K. Dix, K. Moynihan, J. Mathis, B. Erwin, P. Grass, B. Hines and A. Duesterhoeft, "High-throughput SNP genotyping with the Masscode system," Mol. Diagn. 2000 Dec; 5(4):329-340).

Using the methods described above, seven SNPs were identified in the original transcript, 121P2A3 v.1, at positions 345 (C/G), 469 (G/A), 511 (A/C), 1175 (T/C), 1307 (A/T), 1478 (A/G) and 1911 (T/C). The transcripts or proteins with alternative alleles were designated as variants 121P2A3 v.3, v.4, v.5, v.6, v.7, v.8 and v.9. Figure 10 and Figure 12 show the schematic alignment of the nucleotide variants. Figure 11 shows the schematic alignment of protein variants, corresponding to nucleotide variants. Nucleotide variants that code for the same amino acid sequence as variant 1 are not shown in Figure 11, These alleles of the SNPs, though shown separately here, can occur in different combinations (haplotypes) and in any one of the transcript variants (such as 121P2A3 v.2) that contains the sequence context of the SNPs. Figure 4A and Table LVIII show detailed sequence alignments of the variant proteins; variant locations are shaded.

### Example 7: Production Of Recombinant 121 p2a3 In Prokaryotic Systems

To express recombinant 121P2A3 and 121P2A3 variants in prokaryotic cells, the full or partial length 121P2A3 and 121P2A3 variant cDNA sequences are cloned into any one of a variety of expression vectors known in the art One or more of the following regions of 121P2A3 variants are expressed: the full length sequence presented in Figures 2 and 3, or any 8, 9, 10, 11, 12, 13, 14, 15, 16, 17, 18, 19, 20, 21, 22, 23, 24, 25, 26, 27, 28, 29, 30 or more contiguous amino acids from 121P2A3, variants, or analogs thereof.

### A. In vitro transcription and translation constructs:

pCRII: To generate 121P2A3 sense and anti-sense RNA probes for RNA *in situ* investigations, pCRII constructs (Invitrogen, Carlsbad CA) are generated encoding either all or fragments of the 121P2A3 cDNA. The pCRII vector has Sp6 and T7 promoters flanking the insert to drive the transcription of 121P2A3 RNA for use as probes in RNA *in situ* hybridization experiments. These probes are used to analyze the cell and tissue expression of 121P2A3 at the RNA levels. Transcribed 121P2A3 RNA representing the cDNA amino acid coding region of the 121P2A3 gene is used in *in vitro* translation systems such as the TnT^{™} Coupled Reticulolysate System (Promega, Corp., Madison, WI) to synthesize 121P2A3 protein.

### B. Bacterial Constructs:

pGEX Constructs: To generate recombinant 121P2A3 proteins in bacteria that are fused to the Glutathione S-transferase (GST) protein, all or parts of the 121P2A3 cDNA protein coding sequence are cloned into the pGEX family of GST-fusion vectors (Amersham Pharmacia Biotech, Piscataway, NJ). These constructs allow controlled expression of recombinant 121P2A3 protein sequences with GST fused at the amino-terminus and a six histidine epitope (6X His) at the carboxyl-terminus. The GST and 6X His tags permit purification of the recombinant fusion protein from induced bacteria with the appropriate affinity matrix and allow recognition of the fusion protein with anti-GST and anti-His antibodies. The 6X His tag is generated by adding 6 histidine codons to the cloning primer at the 3' end, e.g., of the open reading frame (ORF). A proteolytic cleavage site, such as the PreScission^{™} recognition site in pGEX-6P-1, may be employed such that it permits cleavage of the GST tag from 121P2A3-related protein. The ampicillin resistance gene and pBR322 origin permits selection and maintenance of the pGEX plasmids in *E*. *coli.*

pMAL Constructs: To generate, in bacteria, recombinant 121P2A3 proteins that are fused to maltose-binding protein (MBP), all or parts of the 121P2A3 cDNA protein coding sequence arc fused to the MBP gene by cloning into the pMAL-c2X and pMAL-p2X vectors (New England Biolabs, Beverly, MA). These constructs allow controlled expression of recombinant 121P2A3 protein sequences with MBP fused at the amino-terminus and a 6X His epitope tag at the carboxyl-terminus. The MBP and 6X His tags permit purification of the recombinant protein from induced bacteria with the appropriate affinity matrix and allow recognition of the fusion protein with anti-MBP and anti-His antibodies. The 6X His epitope tag is generated by adding 6 histidine codons to the 3' cloning primer. A Factor Xa recognition site permits cleavage of the pMAL tag from 121P2A3. The pMAL-c2X and pMAL-p2X vectors are optimized to express the recombinant protein in the cytoplasm or periplasm respectively. Periplasm expression enhances folding of proteins with disulfide bonds.

pET Constructs: To express 121P2A3 in bacterial cells, all or parts of the 121P2A3 cDNA protein coding sequence are cloned into the pET family of vectors (Novagen, Madison, WI). These vectors allow tightly controlled expression of recombinant 121P2A3 protein in bacteria with and without fusion to proteins that enhance solubility, such as NusA and thioredoxin (Trx), and epitope tags, such as 6X His and S-Tag^{™} that aid purification and detection of the recombinant protein. For example, constructs are made utilizing pET NusA fusion system 43.1 such that regions of the 121P2A3 protein are expressed as amino-terminal fusions to NusA.

### C. Yeast Constructs:

pESC Constructs: To express 121P2A3 in the yeast species *Saccharomyces cerevisiae* for generation of recombinant protein and functional studies, all or parts of the 121P2A3 cDNA protein coding sequence arc cloned into the pESC family of vectors each of which contain 1 of 4 selectable markers, HIS3, TRP1, LEU2, and URA3 (Stratagene, La Jolla, CA). These vectors allow controlled expression from the same plasmid of up to 2 different genes or cloned sequences containing either Flag^{™} or Myc epitope tags in the same yeast cell. This system is useful to confirm protein-protein interactions of 121P2A3. In addition, expression in yeast yields similar post-translational modifications, such as glycosylations and phosphorylations, that are found when expressed in eukaryotic cells.

pESP Constructs: To express 121P2A3 in the yeast species *Saccharomyces pombe*, all or parts of the 121P2A3 cDNA protein coding sequence are cloned into the pESP family of vectors. These vectors allow controlled high level of expression of a 121P2A3 protein sequence that is fused at either the amino terminus or at the carboxyl terminus to GST which aids purification of the recombinant protein. A Flag^{™} epitope tag allows detection of the recombinant protein with anti- Flag^{™} antibody.

### Example 8: Production of Recombinant 121P2A3 in Eukaryotic Systems

### A. Mammalian Constructs:

To express recombinant 121P2A3 in eukaryotic cells, the full or partial length 121P2A3 cDNA sequences can be cloned into any one of a variety of expression vectors known in the art One or more of the following regions of 121P2A3 are expressed in these constructs, amino acids 1 to 464 of 121P2A3 v.1, v.3, v.4, v.6, v.7 and v.8, amino acids 1 to 295 of 121P2A3 v.2, or any 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more contiguous amino acids from 121P2A3, variants, or analogs thereof. In certain embodiments a region of a specific variant of 121P2A3 is expressed that encodes an amino acid at a specific position which differs from the amino acid of any other variant found at that position. In other embodiments, a region of a variant of 121P2A3 is expressed that lies partly or entirely within a sequence that is unique to that variant.

The constructs can be transfected into any one of a wide variety of mammalian cells such as 293T cells. Transfected 293T cell lysates can be probed with the anti-121P2A3 polyclonal serum, described herein.

**pcDNA4/HisMax Constructs:** To express 121P2A3 in mammalian cells, a 121P2A3 ORF, or portions thereof, of 121P2A3 are cloned into pcDNA4/HisMax Version A (Invitrogen, Carlsbad, CA). Protein expression is driven from the cytomegalovirus (CMV) promoter and the SP16 translational enhancer. The recombinant protein has Xpress^{™} and six histidine (6X His) epitopes fused to the amino-terminus. The pcDNA4/HisMax vector also contains the bovine growth hormone (BGH) polyadenylation signal and transcripton termination sequence to enhance mRNA stability along with the SV40 origin for episomal replication and simple vector rescue in cell lines expressing the large T antigen. The Zeocin resistance gene allows for selection of mammalian cells expressing the protein and the ampicillin resistance gene and ColE1 origin permits selection and maintenance of the plasmid in E. *coli*.

**pcDNA3.1/MycHis Constructs:** To express 121P2A3 in mammalian cells, a 121P2A3 ORF, or portions thereof, of 121P2A3 with a consensus Kozak translation initiation site was cloned into pcDNA3.1/MycHis Version A (Invitrogen, Carlsbad, CA). Protein expression is driven from the cytomegalovirus (CMV) promoter. The recombinant protein has the myc epitope and 6X His epitope fused to the carboxyl-terminus. The pcDNA3.1/MycHis vector also contains the bovine growth hormone (BGH) polyadenylation signal and transcription termination sequence to enhance mRNA stability, along with the SV40 origin for episomal replication and simple vector rescue in cell lines expressing the large T antigen. The Neomycin resistance gene was used, as it allows for selection of mammalian cells expressing the protein and the ampicillin resistance gene and ColE1 origin permits selection and maintenance of the plasmid in *E*. *coli.* Results of expression from 121P2A3.pcDNA3.1/MycHis construct are shown in Figure 21.

**pcDNA3.1/CT-GFP-TOPO Construct:** To express 121P2A3 in mammalian cells and to allow detection of the recombinant proteins using fluorescence, a 121P2A3 ORF, or portions thereof, with a consensus Kozak translation initiation site are cloned into pcDNA3.1/CT-GFP-TOPO (Invitrogen, CA). Protein expression is driven from the cytomegalovirus (CMV) promoter, The recombinant proteins have the Green Fluorescent Protein (GFP) fused to the carboxyl-terminus facilitating non-invasive, *in vivo* detection and cell biology studies. The pcDNA3.1CT-GFP-TOPO vector also contains the bovine growth hormone (BGH) polyadenylation signal and transcription termination sequence to enhance mRNA stability along with the SV40 origin for episomal replication and simple vector rescue in cell lines expressing the large T antigen. The Neomycin resistance gene allows for selection of mammalian cells that express the protein, and the ampicillin resistance gene and ColE1 origin permits selection and maintenance of the plasmid in *E. coli.* Additional constructs with an amino-terminal GFP fusion are made in pcDNA3.1/NT-GFP-TOPO spanning the entire length of a 121P2A3 protein.

**PAPtag:** A 121P2A3 ORF, or portions thereof, is cloned into pAPtag-5 (GenHunter Corp. Nashville, TN). This construct generates an alkaline phosphatase fusion at the carboxyl-terminus of a 121P2A3 protein while fusing the IgGκ signal sequence to the amino-terminus. Constructs are also generated in which alkaline phosphatase with an amino-terminal IgGκ signal sequence is fused to the amino-terminus of a 121P2A3 protein. The resulting recombinant 121P2A3 proteins are optimized for secretion into the media of transfected mammalian cells and can be used to identify proteins such as ligands or receptors that interact with 121P2A3 proteins. Protein expression is driven from the CMV promoter and the recombinant proteins also contain myc and 6X His epitopes fused at the carboxyl-terminus that facilitates detection and purification. The Zeocin resistance gene present in the vector allows for selection of mammalian cells expressing the recombinant protein and the ampicillin resistance gene permits selection of the plasmid in *E*. *coli.*

**ptag5:** A 121P2A3 ORF, or portions thereof, is cloned into pTag-5. This vector is similar to pAPtag but without the alkaline phosphatase fusion. This construct generates 121P2A3 protein with an amino-terminal IgGκ signal sequence and myc and 6X His epitope tags at the carboxyl-terminus that facilitate detection and affinity purification. The resulting recombinant 121P2A3 protein is optimized for secretion into the media of transfected mammalian cells, and is used as immunogen or ligand to identify proteins such as ligands or receptors that interact with the 121P2A3 proteins. Protein expression is driven from the CMV promoter. The Zeocin resistance gene present in the vector allows for selection of mammalian cells expressing the protein, and the ampicillin resistance gene permits selection of the plasmid in *E*. *coli.*

**PsecFc:** A 121P2A3 ORF, or portions thereof, is also cloned into psecFc. The psecFc vector was assembled by cloning the human immunoglobulin G1 (IgG) Fc (hinge, CH2, CH3 regions) into pSecTag2 (Invitrogen, California). This construct generate an IgG1 Fc fusion at the carboxyl-terminus of the 121P2A3 proteins, while fusing the IgGK signal sequence to N-terminus. 121P2A3 fusions utilizing the murine IgG1 Fc region are also used. The resulting recombinant 121P2A3 proteins are optimized for secretion into the media of transfected mammalian cells, and can be used as immunogens or to identify proteins such as ligands or receptors that interact with 121P2A3 protein. Protein expression is driven from the CMV promoter. The hygromycin resistance gene present in the vector allows for selection of mammalian cells that express the recombinant protein, and the ampicillin resistance gene permits selection of the plasmid in *E*. *coli.*

**pSRα Constructs:** To generate mammalian cell lines that express 121P2A3 constitutively, 121P2A3 ORF, or portions thereof, of 121P2A3 are cloned into pSRα constructs. Amphotropic and ecotropic retroviruses are generated by transfection of pSRα constructs into the 293T-10A1 packaging line or co-transfection of pSRα and a helper plasmid (containing deleted packaging sequences) into the 293 cells, respectively. The retrovirus is used to infect a variety of mammalian cell lines, resulting in the integration of the cloned gene, 121P2A3, into the host cell-lines. Protein expression is driven from a long terminal repeat (LTR). The Neomycin resistance gene present in the vector allows for selection of mammalian cells that express the protein, and the ampicillin resistance gene and ColE1 origin permit selection and maintenance of the plasmid in *E*. *coli.* The retroviral vectors can thereafter be used for infection and generation of various cell lines using, for example, PC3, NIH 3T3, TsuPr1, 293 or rat-1 cells.

Additional pSRα constructs are made that fuse an epitope tag such as the FLAG^{™} tag to the carboxyl-terminus of 121P2A3 sequences to allow detection using anti-Flag antibodies. For example, the FLAG^{™} sequence 5' gat tac aag gat gac gac gat aag 3' is added to cloning primer at the 3' end of the ORF. Additional pSRα constructs are made to produce both amino-terminal and carboxyl-terminal GFP and myc/6X His fusion proteins of the full-length 121P2A3 proteins.

**Additional Viral Vectors:** Additional constructs are made for viral-mediated delivery and expression of 121P2A3. High virus titer leading to high level expression of 121P2A3 is achieved in viral delivery systems such as adenoviral vectors and herpes amplicon vectors. A 121P2A3 coding sequences or fragments thereof are amplified by PCR and subcloned into the AdEasy shuttle vector (Stratagene). Recombination and virus packaging arc performed according to the manufacturer's instructions to generate adenoviral vectors. Alternatively, 121P2A3 coding sequences or fragments thereof are closed into the HSV-1 vector (Imgenex) to generate herpes viral vectors. The viral vectors are thereafter used for inflection of various cell lines such as PC3, NIH 3T3, 293 or rat-1 cells.

**Regulated Expression Systems:** To control expression of 121P2A3 in mammalian cells, coding sequences of 121P2A3, or portions thereof, are cloned into regulated mammalian expression systems such as the T-ReX System (Invitrogen), the GeneSwitch System (Invitrogen) and the tightly-regulated Ecdysone System (Sratagene). These systems allow the study of the temporal and concentration dependent effects of recombinant 121P2A3. These vectors are thereafter used to control expression of 121P2A3 in various cell lines such as PC3, NIH 3T3, 293 or rat-1 cells.

### B. Baculovirus Expression Systems

To generate recombinant 121P2A3 proteins in a baculovirus expression system, 121P2A3 ORF, or portions thereof, are cloned into the baculovirus transfer vector pBlueBac 4.5 (Invitrogen), which provides a His-tag at the N-terminus. Specifically, pBlueBac-121P2A3 is co-transfected with helper plasmid pBac-N-Blue (Invitrogen) into SF9 (*Spodoptera frugiperda*) insect cells to generate recombinant baculovirus (see Invitrogen instruction manual for details). Baculovirus is then collected from cell supernatant and purified by plaque assay.

Recombinant 121P2A3 protein is then generated by infection of HighFive insect cells (Invitrogen) with purified baculovirus. Recombinant 121P2A3 protein can be detected using anti-121P2A3 or anti-His-tag antibody. 121P2A3 protein can be purified and used in various cell-based assays or as immunogen to generate polyclonal and monoclonal antibodies specific for 121P2A3.

### Example 9 Antigenicity Profiles and Secondary Structure

Figure 5, Figure 6, Figure 7, Figure 8, and Figure 9 depict graphically five amino acid profiles of 121P2A3 variants 1 and 2, each assessment available by accessing the ProtScale website (URL www.expasy.ch/cgi-bin/protscale.pl) on the ExPasy molecular biology server.

These profiles: Figure 5, Hydrophilicity, (Hopp T.P., Woods K.R., 1981. Proc. Natl. Acad. Sci. U.S.A. 78:3824-3828); Figure 6, Hydropathicity, (Kyte J., Doolittle R.F., 1982. J. Mol. BioL 157:105-132); Figure 7, Percentage Accessible Residues (Janin J., 1979 Nature 277:491-492); Figure 8, Average Flexibility, (Bhaskaran R., and Ponnuswarny P.K., 1988. Int. J. Pept. Protein Res. 32:242-255); Figure 9, Beta-turn (Deltage, G., Roux B. 1987 Protein Engineering 1:289-294); and optionally others available in the art, such as on the ProtScale website, were used to identify antigenic regions of the 121P2A3 protein. Each of the above amino acid profiles of 121P2A3 were generated using the following ProtScale parameters for analysis: 1) A window size of 9; 2) 100% weight of the window edges compared to the window center; and, 3) amino acid profile values normalized to lie between 0 and 1.

Hydrophilicity (Figure 5), Hydropathicity (Figure 6) and Percentage Accessible Residues (Figure 7) profiles were used to determine stretches of hydrophilic amino acids (i.e., values greater than 0.5 on the Hydrophilicity and Percentage Accessible Residues profiles, and values less than 0.5 on the Hydropathicity profile). Such regions are likely to be exposed to the aqueous environment, be present on the surface of the protein, and thus available for immune recognition, such as by antibodies.

Average Flexibility (Figure 8) and Beta-turn (Figure 9) profiles determine stretches of amino acids (i.e., values greater than 0.5 on the Beta-turn profile and the Average Flexibility profile) that are not constrained in secondary structures such as beta sheets and alpha helices. Such regions are also more likely to be exposed on the protein and thus accessible to immune recognition, such as by antibodies.

Antigenic sequences of the 121P2A3 protein indicated, e.g., by the profiles set forth in Figure 5, Figure 6, Figure 7, Figure 8, and/or Figure 9 are used to prepare immunogens, either peptides or nucleic acids that encode them, to generate therapeutic and diagnostic anti-121P2A3 antibodies. The immunogen can be any 5, 6, 7, 8, 9, 10, 11, 12, 13, 14, 15, 16, 17, 18, 19, 20, 21, 22, 23, 24, 25, 30, 35, 40, 45, 50 or more than 50 contiguous amino acids, or the corresponding nucleic acids that encode them, from the 121P2A3 protein or variants listed in Figures 2 and 3. In particular, peptide immunogens can comprise, a peptide region of at least 5 amino acids of Figures 2 and 3 in any whole number increment that includes an amino acid position having a value greater than 0.5 in the Hydrophilicity profiles of Figure 5; a peptide region of at least 5 amino acids of Figures 2 and 3 in any whole number increment that includes an amino acid position having a value less than 0.5 in the Hydropathicity profile of Figures 6 ; a peptide region of at least 5 amino acids of Figures 2 and 3 in any whole number increment that includes an amino acid positions having a value greater than 0.5 in the Percent Accessible Residues profiles of Figure 7; a peptide region of at least 5 amino acids of Figures 2 and 3 in any whole number increment that includes an amino acid position having a value greater than 0.5 in the Average Flexibility profiles on Figure 8; and, a peptide region of at least 5 amino acids of Figures 2 and 3 in any whole number increment that includes an amino acid position having a value greater than 0.5 in the Beta-turn profile of Figure 9. Peptide immunogens can also comprise nucleic acids that encode any of the forgoing.

All immunogens, peptide or nucleic acid, can be embodied in human unit dose form, or comprised by a composition that includes a pharmaceutical excipient compatible with human physiology.

The secondary structure of 121P2A3 protein, namely the predicted presence and location of alpha helices, extended strands, and random coils, is predicted from the primary amino acid sequence using the HNN- Hierarchical Neural Network method (Guermeur, 1997, URL pbil.ibcp.ft/cgi-bin/npsa automat.pl?page=npsa nn.html), accessed from the ExPasy molecular biology server (URL www.expasy.ch/tools/). The analysis indicates that 121P2A3 protein is composed of 63.79% alpha helix, 4.74% extended strand, and 31.47% random coil (Figure 13).

Analysis for the potential presence of transmembrane domains in the 121P2A3 variant proteins was carried out using a variety of transmembrane prediction algorithms accessed from the ExPasy molecular biology server (URL www.expasy.ch/tools/). The programs do not predict the presence of transmembrane domains in 121P2A3 protein, suggesting that that it is a soluble protein.

### Example 10: Generation of 121P2A3 Polyclonal Antibodies

Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injection. In addition to immunizing with a full length 121P2A3 protein variant, computer algorithms are employed in design of immunogens that, based on amino acid sequence analysis contain characteristics of being antigenic and available for recognition by the immune system of the immunized host (see the Example entitled "Antigenicity Profiles"). Such regions would be predicted to be hydrophilic, flexible, in beta-turn conformation, and be exposed on the surface of the protein (see, e.g., Figure 5, Figure 6, Figure 7, Figure 8, or Figure 9 for amino acid profiles that indicate such regions of 121P2A3 protein).

For example, recombinant bacterial fusion proteins or peptides containing hydrophilic, flexible, beta-turn regions of 121P2A3 protein are used as antigens to generate polyclonal antibodies in New Zealand White rabbits. For example, such regions include, but are not limited to, amino acids 1-38, amino acids 97-12, amino acids, 213-238, and amino acids 284-330. It is useful to conjugate the immunizing agent to a protein known to be immunogenic in the mammal being immunized. Examples of such immunogenic proteins include, but are not limited to, keyhole limpet hemocyanin (KLH), serum albumin, bovine thyroglobulin, and soybean trypsin inhibitor. In one embodiment, a peptide encoding amino acids 1-38 of 121P2A3 variant 1 is conjugated to KLH and used to immunize the rabbit. Alternatively the immunizing agent may include all or portions of the 121P2A3 variant proteins, analogs or fusion proteins thereof. For example, the 121P2A3 variant 1 amino acid sequence can be fused using recombinant DNA techniques to any one of a variety of fusion protein partners that are well known in the art, such as glutathione-S-transferase (GST) and HIS tagged fusion proteins. Such fusion proteins are purified from induced bacteria using the appropriate affinity matrix.

In one embodiment, a GST-fusion proteins encoding amino acids 1-150 of 121P2A3 variant 1, is produced, purified and used as immunogen. Other recombinant bacterial fusion proteins that may be employed include maltose binding protein, LacZ, thioredoxin, NusA, or an immunoglobulin constant region (see the section entitled "Production of 121P2A3 in Prokaryotic Systems" and Current Protocols In Molecular Biology, Volume 2, Unit 16, Frederick M. Ausubul et al. eds., 1995; Linsley, P.S., Brady, W., Urnes, M., Grosmaire, L., Damle, N., and Ledbetter, L.(1991) J.Exp. Med. 174, 561-566).

In addition to bacterial derived fusion proteins, mammalian expressed protein antigens are also used. These antigens are expressed from mammalian expression vectors such as the Tag5 and Fc-fusion vectors (see the section entitled "Production of Recombinant 121P2A3 in Eukaryotic Systems"), and retain post-translational modifications such as glycosylations found in native protein. In one embodiment, amino acids 1-464 of variant 1, is cloned into the Tag5 mammalian secretion vector. The recombinant protein is purified by metal chelate chromatography from tissue culture supernatants of 293T cells stably expressing the recombinant vector. The purified Tag5 121P2A3 protein is then used as immunogen.

During the immunization protocol it is useful to mix or emulsify the antigen in adjuvants that enhance the immune response of the host animal. Example of adjuvants include, but are not limited to, complete Freund's adjuvant (CFA) and MPL-TDM adjuvant (monophosphoryl Lipid A, synthetic trehalose dicorynomycolate).

In a typical protocol, rabbits are initially immunized subcutaneously with up to 200 µg, typically 100-200 µg, of fusion protein or peptide conjugated to KLH mixed in complete Freund's adjuvant (CFA). Rabbits are then injected subcutaneously every two weeks with up to 200 µg, typically 100-200 µg, of the immunogen in incomplete Freund's adjuvant (IFA). Test bleeds are taken approximately 7-10 days following each immunization and used to monitor the titer of the antiserum by ELISA.

- To test reactivity and specificity of immune serum, such as the rabbit serum derived from immunization with the Tag5 -121P2A3 protein, the full-length 121P2A3 cDNA is cloned into pCDNA 3.1 myc-his expression vector (Invitrogen, see the Example entitled "Production of Recombinant 121P2A3 in Eukaryotic. Systems"). After transfection of the constructs into 293T cells, cell lysates are probed with the anti-121P2A3 serum and with anti-His antibody. (Santa Cruz Biotechnologies, Santa Cruz, CA) to determine specific reactivity to denatured 121P2A3 protein using the Western blot technique. Figure 21 shows expression of Myc His epitope tagged 121P2A3 variant 1 protein in 293T cells as detected by an anti-His antibody. In addition, the immune serum is tested by fluorescence microscopy, flow cytometry and immunoprecipitation against 293T and other recombinant 121P2A3-expressing cells to determine specific recognition of native protein. Western blot, immunoprecipitation, fluorescent microscopy, and flow cytometric techniques using cells that endogenously express 121P2A3 are also carried out to test reactivity and specificity.

Anti-serum from rabbits immunized with 121P2A3 variant fusion proteins, such as GST and MBP fusion proteins, are purified by depletion of antibodies reactive to the fusion partner sequence by passage over an affinity column containing the fusion partner either alone or in the context of an irrelevant fusion protein. For example, antiserum derived from a GST-121P2A3 variant 1 fusion protein encoding amino acids 1-150 is first purified by passage over a column of GST protein covalently coupled to AffiGel matrix (BioRad, Hercules, Calif.). The antiserum is then affinity purified by passage over a column composed of a MBP-fusion protein also encoding amino acids 1-150 covalently coupled to Affigel matrix. The serum is then further purified by protein G affinity chromatography to isolate the IgG fraction. Sera from other His-tagged antigens and peptide immunized rabbits as well as fusion partner depleted sera are affinity purified by passage over a column matrix composed of the original protein immunogen or free peptide.

### Example 11: Generation of 121P2A3 Monoclonal Antibodies (mAbs)

In one embodiment, therapeutic mAbs to 121P2A3 variants comprise those that react with epitopes specific for each variant protein or specific to sequences in common between the variants that would disrupt or modulate the biological function of the 121P2A3 variants, for example those that would disrupt the interaction with ligands and binding partners. Immunogens for generation of such mAbs include those designed to encode or contain the entire 121P2A3 protein variant sequence, regions of the 121P2A3 protein variants predicted to be antigenic from computer analysis of the amino acid sequence (see, e.g., Figure 5, Figure 6, Figure 7, Figure 8, or Figure 9, and the Example entitled "Antigenicity Profiles"). Immunogens include peptides, recombinant bacterial proteins, and mammalian expressed Tag 5 proteins and human and murine IgG FC fusion proteins. In addition, cells engineered to express high levels of a respective 121P2A3 variant, such as 293T-121P2A3 variant 1 or 300.19-121P2A3 variant Imurine Pre-B cells, are used to immunize mice.

To generate mAbs to a 121P2A3 variant, mice are first immunized intraperitoneally (IP) with, typically, 10-50 µg of protein immunogen or 10⁷ 121P2A3-expressing cells mixed in complete Freund's adjuvant. Mice are then subsequently immunized IP every 2-4 weeks with, topically, 10-50 µg of protein immunogen or 10⁷ cells mixed in incomplete Freund's adjuvant. Alternatively, MPL-TDM adjuvant is used in immunizations. In addition to the above protein and cell-based immunization strategic, a DNA-based immunization protocol is employed in which a mammalian expression vector encoding a 121P2A3 variant sequence is used to immune mice by direct injection of the plasmid DNA. For example, amino acids 1-464 is cloned into the Tag5 mammalian secretion vector and the recombinant vector is used as immunogen. In another example the same amino acids are cloned into an Fc-fusion secretion vector in which the 121P2A3 variant 1 sequence is fused at the amino-terminus to an IgK leader sequence and at the carboxyl-terminus to the coding sequence of the human or murine IgG Fc region. This recombinant vector is then used as immunogen. The plasmid immunization protocols are used in combination with purified proteins expressed from the same vector and with cells expressing the respective 121P2A3 variant.

During the immunization protocol, test bleeds are taken 7-10 days following an injection to monitor titer and specificity of the immune response. Once appropriate reactivity and specificity is obtained as determined by ELISA, Western blotting, immunoprecipitation, fluorescence microscopy, and flow cytometric analyses, fusion and hybridoma generation is then carried out with established procedures well known in the art (see, e.g., Harlow and Lane, 1988).

In one embodiment for generating 121P2A3 monoclonal antibodies, a Tag5-121P2A3 variant 1 antigen encoding amino acids 1-464, is expressed and purified from stably transfected 293T cells. Balb C mice are initially immunized intraperitoneally with 25 µg of the Tag5-121P2A3 variant 1 protein mixed in complete Freund's adjuvant. Mice are subsequently immunized every two weeks with 25 µg of the antigen mixed in incomplete Freund's adjuvant for a total of three Immunizations. ELISA using the Tag5 antigen determines the titer of serum from immunized mice. Reactivity and specificity of serum to full length 121P2A3 variant 1 protein is monitored by Western blotting, immunoprecipitation and flow cytometry using 293T cells transfected with an expression vector encoding the 121P2A3 variant 1 cDNA (see e.g., the Example entitled "Production of Recombinant 121P2A3 in Eukaryotic Systems" and Figure 21). Other recombinant 121P2A3 variant 1-expressing cells or cells endogenously expressing 121P2A3 variant 1 are also used. Mice showing the strongest reactivity are rested and given a final injection of Tag5 antigen in PBS and then sacrificed four days later. The spleens of the sacrificed mice are harvested and fused to SPO/2 myeloma cells using standard procedures (Harlow and Lane, 1988). Supernatants from HAT selected growth wells are screened by ELISA, Western blot, immunoprecipitation, fluorescent microscopy, and flow cytometry to identify 121P2A3 specific antibody-producing clones.

The binding affinity of a 121P2A3 monoclonal antibody is determined using standard technologies. Affinity measurements quantify the strength of antibody to epitope binding and are used to help define which 121P2A3 monoclonal antibodies preferred for diagnostic or therapeutic use, as appreciated by one of skill in the art. The BIAcore system (Uppsala, Sweden) is a preferred method for determining binding affinity. The BIAcore system uses surface plasmon resonance (SPR, Welford K. 1991, Opt Quant. Elect. 23:1; Morton and Myszka, 1998, Methods in Enzymology 295: 268) to monitor biomolecular interactions in real time. BIAcore analysis conveniently generates association rate constants, dissociation rate constants, equilibrium dissociation constants, and affinity constants.

### Example 12: Diagnostic use of Anti-121P2A3 Antibodies in humans.

Anti-121P2A3 monoclonal antibodies are safely and effectively used for diagnostic, prophylactic, and/or prognostic purposes in humans. Western blot and immunohistochemical analysis of cancer tissues and cancer xenografts with anti-121P2A3 mAb show strong extensive staining in carcinoma but significantly lower or undetectable levels in normal tissues. Detection of 121P2A3 in carcinoma and in metastatic disease demonstrates the usefulness of the mAb as a diagnostic and/or prognostic indicator. Anti-121P2A3 antibodies are therefore used in diagnostic applications such as immunohistochemistry of kidney biopsy specimens to detect cancer from suspect patients.

As determined by flow cytometry, anti-121P2A3 mAb specifically binds to carcinoma cells. Thus, anti-121P2A3 antibodies are used in diagnostic whole body imaging applications, such as radioimmunoscintigraphy and radioimmunotherapy, (see, e.g., Potamianos S-, et. al. Anticancer Res 20(2A):925-948 (2000)) for the detection of localized and metastatic cancers that exhibit expression of 121P2A3. Shedding or release of an extracellular domain of 121P2A3 into the extracellular milieu, such as that seen for alkaline phosphodiesterase B10 {Meerson, N. R., Hepatology 37:563-568 (1998)), allows diagnostic detection of 121P2A3 by anti-121P2A3 antibodies in serum and*l*or mine samples from suspect patients.

### Example 13: Human Clinical Trials for the Diagnostic of Human Carcinomas trough use of Human Anti-121P2A3 Antibodies In vivo

Antibodies are used in accordance with the present invention which recognize an epitope on 121P2A3, and are used in the treatment of certain tumors such as those listed in Table I. Based upon a number of factors, including 121P2A3 expression levels, tumors such as those listed in Table I are presently preferred indications.

Through binding a radionuclide (e.g., iodine or yttrium (I¹³¹, Y⁹⁰) to anti-121P2A3 antibodies, the radiolabeled antibodies are utilized as a a diagnostic and*l*or imaging agent. In such a role, the labeled antibodies localize to both solid tumors, as well as, metastatic lesions of cells expressing 121P2A3. In connection with the use of the anti-121P2A3 antibodies as imaging agents, the antibodies are used as an adjunct to surgical treatment of solid tumors, as both a pre-surgical screen as well as a postoperative follow-up to determine what tumor remains and/or returns. In one embodiment, a (¹¹¹In)-121P2A3 antibody is used as an imaging agent in a Phase I human clinical trial in patients having a carcinoma that expresses 121P2A3 (by analogy see, *e*.*g*., Divgi et al. J. Natl. Cancer Inst. 83:97-104 (1991)). Patients are followed with standard anterior and posterior gamma camera. The results indicate that primary lesions and metastatic lesions are identified

### Dose and Route of Administration

As appreciated by those of ordinary skill in the art, dosing considerations can be determined through comparison with the analogous products that are in the clinic. Thus, anti-121P2A3 antibodies can be administered with doses in the range of 5 to 400 mg/m², with the lower doses used, e.g., in connection with safety studies. The affinity of anti-121P2A3 antibodies relative to the affinity of a known antibody for its target is one parameter used by those of skill in the art for determining analogous dose regimens. Further, anti-121P2A3 antibodies that are fully human antibodies, as compared to the chimeric antibody, have slower clearance; accordingly, dosing in patients with such fully human anti-121P2A3 antibodies can be lower, perhaps in the range of 50 to 300 mg/m², and still remain efficacious. Dosing in mg*l*m², as opposed to the conventional measurement of dose in mg/kg, is a measurement based on surface area and is a convenient dosing measurement that is designed to include patients of all sizes from infants to adults.

Three distinct delivery approaches are useful for delivery of anti-121P2A3 antibodies. Conventional intravenous delivery is one standard delivery technique for many tumors. However, in connection with tumors in the peritoneal cavity, such as tumors of the ovaries, biliary duct, other ducts, and the like, intraperitoneal administration may prove favorable for obtaining high dose of antibody at the tumor and to also minimize antibody clearance. In a similar manner, certain solid tumors possess vasculature that is appropriate for regional perfusion. Regional perfusion allows for a high dose of antibody at the site of a tumor and minimizes short term clearance of the antibody.

### Ç1inical Development Plan (CDP)

Overview. The CDP follows and develops treatments of anti-121P2A3 antibodies as an imaging agent. Trials initially demonstrate safety and thereafter confirm efficacy in repeat doses. Trails are open label comparing standard chemotherapy with standard therapy plus anti- 121P2A3 antibodies. As will be appreciated, one criteria that can be utilized in connection with enrollment of patients is 121P2A3 expression levels in their tumors as determined by biopsy.

As with any protein or antibody infusion-based therapeutic, safety concerns are related primarily to (i) cytokine release syndrome, i.e., hypotension, fever, shaking, chills; (ii) the development of an immunogenic response to the material (i.e., development of human antibodies by the patient to the antibody therapeutic, or HAHA response); and, (iii) toxicity to normal cells that express 121P2A3. Standard tests and follow-up are utilized to monitor each of these safety concerns. Anti-121P2A3 antibodies are found to be safe upon human administration.

### Example 14: Human Clinical Trial: Diagnostic Imagine with Anti-121P2A3 Antibody

Once again, as the adjunctive therapy discussed above is safe within the safety criteria discussed above, a human clinical trial is conducted concerning the use of anti-121P2A3 antibodies as a diagnostic imaging agent. The protocol is designed in a substantially similar manner to those described in the art, such as in Divgi et al. J, Natl. Cancer Inst. 83:97-104 (1991). The antibodies are found to be both safe and efficacious when used as a diagnostic modality. and luminescence of the reaction is monitored in a luminometer. Moreover, the 121P2A3 protein contains several phosphorylation sites (Table XX), indicating its association with specific signaling cascades.

Signaling pathways activated by 121P2A3 are mapped and used for the identification and validation of therapeutic targets. When 121P2A3 is involved in cell signaling, it is used as target for diagnostic, prognostic, preventative and therapeutic purposes.

### Example 15: Involvement in Tumor Progression

The 121P2A3 gene can contribute to the growth of cancer cells. The role of 121P2A3 in tumor growth is investigated in a variety of primary and transfected cell lines including prostate, colon, bladder and kidney cell lines as well as NIH 3T3 cells engineered to stably express 121P2A3. Parental cells lacking 121P2A3 and cells expressing 121P2A3 are evaluated for cell growth using a well-documented proliferation assay (Fraser SP, Grimes IA, Djamgoz MB. Prostate. 2000;44:61, Johnson DE, Ochieng J, Evans SL. Anticancer Drugs. 1996, 7:288).

To determine the role of 121P2A3 in the transformation process, its effect in colony forming assays is investigated. Parental NIH3T3 cells lacking 121P2A3 are compared to NHI-3T3 cells expressing 121P2A3, using a soft agar assay under stringent and more permissive conditions (Song Z. et al. Cancer Res. 2000;60:6730).

To determine the role of 12 1 P2A3 in invasion and metastasis of cancer cells, a well-established assay is used, e.g., a Transwell Insert System assay (Becton Dickinson) (Cancer Res. 1999; 59:6014). Control cells, including prostate, colon, bladder and kidney cell lines lacking 121P2A3 are compared to cells expressing 121P2A3. Cells are loaded with the fluorescent dye, calcein, and plated in the top well of the Transwell insert coated with a basement membrane analog. Invasion is determined by fluorescence of cells in the lower chamber relative to the fluorescence of the entire cell population.

121P2A3 can also play a role in cell cycle and apoptosis, Parental cells and cells expressing t21P2A3 are compared for differences in cell cycle regulation using a well-established BrdU assay (Abdel-Malek ZA. J Cell Physiol. 1988, 136:247). In short, cells are grown under both optimal (full serum) and limiting (low serum) conditions, then are labeled with BrdU and stained with anti-BrdU Ab and propidium iodide. Cells are analyzed for entry into the G1, S, and G2M phases of the cell cycle. Alternatively, the effect of stress on apoptosis is evaluated in control parental cells and cells expressing 121P2A3, including normal and tumor prostate, colon and lung cells. Engineered and parental cells are treated with various chemotherapeutic agents, such as etoposide, flutamide, etc, and protein synthesis inhibitors, such as cycloheximide. Cells are stained with annexin V-FITC and cell death is measured by FACS analysis. The modulation of cell death by 121P2A3 can play a critical role in regulating tumor progression and tumor load.

When 121 P2A3 plays a role in cell growth, transformation, invasion or apoptosis, it is used as a target for diagnostic or prognostic purposes.

### Example 16: Involvement in Angiogenesis

Angiogenesis or new capillary blood vessel formation is necessary for tumor growth (Hanahan D, Folkman J. Cell. 1996, 86:353; Folkman J. Endocrinology. 1998 139:441). Several assays have been developed to measure angiogenesis *in vitro* and *in vivo*, such as the tissue culture assays endothelial cell tube formation and endothelial cell proliferation. Using these assays as well as *in vitro* neo-vascularization, it is determined whether 121P2A3 enhances or inhibits angiogenesis,

For example, endothelial cells engineered to express 121P2A3 are evaluated using tube formation and proliferation assays. The effect of 121P2A3 can also be evaluated in animal models *in vivo*. For example, cells either expressing or lacking 121P2A3 are implanted subcutaneously in ïmmunocompromised mice. Endothelial cell migration and angiogenesis are evaluated 5-15 days later using ummunohistochemistry techniques. When 121P2A3 affects angiogenesis, it is used as a target for diagnostic or prognostic purposes

### Example 17: Regulation of Transcription

The localization of 121P2A3 in the nucleus and its similarity to NAF-1 indicate that 121P2A3 plays a role in the transcriptional regulation of eukaryotic genes. Regulation of gene expression is evaluated, e.g., by studying gene expression in cells expressing or lacking 121P2A3. For this purpose, two types of experiments are performed.

In the first set of experiments, RNA from parental and 121P2A3-expressing cells are extracted and hybridized to commercially available gene arrays (Clontech) (Smid-Koopman E et al. Br J Cancer. 2000. 83:246). Resting cells as well as cells treated with FBS or androgen are compared. Differentially expressed genes are identified in accordance with procedures known in the art. The differentially expressed genes are then mapped to biological pathways (Chen K. et al. Thyroid. 2001.11:41.).

In the second set of experiments, specific transcriptional pathway activation is evaluated using commercially available (Stratagene) luciferase reporter constructs including: NFkB-luc, SRE-luc, ELK1-luc, ARE-luc, p53-luc, and CRE-luc. These transcriptional reporters contain consensus binding sites for known transcription factors that lie downstream of well-characterized signal transduction pathways, and represent a good tool to ascertain pathway activation and screen for positive and negative modulators of pathway activation.

When 121P2A3 plays a role in gene regulation, it is used as a target for diagnostic or prognostic, purposes.

### Example 18: Involvement in Cell Adhesion

Cell adhesion plays a critical role in tissue colonization and metastasis. Based on its homology to CLIP-190, 121P2A3 can participate in cellular organization, and as a consequence cell adhesion and motility. To determine that 121P2A3 regulates cell adhesion, control cells lacking 121P2A3 are compared to cells expressing 121P2A3, using techniques previously described (see, e.g., Haier et al, Br. J. Cancer. 1999, 80:1867; Lehr and Pienta J. Natl. Cancer lnst. 1998, 90,118). Briefly, in one embodiment, cells labeled with a fluorescent indicator, such as calcein, are incubated on tissue culture wells coated with media alone or with matrix proteins. Adherent cells are detected by fluorimetric analysis and percent adhesion is calculated. In another embodiment, cells lacking or expressing 121P2A3 are analyzed for their ability to mediate cell-cell adhesion using similar experimental techniques as described above. Both of these experimental systems are used to identify proteins, antibodies and/or small molecules that modulate cell adhesion to extracellular matrix and cell-cell interaction. Since cell adhesion plays a critical role in tumor growth, progression, and, colonization, when 121 P2A3 is involved in this processes it serves as a diagnostic, preventative and therapeutic modality

### Example 19: Involvement of 121P2A3 in Protein Trafficking.

Due to its similarity to CLIP- 190, 121P2A3 can regulate intracellular trafficking. Trafficking of proteins can be studied using well-established methods (valetti C. et al. Mol Biol Cell. 1999, 10:4107). For example, FITC-conjugated α2-macroglobulin is incubated with 121P2A3-expressing and 121P2A3-negative cells. The location and uptake of FITC-α2-macroglobulin is visualized using a fluorescent microscope. In another set of experiments, the co-localization of 121P2A3 with vesicular proteins is evaluated by coprecipitation and Western blotting techniques and fluorescent microscopy.

Alternatively, 121P2A3-expresing and 121P2A3-lacking cells are compared using bodipy-ceramide labeled bovine serum albumine (Huber L et al. Mol. Cell. Biol. 1995, 15:918). Briefly, cells are allowed to injest the labeled BSA and are placed intermittently at 4°C and 18°C to allow for trafficking to take place. Cells are examined under fluorescent microscopy at different time points for the presence of labeled BSA in specific vesicular compartments, including Golgi, endoplasmic reticulum, etc. In another embodiment, the effect of 121P2A3 on membrane transport is examined using biotin-avidin complexes. Cells either expressing or lacking 121P2A3 are transiently incubated with biotin. The cells are placed at 4°C or transiently warmed to 37°C for various periods of time. The cells are fractionated and examined by avidin affinity precipitation for the presence of biotin in specific cellular compartments. Using such assay systems, proteins, antibodies and small molecules are identified that modify the effect of 121P2A3 on vesicular transport. When 121P2A3 plays a role in intracellular trafficking, 121P2A3 is a target for diagnostic or prognostic purposes

### Example 20: Protein-Protein Association

The Naf-1 protein homologous to 121P2A3 has been shown to interact with other proteins, thereby forming a protein complex that can regulate cell division, gene transcription, and cell transformation (Renkema GH et al, Curr Biol. 1999, 9:1407; Baur AS et al, Immunity. 1997, 6:283; Karakesisoglou I, Yang Y, Fuchs E. J Cell Biol. 2000, 149:195.). Using immunoprecipitation techniques as well as two yeast hybrid systems, proteins are identified that associate with 121P2A3. immunoprecipitates from cells expressing 121P2A3 and cells lacking 121P2A3 are compared for specific protein-protein associations.

Studies are performed to determine whether 121 P2A3 associates with effector molecules, such as adaptor proteins and SH2-containing proteins. Studies comparing 121P2A3 positive and 121P2A3 negative cells as well as studies comparing unstimulated/resting cells and cells treated with epithelial cell activators, such as cytokines, growth factors, androgen and anti-integrin Ab reveal unique interactions. In addition, protein-protein interactions are studied using two yeast hybrid methodology (Curr Opin Chem Biol. 1999, 3:64), A vector carrying a library of proteins fused to the activation domain of a transcription factor is introduced into yeast expressing a 121P2A3 -DNA-binding domain fusion protein and a reporter construct. Protein-protein interaction is detected by calorimetric reporter activity. Specific association with effector molecules and transcription factors indicates the mode of action of 121P2A3, and thus identifies therapeutic, preventative and/or diagnostic targets for cancer. This and similar assays can also be used to identify and screen for small molecules that interact with 121P2A3.

When 121P2A3 associates with proteins or small molecules it is used as a target for diagnostic or prognostic purposes.

**TABLE I: Tissues that Express 121P2A3 When Malignant**

| |
|---|
| - Prostate |
| - Bladder |
| - Kidney |
| - Colon |
| - Lung |
| - Ovary |
| - Breast |
| - Stomach |
| - Rectum |
| - Pancreas |
| - Testis |
| - Brain |
| - Bone |
| - Cervix |

**TABLE II: Amino Acid Abbreviations**

| **SINGLE LETTER** | **THREE LETTER** | **FULL NAME** |
|---|---|---|
| | | |
| F | Phe | phenylalanine |
| L | Leu | leucine |
| S | Ser | serine |
| Y | Tyr | tyrosine |
| C | Cys | cysteine |
| W | Trp | tryptophan |
| P | Pro | proline |
| H | His | histidine |
| Q | Gln | glutamine |
| R | Arg | arginine |
| I | Ile | isoleucine |
| M | Met | methionine |
| T | Thr | threonine |
| N | Asn | asparagine |
| K | Lys | lysine |
| V | Val | valine |
| A | Ala | alanine |
| D | Asp | aspartic acid |
| E | Glu | plutamic acid |
| G | Gly | glycine |

**Table III. Exon compositions of 121P2A3 v.1**

| Exon Number | Start | End |
|---|---|---|
| Exon 1 | 2 | 162 |
| Exon 2 | 163 | 357 |
| Exon 3 | 358 | 633 |
| Exon 4 | 634 | 702 |
| Exon 5 | 703 | 853 |
| Exon 6 | 854 | 1167 |
| Exon 7 | 1168 | 1239 |
| Exon 8 | 1240 | 1365 |
| Exon 9 | 1366 | 2473 |

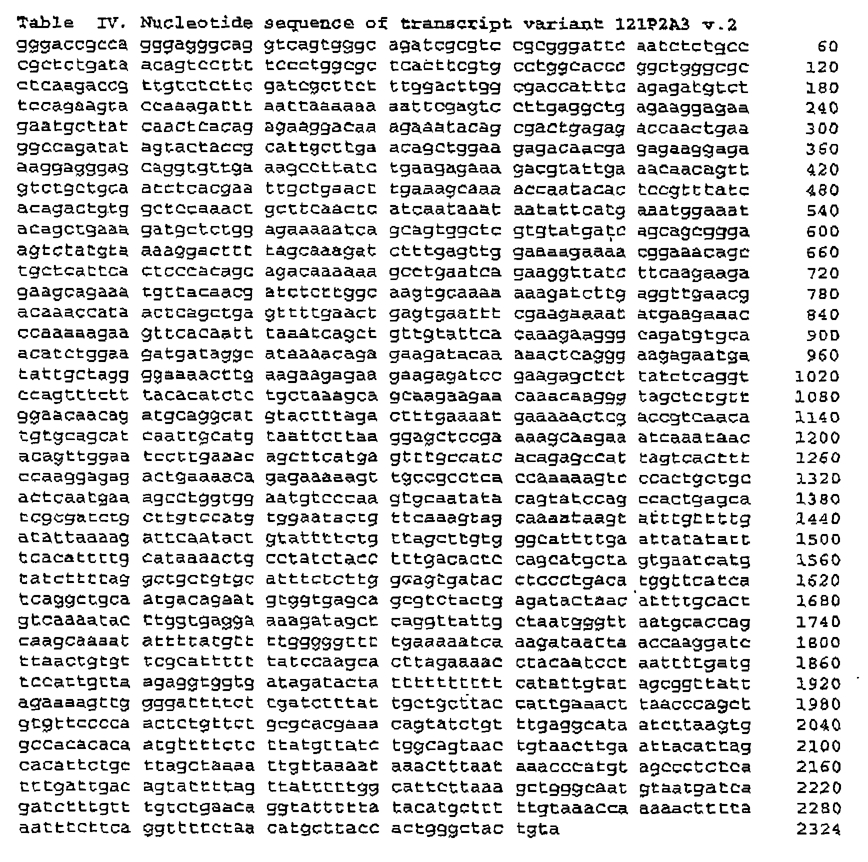

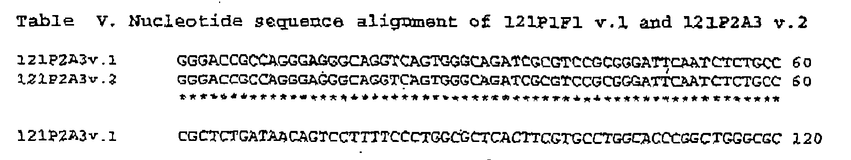

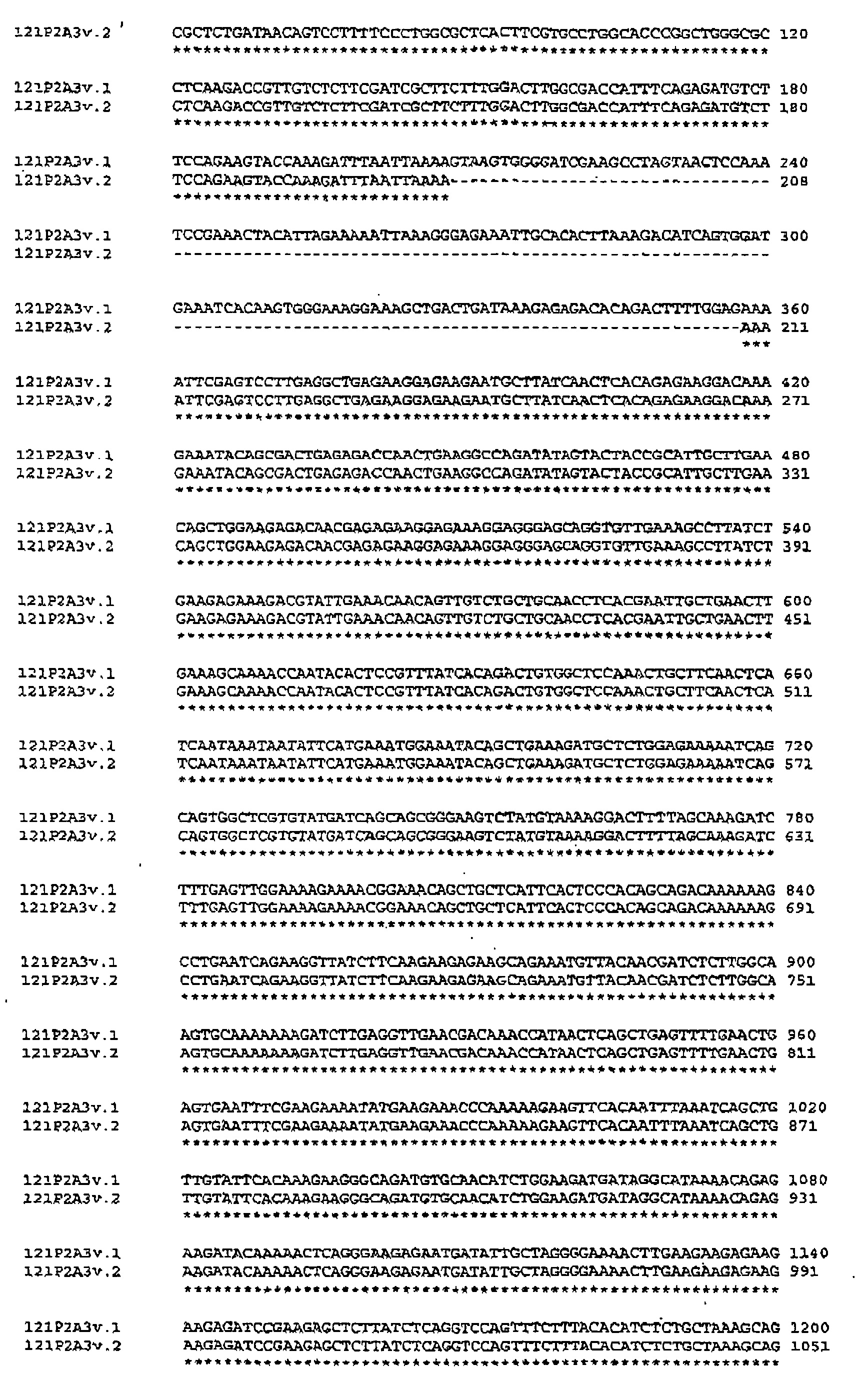

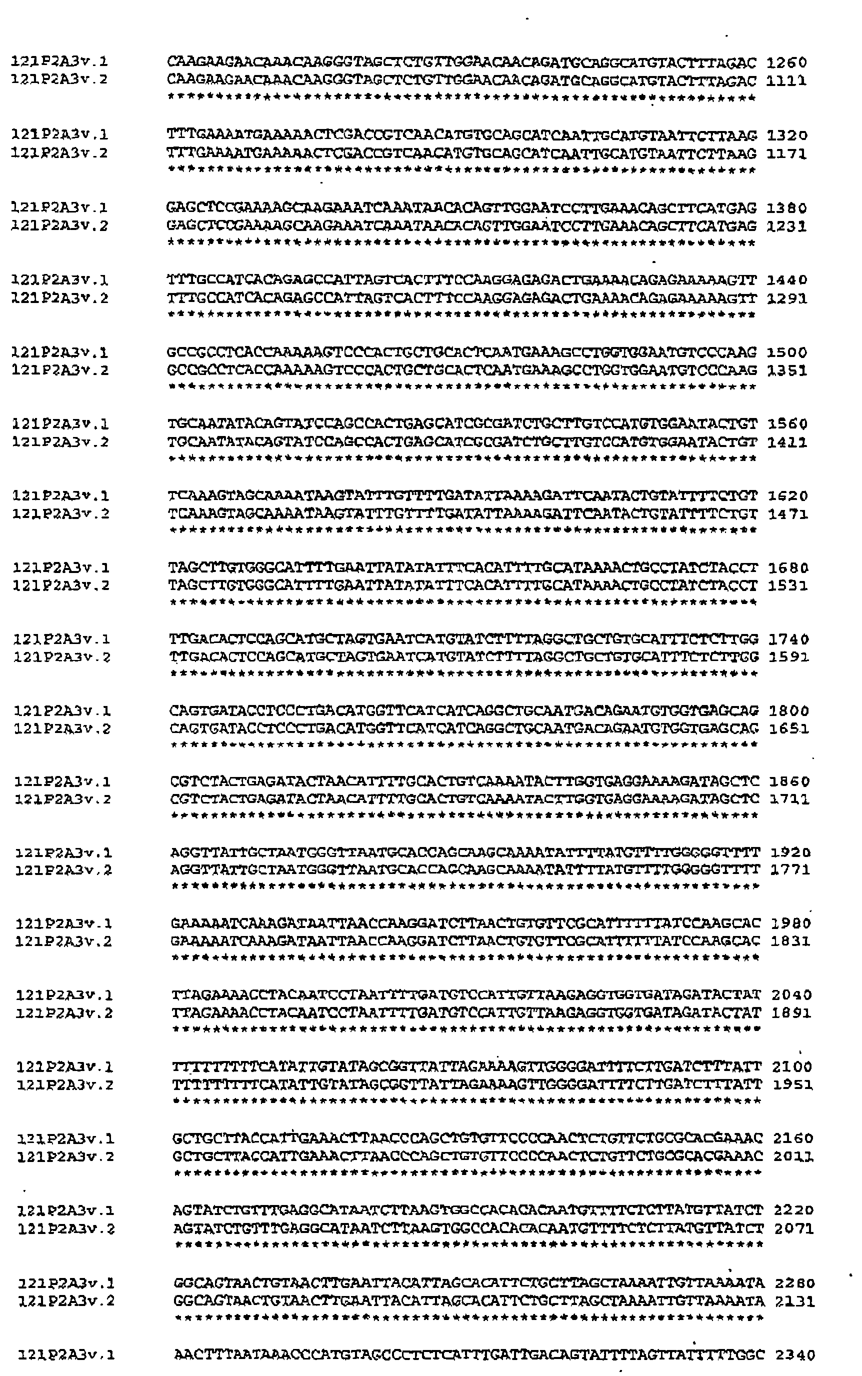

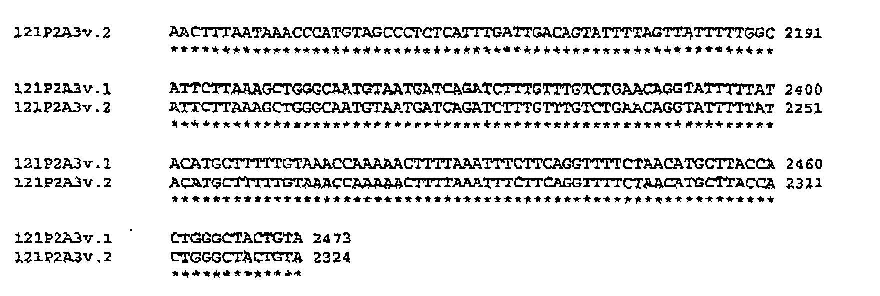

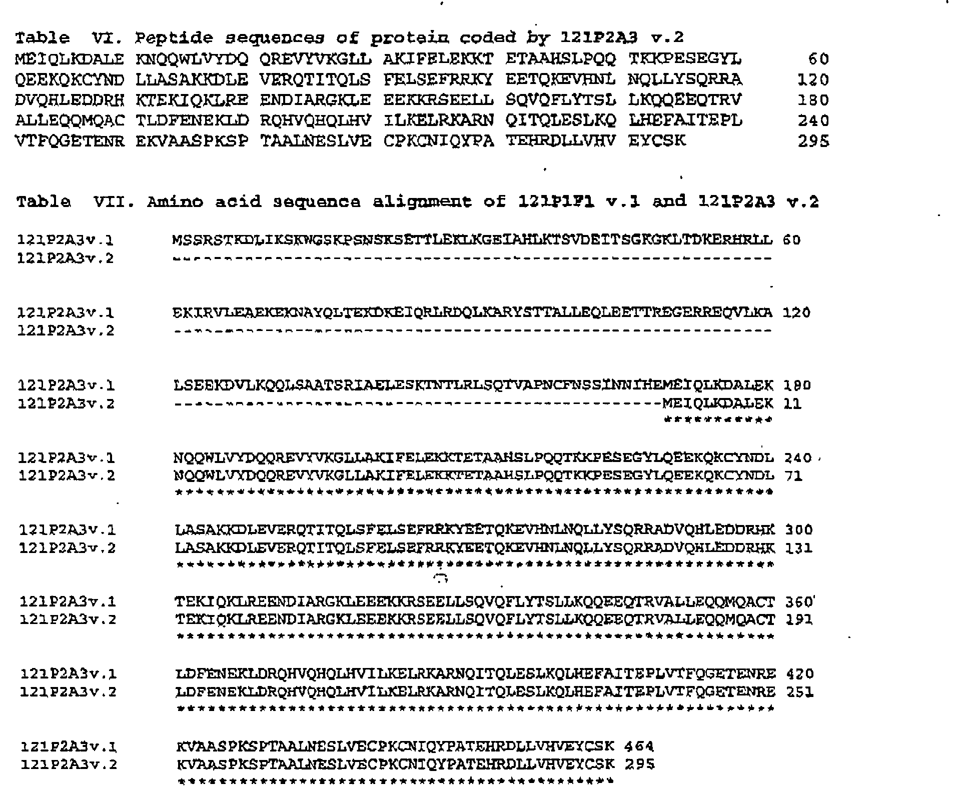

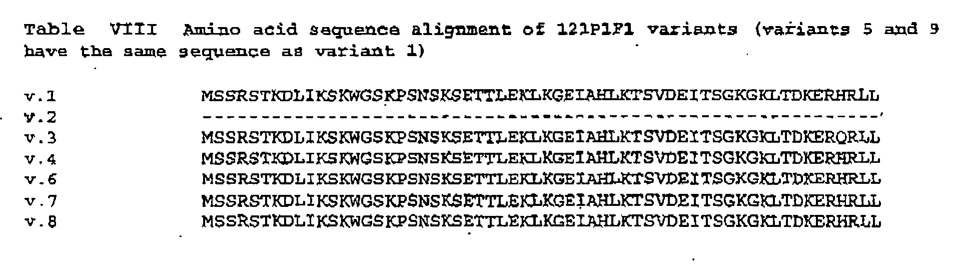

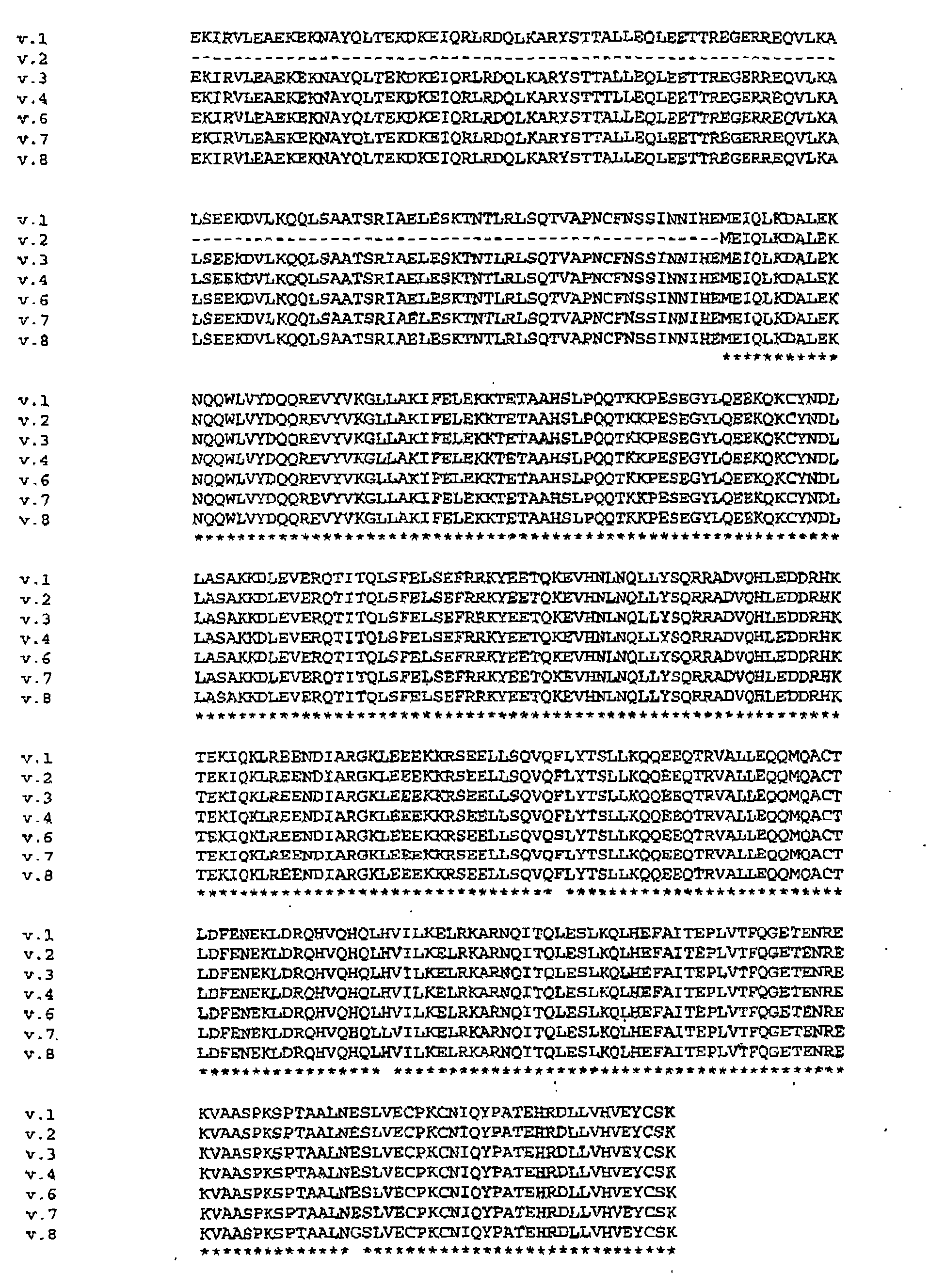

## Claims

1. A method for diagnosing cancer in a test prostate or colon sample, comprising:
contacting the test sample with an antibody or antigen binding fragment thereof that specifically binds to a protein having an identical amino acid sequence as that shown in Figure 3A, 3B, 3C, 3D, 3E, 3F or 3G and does not bind to a peptide or a protein that (a) does not have an identical amino acid sequence as that shown in Figure 3A, 3B, 3C, 3D, 3E, 3F or 3G or (b) is not a fragment of a sequence shown in Figure 3A, 3B, 3C, 3D, 3E, 3F or 3G;
determining the level of expression of the protein in the test sample; and
comparing the level so determined to the level of expression of the protein in a corresponding normal sample;
wherein an increased level of expression of the protein in the test sample relative to the normal sample indicates the presence of cancer.

2. The method of claim 1, wherein the antibody or fragment thereof is labelled with a detectable marker.

3. The method of claim 2, wherein the detectable marker is a radioactive isotope, a fluorescent compound, a bioluminescent compound, a metal chelator or an enzyme.

## Patentansprüche

1. Methode zum Diagnostizieren von Krebs in einer Test-Prostata- oder Dickdarm-Probe, umfassend
Kontaktieren der Testprobe mit einem Antikörper oder Antigen-bindenden Fragment davon, der/das spezifisch an ein Protein mit einer identischen Aminosäuresequenz bindet wie der, die in Figur 3A, 3B, 3C, 3D, 3E, 3F oder 3G gezeigt ist, und nicht an ein Peptid oder eine Protein bindet, das (a) keine identische Aminosäuresequenz aufweist wie die, die in Figur 3A, 3B, 3C, 3D, 3E, 3F oder 3G gezeigt ist, oder (b) kein Fragment einer Sequenz, wie in Figur 3A, 3B, 3C, 3D, 3E, 3F oder 3G gezeigt, ist;
Bestimmen der Höhe der Expression des Proteins in der Testprobe; und
Vergleichen der derart bestimmten Höhe mit der Höhe der Expression des Proteins in einer entsprechenden normalen Probe;
worin ein erhöhter Grad der Expression des Proteins in der Testprobe relativ zu der normalen Probe das Vorhandensein von Krebs anzeigt.

2. Methode nach Anspruch 1, worin der Antikörper oder das Fragment davon mit einer nachweisbaren Markierung markiert ist.

3. Methode nach Anspruch 2, worin die nachweisbare Markierung ein radioaktives Isotop, eine fluoreszierende Verbindung, einem biolumineszierende Verbindung, ein Metallchelator oder ein Enzym ist.

## Revendications

1. Méthode pour diagnostiquer un cancer dans un échantillon de test de prostate ou de côlon comprenant :
la mise en contact de l'échantillon de test avec un anticorps ou un fragment de celui-ci se liant à un antigène qui se lie spécifiquement à une protéine ayant une séquence d'acides aminés identique à celle présentée à la figure 3A, 3B, 3C, 3D, 3E, 3F, ou 3G et qui ne se lie pas à un peptide ou à une protéine qui (a) n'a pas une séquence d'acides aminés identique à celle présentée à la figure 3A, 3B, 3C, 3D, 3E, 3F, ou 3G ou (b) n'est pas un fragment d'une séquence présentée à la figure 3A, 3B, 3C, 3D, 3E, 3F, ou 3G ;
la détermination du niveau d'expression de la protéine dans l'échantillon de test ; et
la comparaison du niveau ainsi déterminé avec le niveau d'expression de la protéine dans un échantillon normal correspondant ;
dans laquelle un taux plus élevé d'expression de la protéine dans l'échantillon de test par rapport à l'échantillon normal indique la présence d'un cancer.

2. Méthode de la revendication 1, dans laquelle l'anticorps ou un fragment de celui-ci est marqué avec un marqueur détectable.

3. Méthode de la revendication 2, dans laquelle le marqueur détectable est un isotope radioactif, un composé fluorescent, un composé bioluminescent, un chélateur de métaux ou une enzyme.
